(19) Europäisches Patentamt
European Patent Office
Office européen des brevets

(11) EP 0 602 596 B1

(12) EUROPÄISCHE PATENTSCHRIFT

(45) Veröffentlichungstag und Bekanntmachung des Hinweises auf die Patenterteilung:
**05.11.1997 Patentblatt 1997/45**

(21) Anmeldenummer: **93120134.7**

(22) Anmeldetag: **14.12.1993**

(51) Int. Cl.[6]: **C07C 69/90**, C09K 19/30, C09K 19/32, C09K 19/34, C09K 19/40, C09K 19/12, C09K 19/20, C07C 43/225, C07F 7/08, C07D 239/26, C07D 239/34

(54) **Trifluorphenylen-Verbindungen, Verfahren zu ihrer Herstellung und ihre Verwendung in flüssigkristallinen Mischungen**

Trifluorophenylene compounds, process for their preparation and their use in liquid crystalline mixtures

Composés du trifluorophénylène, procédé pour leur préparation et leur utilisation dans des mélanges de cristaux liquides

(84) Benannte Vertragsstaaten:
**CH DE FR GB LI**

(30) Priorität: **17.12.1992 DE 4242695**
**23.06.1993 DE 4320755**

(43) Veröffentlichungstag der Anmeldung:
**22.06.1994 Patentblatt 1994/25**

(73) Patentinhaber:
**HOECHST AKTIENGESELLSCHAFT**
**65929 Frankfurt am Main (DE)**

(72) Erfinder:
- **Wingen, Rainer, Dr.**
**D-65795 Hattersheim/M (DE)**
- **Pfirmann, Ralf, Dr.**
**D-64347 Griesheim (DE)**

(56) Entgegenhaltungen:
**EP-A- 0 090 183** **WO-A-89/02425**
**DE-A- 4 111 765**
- **Liquid Crystals, Applications and uses. Ed. B. Bahadur. Vol. 1, page 307.**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Rank Xerox (UK) Business Services
2.14.23/3.4

## Beschreibung

Flüssigkristalle haben insbesondere im letzten Jahrzehnt Eingang in verschiedene technische Gebiete gefunden, in denen elektrooptische und Anzeigevorrichtungs-Eigenschaften gefragt sind (z.B. in Uhren-, Taschenrechner-und Schreibmaschinenanzeigen). Diese Anzeigevorrichtungen beruhen auf den dielektrischen Ausrichtungseffekten in den nematischen, cholesterischen und/oder smektischen Phasen der flüssigkristallinen Verbindungen, wobei - verursacht durch die elektrische Anisotropie - die molekulare Längsachse der Verbindungen eine bevorzugte Ausrichtung in einem angelegten elektrischen Feld einnimmt. Die üblichen Schaltzeiten bei diesen Anzeigevorrichtungen sind für viele andere potentielle Anwendungsgebiete von Flüssigkristallen zu lang. Dieser Nachteil macht sich insbesondere dann bemerkbar, wenn eine große Anzahl von Bildpunkten angesteuert werden muß. Die Herstellungskosten von Geräten, die größere Bildschirmflächen enthalten, z.B. von Videogeräten, sind dann im allgemeinen zu hoch.

Neben den nematischen und cholesterischen Flüssigkristallen haben seit einigen wenigen Jahren in zunehmendem Maße auch optisch aktive smektische Flüssigkristall-Phasen an Bedeutung gewonnen.

Clark und Lagerwall konnten zeigen, daß die Verwendung ferroelektrischer Flüssigkristallsysteme in sehr dünnen Zellen zu optoelektrischen Schalt-oder Anzeigeelementen führt, die im Vergleich zu den herkömmlichen TN ("twisted nematic")-Zellen um bis zu einem Faktor 1000 schnellere Schaltzeiten haben (vgl. z.B. Lagerwall et al. "Ferroelectric Liquid Crystals for Displays", SID Symposium, October Meeting 1985, San Diego, Ca., USA). Aufgrund dieser und anderer günstiger Eigenschaften, z.B. der bistabilen Schaltmöglichkeit und des nahezu blickwinkelunabhängigen Kontrasts, sind FLCs grundsätzlich für die obengenannten Anwendungsgebiete, z.B. über eine Matrixansteuerung, gut geeignet.

Für elektrooptische oder vollständig optische Bauelemente benötigt man entweder Verbindungen, die geneigte bzw. orthogonale smektische Phasen ausbilden und selbst optisch aktiv sind, oder man kann durch Dotierung von Verbindungen, die zwar solche smektischen Phasen ausbilden, selbst aber nicht optisch aktiv sind, mit optisch aktiven Verbindungen ferroelektrische smektische Phasen induzieren. Die gewünschte Phase soll dabei über einen möglichst großen Temperaturbereich stabil sein.

Zur Erzielung eines guten Kontrastverhältnisses in elektooptischen Bauelementen ist eine einheitliche planare Orientierung der Flüssigkristalle nötig. Eine gute Orientierung in der $S_A$ und $S^*_C$-Phase läßt sich erreichen, wenn die Phasenfolge der Flüssigkristallmischung mit abnehmender Temperatur lautet:

$$\text{Isotrop} \rightarrow N^* \rightarrow S_A \rightarrow S^*_C$$

Vorraussetzung ist, daß der Pitch (Ganghöhe der Helix) in der N*-Phase sehr groß (größer 10 µm) oder noch besser völlig kompensiert ist (siehe z.B. T. Matsumoto et al., p. 468-470, Proc. of the 6th Int. Display Research Conf., Japan Display, Sept. 30 - Okto. 2, 1986, Tokyo, Japan; M. Murakami et al., ibid. S. 344 - S. 347). Dies erreicht man, indem man zu der chiralen Flüssigkristallmischung, die in der N*-Phase z.B. eine linksdrehende Helix aufweist, einen weiteren optisch aktiven Dotierstoff, der eine rechtsdrehende Helix induziert, in solchen Mengen hinzugibt, daß die Helix gerade kompensiert wird.

Für die Verwendung des SSFLCD-Effektes (Surface Stabilized Ferroelectric Liquid Crystal Display) von Clark und Lagerwall zur einheitlichen, planaren Orientierung ist ferner Voraussetzung, daß der Pitch in der smektischen C*-Phase wesentlich größer ist als die Dicke des Anzeigeelementes (Mol. Cryst. Liq. Cryst. 94 (1983), 213-233 und 114 (1984), 151-187). Dies erreicht man wie im Fall des cholesterischen Pitches durch Verwendung von Dotierstoffen mit entgegengesetztem Drehsinn der Helix.

Ferroelektische Flüssigkristallanzeigen lassen sich auch durch Nutzung des DHF (Distorted Helix Formation) - Effektes oder des PSFLCD-Effektes (Pitch Stabilized Ferroelectric Liquid Crystal Display, auch SBF = Short pitch Bistable Ferroelectric Effect genannt) betreiben. Der DHF-Effekt wurden von B.I. Ostrovski in Advances in Liquid Crystal Research and Applications, Oxford/Budapest, 1980, 469 ff. beschrieben, der PSFLCD-Effekt ist in DE-A 3 920 625 bzw. EP-A 0 405 346 beschrieben. Zur Nutzung dieser Effekte wird im Gegensatz zum SSFLCD-Effekt ein flüssigkristallines Material mit einem kurzen $S_C$-Pitch benötigt.

Die optische Schaltzeit $\tau$ [µs] ferroelektischer Flüssigkristallsysteme, die möglichst kurz sein soll, hängt von der Rotationsviskosität des Systems $y$ [mPas], der spontan Polarisation $P_s$[nC/cm$^2$] und der elektrischen Feldstärke E[V/m] ab nach der Beziehung

$$\tau \sim \frac{y}{P_S \cdot E}$$

Da die Feldstärke E durch den Elektrodenabstand im elektrooptischen Bauteil und durch die angelegte Spannung festgelegt ist, muß das ferroelektrische Anzeigemedium niedrigviskos sein und eine hohe spontan Polarisation aufweisen, damit eine kurze Schaltzeit erreicht wird.

Schließlich wird neben thermischer, chemischer und photochemischer Stabilität eine kleine optische Anisotropie $\Delta n$, vorzugsweise $\approx 0{,}13$, und eine geringe positive oder vorzugsweise negative dielektrische Anisotropie $\Delta\varepsilon$ verlangt (siehe S.T. Lagerwall et al., "Ferroelectric Liquid Crystals for Displays" SID Symposium, Oct. Meeting 1985, San Diego, Ca, USA).

Die Gesamtheit dieser Forderungen ist nur mit Mischungen aus mehreren Komponenten zu erfüllen. Als Basis (oder Matrix) dienen dabei bevorzugt Verbindungen, die möglichst selbst bereits die gewünschte Phasenfolge $I \rightarrow N \rightarrow S_A \rightarrow S_C$ aufweisen. Weitere Komponenten der Mischungen werden oftmals zur Schmelzpunktserniedrigung und zur Verbreiterung der $S_C$- und meist auch N-Phase, zum Induzieren der spontanen Polarisation, zur Pitch-Kompensation und zur Anpassung der optischen und dielektrischen Anisotropie zugesetzt, wobei aber beispielsweise die Rotationsviskosität möglichst nicht vergrößert werden soll.

Mehrfach fluorierte Phenylderivate sind bereits mehrfach als Komponenten von Flüssigkristallmischungen beschrieben, z.B. Derivate des 1,2-Difluorphenylens in EP-B 375 702 und EP-A 500 210, Derivate des 1,3-Difluorphenylens in DE-A 4 127 450, Derivate des 1,2,3,4-Tetrafluorphenylens in DE-A 4 037 519, Derivate des 1,2,4,5-Tetrafluorphenylens (vorgestellt auf der 12th International Liquid Crystal Conference, 1988, Freiburg, Abstract FE 68 und WO 89/09203) sowie spezielle Dotierstoffe mit einer 1,2,4-Trifluorphenylen- bzw. 1,2,4,5-Tetrafluorphenylen-Einheit in JP-A 5-17406.

In der DE-A 41 11 765 und der EP-A 0 090 183 sind Trifluorphenylen-Derivate mit endständigem Fluoratom bzw. endständiger Cyanogruppe zur Verwendung in nematischen Flüssigkristallmischungen beschrieben.

Da aber die Entwicklung, insbesondere von ferroelektrischen Flüssigkristallmischungen, noch in keiner Weise als abgeschlossen betrachtet werden kann, sind die Hersteller von Displays an den unterschiedlichsten Komponenten für Mischungen interessiert. Dieses u.a. auch deshalb, weil erst das Zusammenwirken der flüssigkristallinen Mischungen mit den einzelnen Bauteilen der Anzeigevorrichtung bzw. der Zellen (z.B. der Orientierungsschicht) Rückschlüsse auf die Qualität auch der flüssigkristallinen Mischungen zuläßt.

Aufgabe der vorliegenden Erfindung war es daher, neue Verbindungen bereitzustellen, die in flüssigkristallinen Mischungen geeignet sind, das Eigenschaftsprofil dieser Mischungen zu verbessern.

Diese Aufgabe wird erfindungsgemäß gelöst mit Trifluorphenylen-Verbindungen der Formel I

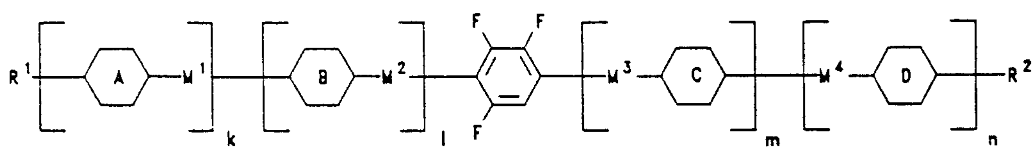

(I)

in der die Symbole und Indizes folgende Bedeutung haben:

$R^1$, $R^2$ sind, unabhängig voneinander Wasserstoff, oder ein geradkettiger oder verzweigter Alkylrest mit 1 bis 20 C-Atomen (mit oder ohne asymmetrische C-Atome), wobei auch eine oder mehrere $CH_2$-Gruppen durch -O-, - S-, -CO-, -CH CH-, $-C{\equiv}C-$, $\Delta$, $-Si(CH_3)_2-$, 1,4-Phenylen, trans-1,4-Cyclohexylen oder trans-1,3-Cyclopentylen ersetzt sein können, mit der Maßgabe, daß Sauerstoffatome und Schwefelatome (im folgenden Chalkogene genannt) nicht unmittelbar miteinander gebunden sein dürfen, und/oder wobei ein oder mehrere H-Atome des Alkylrestes durch -F, -Cl, -Br, $-OR^3$ substituiert sein können; oder einer der nachfolgend aufgeführten Reste

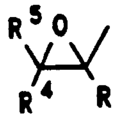 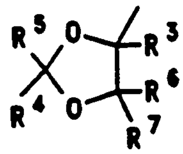 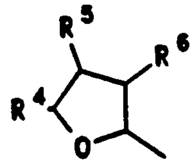

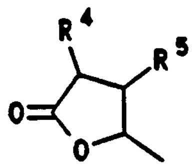 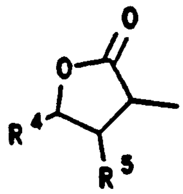 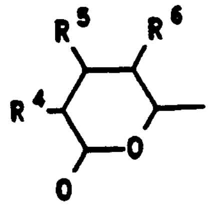

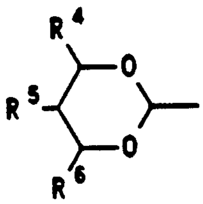 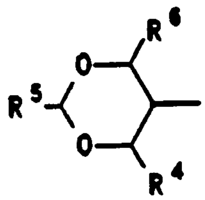 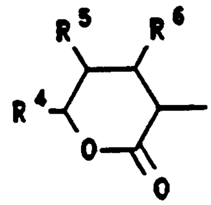

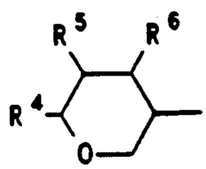 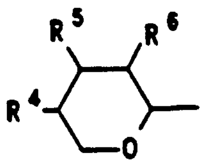 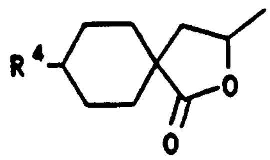

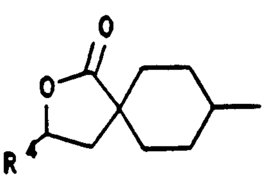 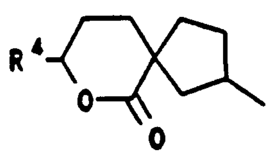 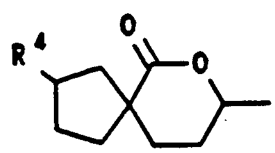

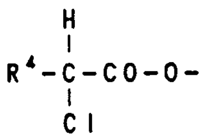 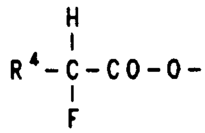 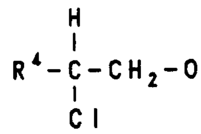 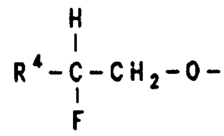

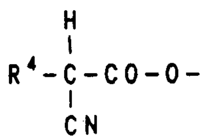 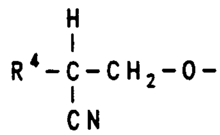 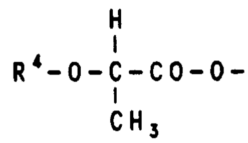 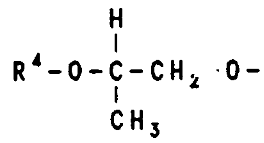

$R^3$, $R^4$, $R^5$, $R^6$, $R^7$ sind unabhängig voneinander Wasserstoff oder ein geradkettiger oder verzweigter Alkylrest mit 1-16 C-Atomen (mit oder ohne asymmetrische C-Atome), wobei auch eine oder mehrere $CH_2$-Gruppen durch -O- oder - CH=CH- ersetzt sein können (mit der Maßgabe, daß Sauerstoffatome nicht unmittelbar miteinander verbunden sein dürfen) und/oder ein oder mehrere H-Atome des Alkylrestes durch - F oder -Cl substituiert sein können; $R^4$ und $R^5$ können zusammen auch -$(CH_2)_4$- oder -$(CH_2)_5$-sein, wenn sie an ein Oxiran-, Dioxolan-, Tetrahydrofuran-, Tetrahydropyran-, Butyrolacton- oder Valerolacton-System gebunden sind;

$M^1$, $M^2$, $M^3$, $M^4$ sind gleich oder verschieden -CO-O-, -O-CO-, -OOC-$CH_2$-$CH_2$-, -$CH_2$-$CH_2$-COO-, -$(CH_2)_4$-, -O$(CH_2)_3$-, -$(CH_2)_3$O-, -$CH_2$-O-, -O-$CH_2$-, -CH=CH-, -C≡C- oder eine Einfachbindung;

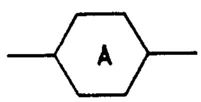 , 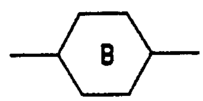 ,

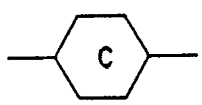 , 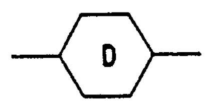 ,

sind gleich oder verschieden 1,4-Phenylen, wobei ein, zwei, drei oder vier H-Atome durch F, Cl und/oder CN ersetzt sein können, Pyrazin-2,5-diyl, Pyridazin-3,6-diyl, Pyridin-2,5-diyl, bei dem auch ein H-Atom durch F ersetzt sein kann, Pyrimidin-2-5-diyl, bei dem auch ein H-Atom durch F ersetzt sein kann, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl-, Bicyclo[2.2.2]octan=1,4-diyl oder 1,3-Dioxaborinan-2,5-diyl;

k, l, m, n sind null oder eins, unter der Bedingung, daß die Summe k + l + m + n 1, 2 oder 3 ist

Bevorzugt sind Verbindungen der Formel (I), bei denen die Symbole und Indizes folgende Bedeutungen haben:

$R^1$, $R^2$ unabhängig voneinander H oder Alkyl mit 1 bis 20 C-Atomen, wobei auch eine oder mehrere $CH_2$-Gruppen durch -O-, -CH = CH-, -CO-, -C≡C- oder -Si$(CH_3)_2$-, ersetzt sein können und eine oder mehrere H-Atome durch F ersetzt sein können; oder eine der Gruppen

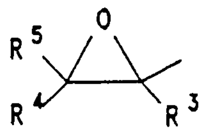, 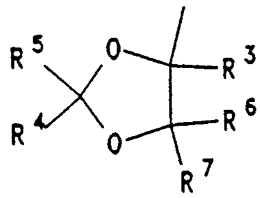

$M^1$, $M^2$, $M^3$, $M^4$ -CO-O-, -O-CO-, $-CH_2-O-$, $-OCH_2-$, -C≡C- oder eine Einfachbindung

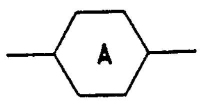, 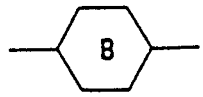,

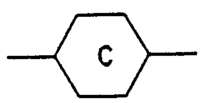, 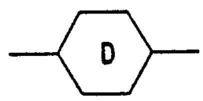,

1,4-Phenylen, wobei ein, zwei oder drei H-Atome durch F ersetzt sein können, Pyrimidin-2,5-diyl, Pyridin-2,5-diyl, bei denen auch 1 H-Atom durch F ersetzt sein kann, 1,4-Cyclohexylen, 1,3-Dioxan-2,5-diyl, (1,3,4)-Thiadiazol, Naphthalin-2,6-diyl,

k + l + m + n 1 oder 2.

Besonders bevorzugt sind Verbindungen der Formel (I), bei denen die Symbole und Indizes folgende Bedeutungen haben:

$R^1$, $R^2$ Alkyl mit 1 bis 16 C-Atomen, wobei auch eine oder mehrere - $CH_2$-Gruppen durch -O-, -CH = CH-, oder $-Si(CH_3)_2-$ ersetzt sein können und ein oder mehrere H-Atome durch F ersetzt sein können;

$M^1$, $M^2$, $M^3$, $M^4$ -CO-O-, -O-CO-, oder eine Einfachbindung,

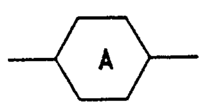, 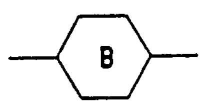,

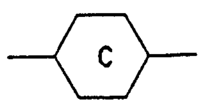, 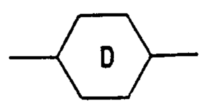,

1,4-Phenylen, wobei ein, zwei oder drei H-Atome durch F ersetzt sein können, Pyrimidin-2,5-diyl, Pyridin-2,5-diyl, bei denen auch 1 H-Atom durch F ersetzt sein kann, 1,4-Cyclohexylen, Naphthalin-2,6-diyl

k + l + m + n 1 oder 2.

Ebenso bevorzugt sind die Verbindungen der Teilstruktur (Ia)

(Ia) 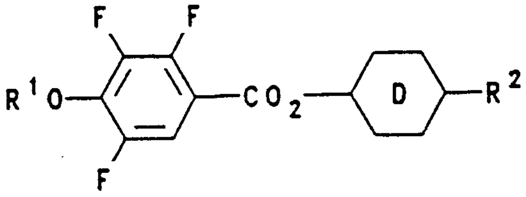

und darin besonders bevorzugt die Verbindungen

(Ia1) 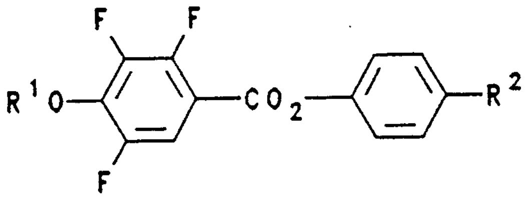

(Ia2)

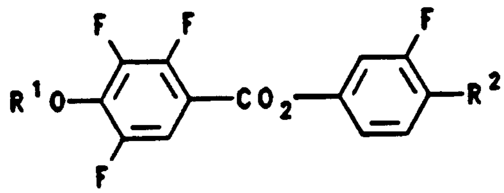

(Ia3)

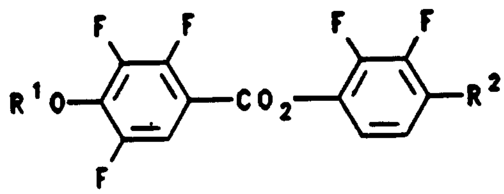

(Ia4)

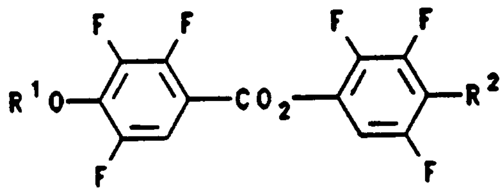

(Ia5)

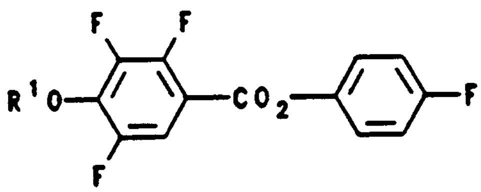

(Ia6)

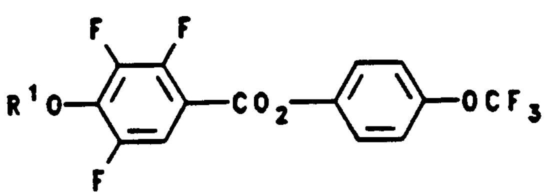

(Ia7)

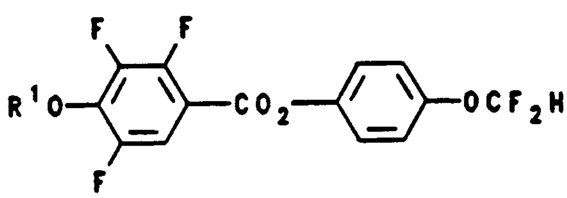

(Ia8)

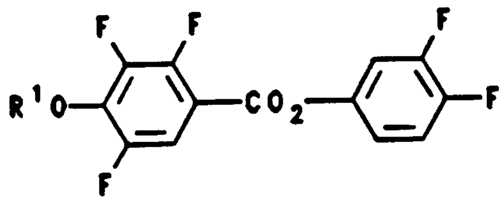

(Ia9)

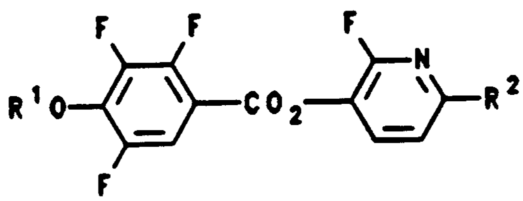

(Ia10)

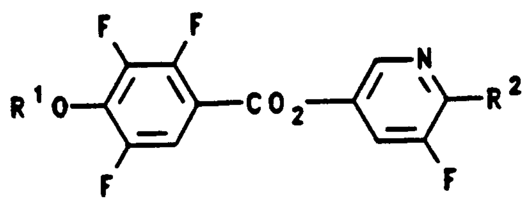

Bevorzugt sind ferner die Verbindungen der Teilstruktur

(Ib)

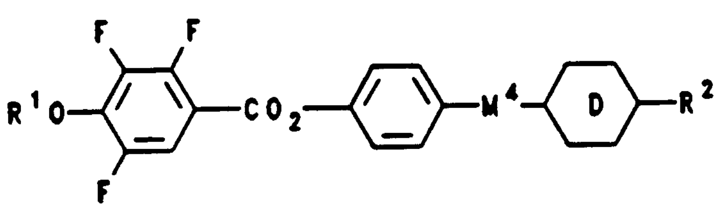

und darunter besonders bevorzugt die Verbindungen

(Ib1)

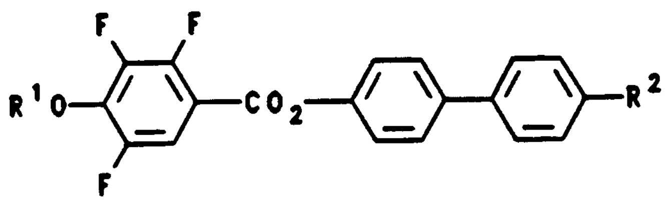

(Ib2)

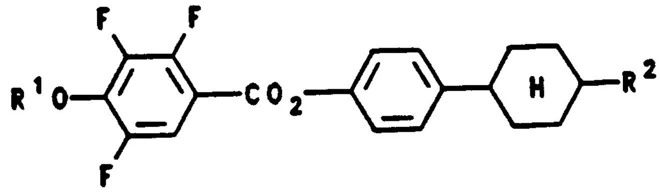

(Ib3)

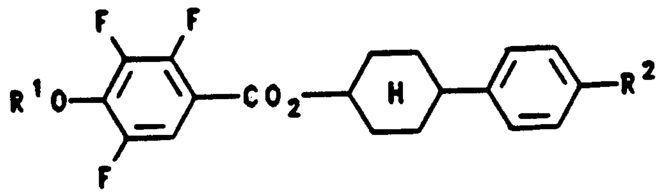

(Ib4)

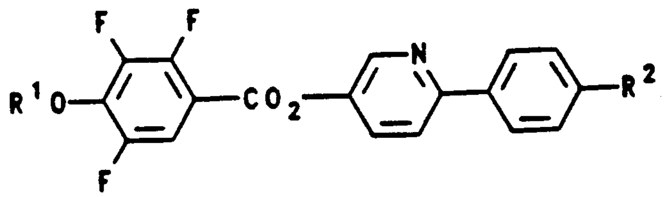

(Ib5)

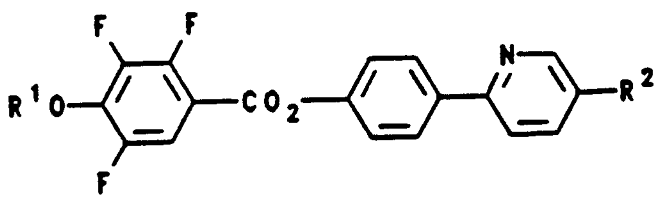

(Ib6)

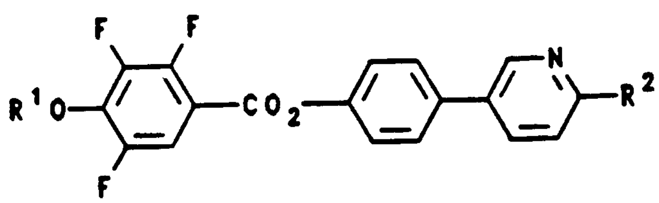

(Ib7)

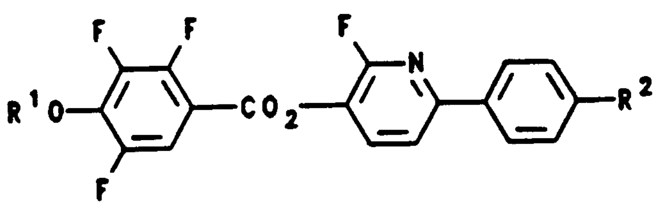

(Ib8)

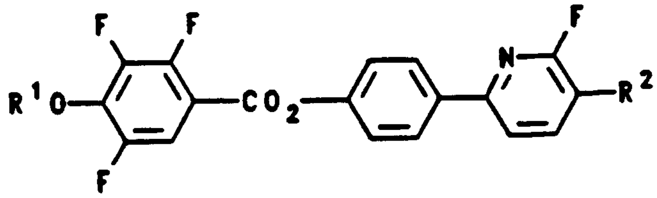

(Ib9)

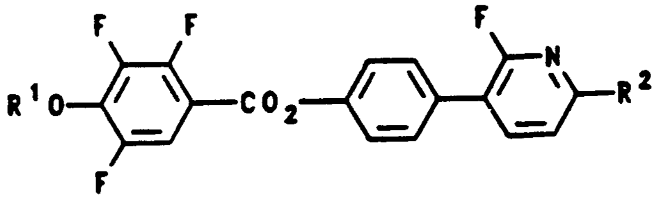

(Ib10)

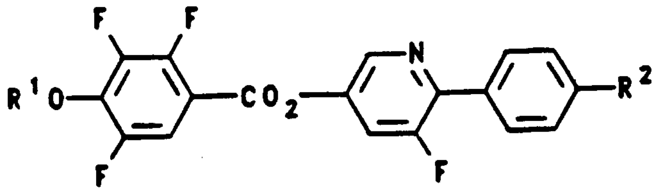

(Ib11)

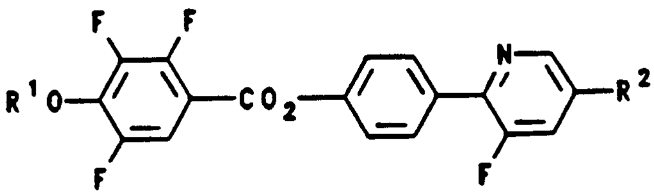

(Ib12)

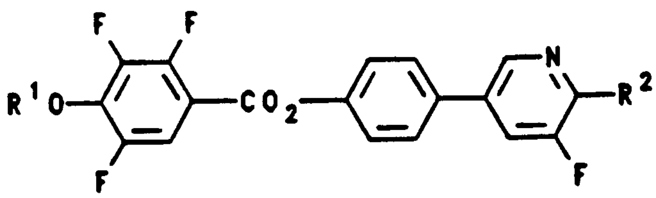

(Ib13)

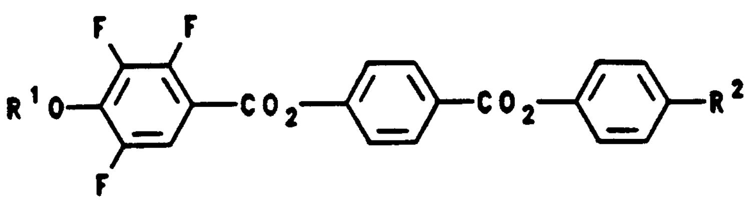

(Ib14) 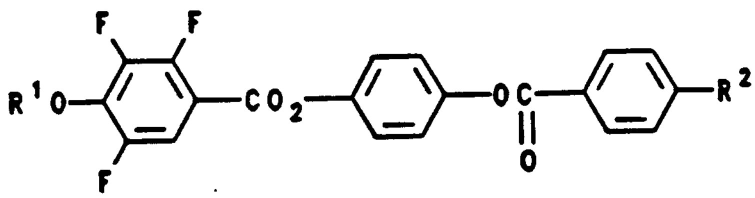

(Ib15) 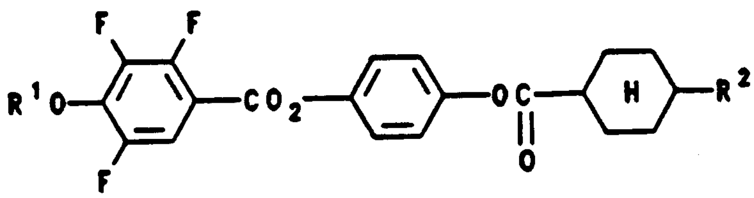

(Ib16) 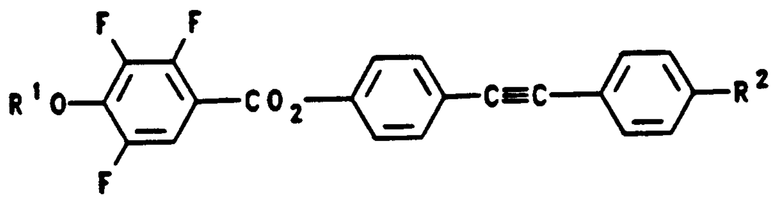

Bevorzugt sind ferner die Verbindungen der Teilstruktur

(Ic) 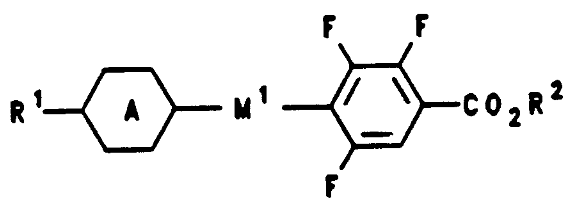

und darunter besonders bevorzugt die Verbindungen

(Ic1) 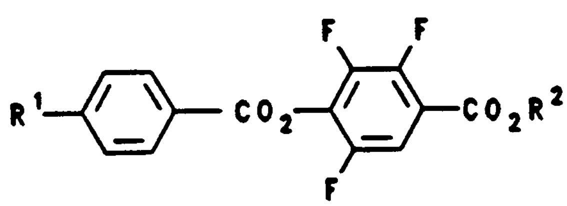

(Ic2) 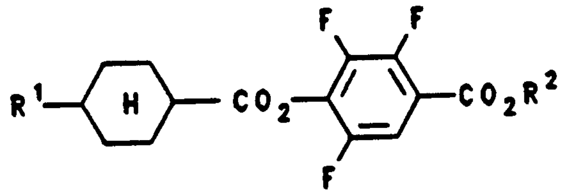

(Ic3) 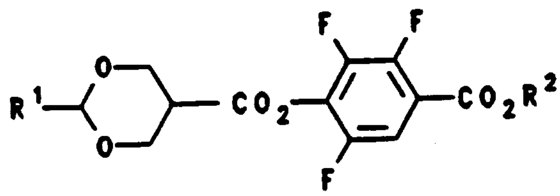

(Ic4) 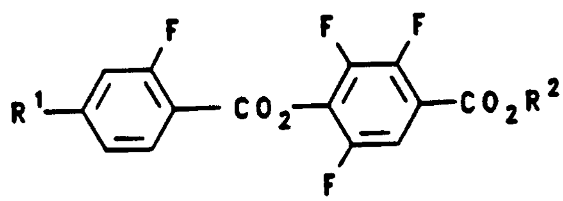

(Ic5) 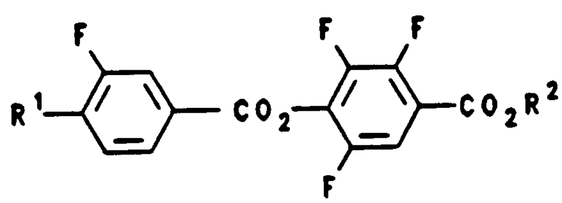

(Ic6) 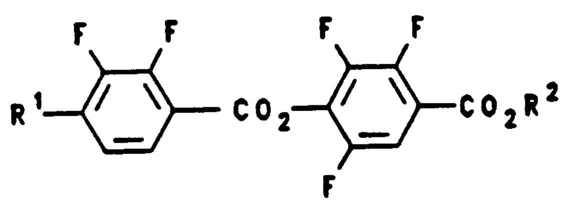

(Ic7) 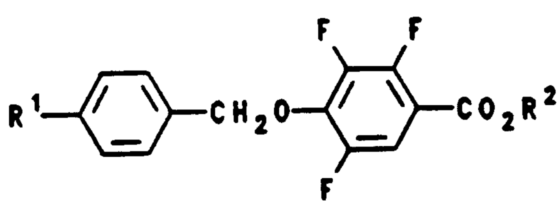

(Ic8) 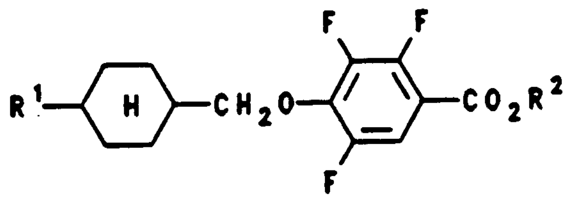

(Ic9) 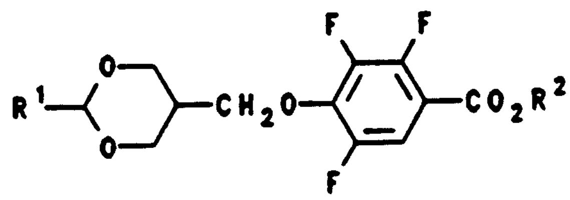

(Ic10) 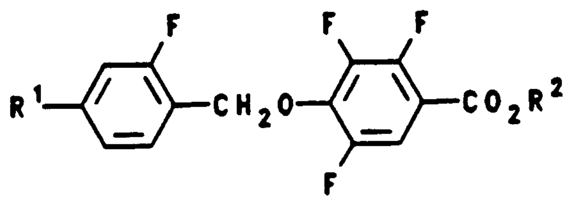

(Ic11) 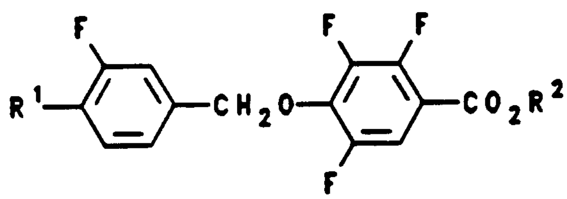

(Ic12) 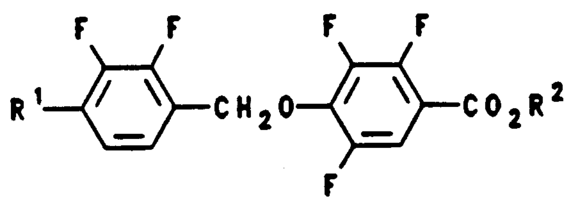

Bevorzugt werden ferner die Verbindungen der Formel (Id)

(Id) 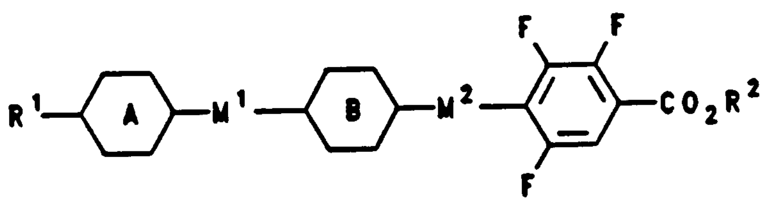

und darunter besonders bevorzugt die Verbindungen

(Id1)

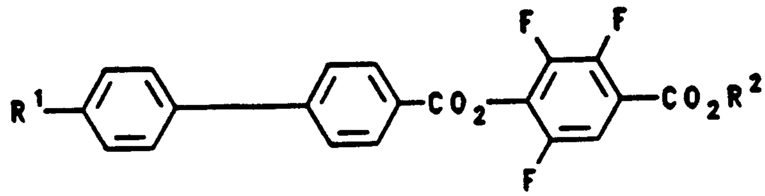

(Id2)

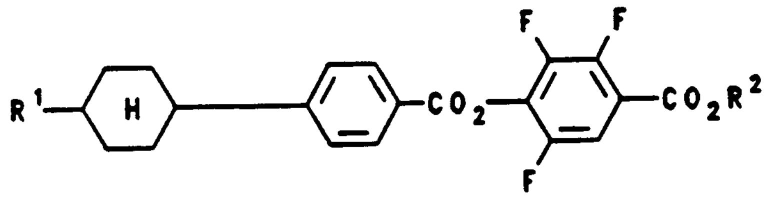

(Id3)

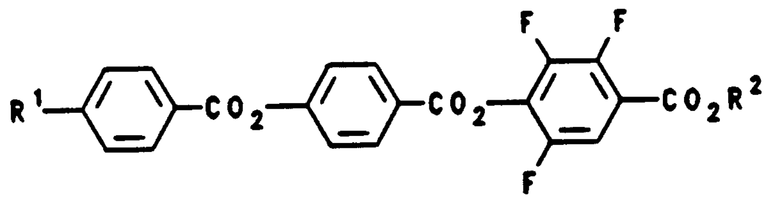

(Id4)

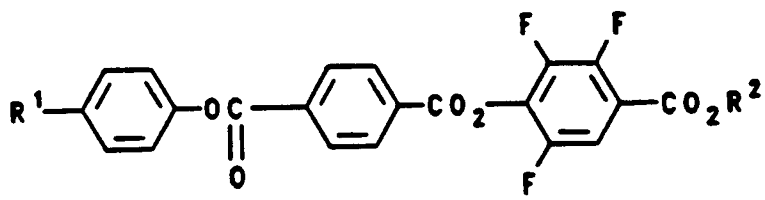

(Id5) 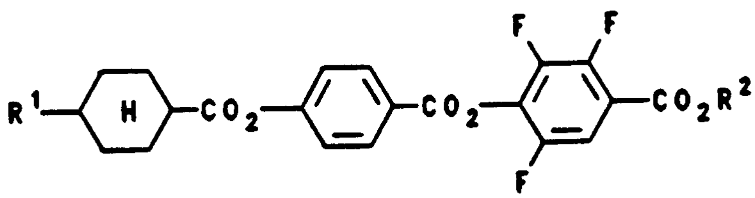

(Id6) 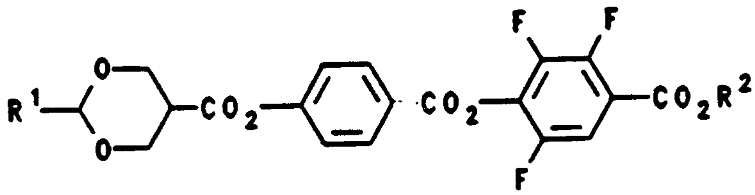

(Id7) 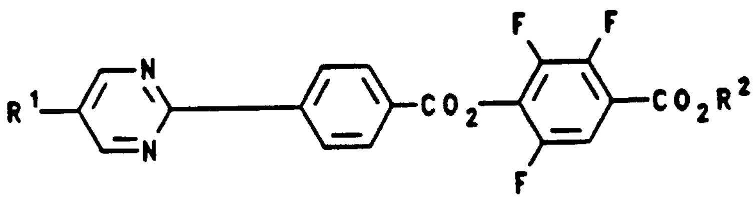

(Id8) 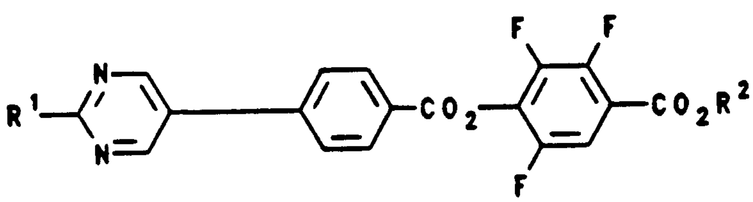

(Id9) 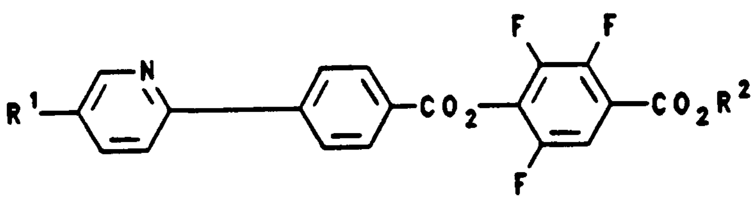

(Id10) 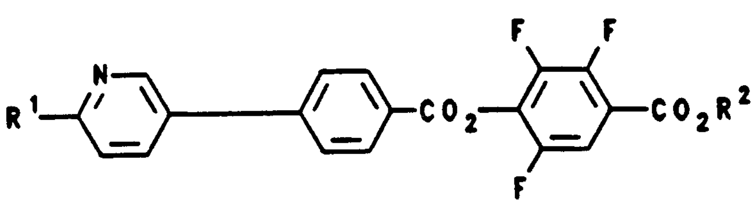

(Id11)

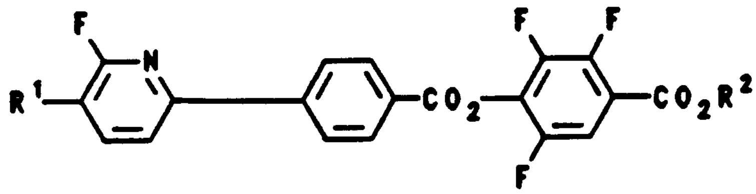

(Id12)

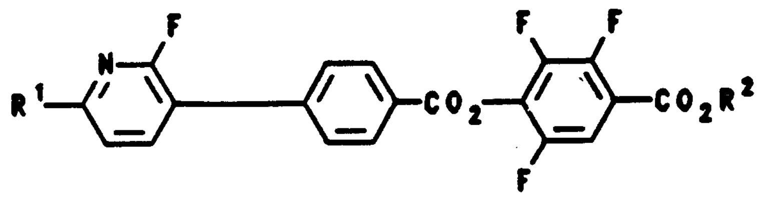

(Id13)

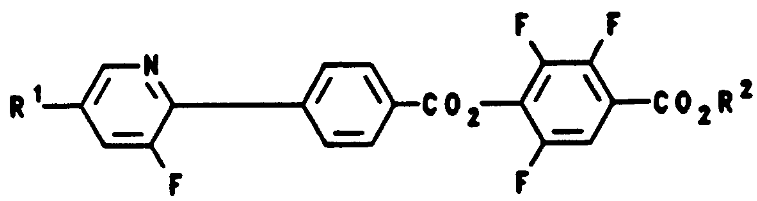

(Id14)

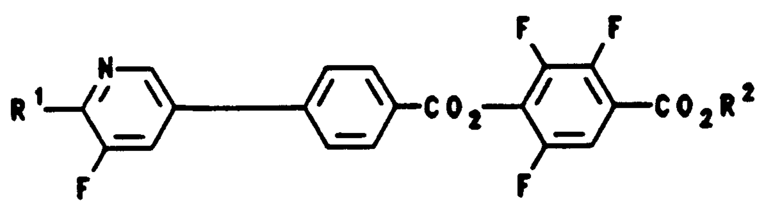

(Id15)

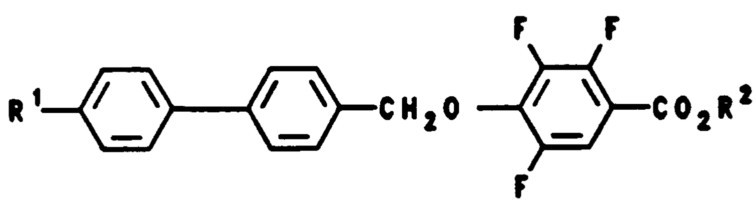

(Id16)

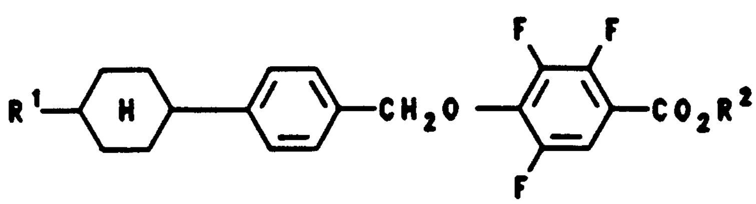

(Id17)

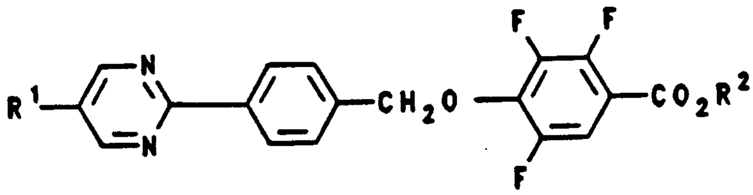

(Id18)

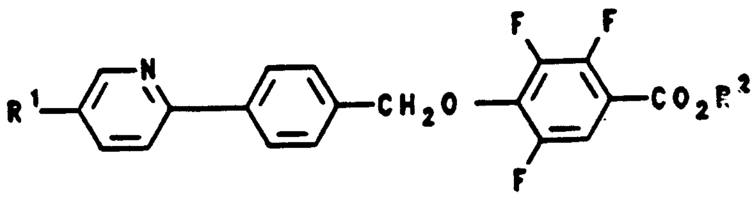

(Id19)

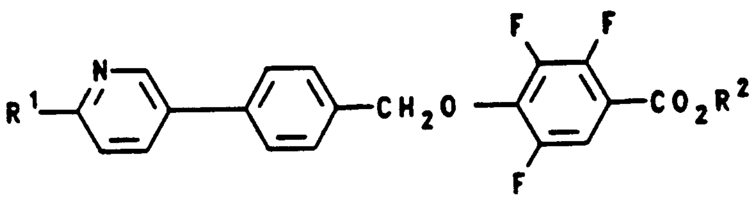

(Id20)

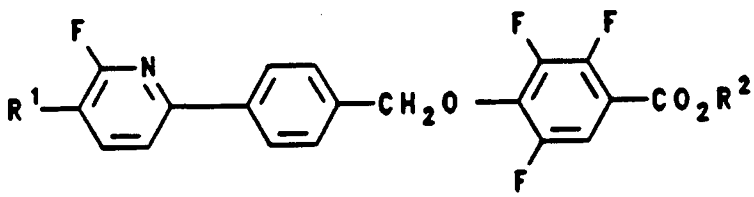

(Id21)

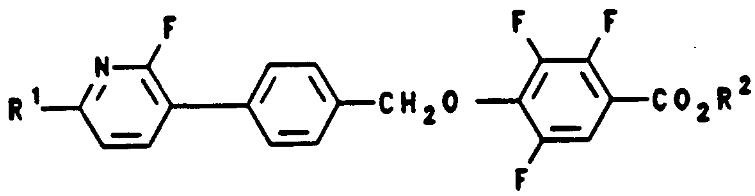

(Id22)

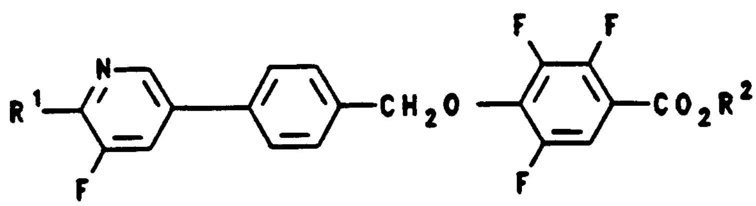

(Id23) 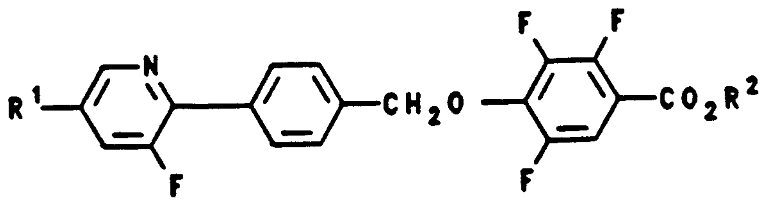

(Id24) 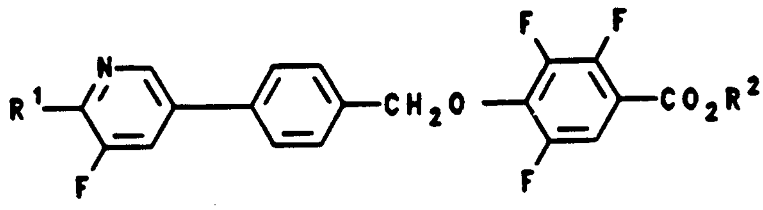

(Id25) 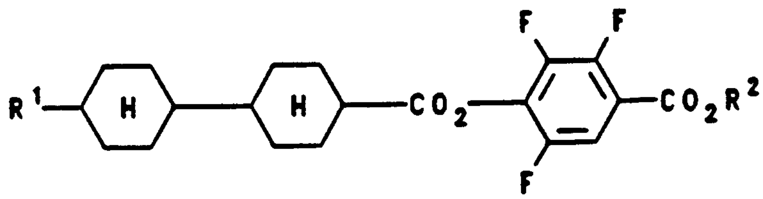

(Id26) 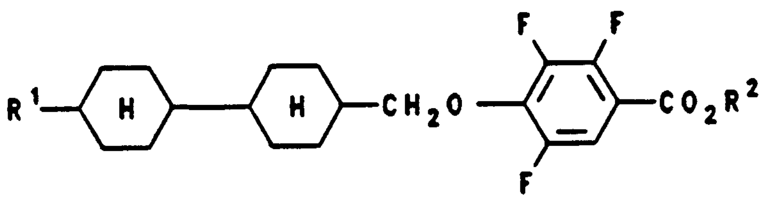

Bevorzugt werden ferner die Verbindungen der Formel

(Ie) 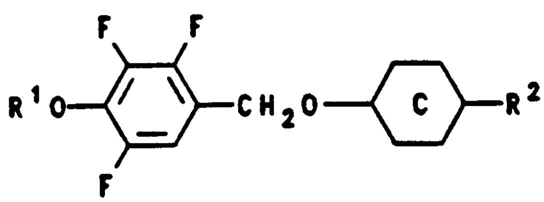

und darunter besonders bevorzugt die Verbindungen

(Ie1) 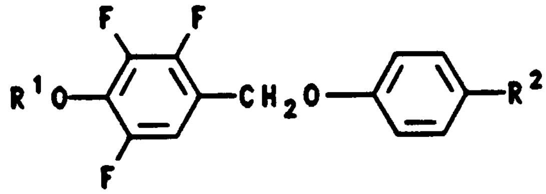

(Ie2) 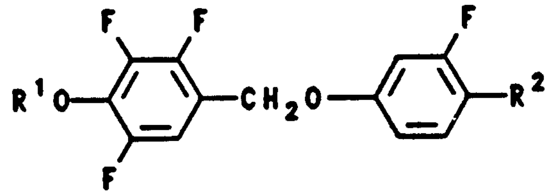

(Ie3) 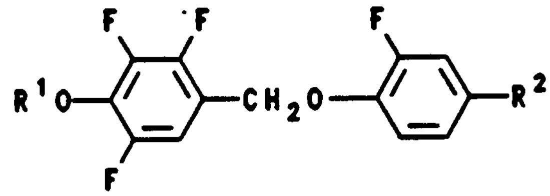

(Ie4) 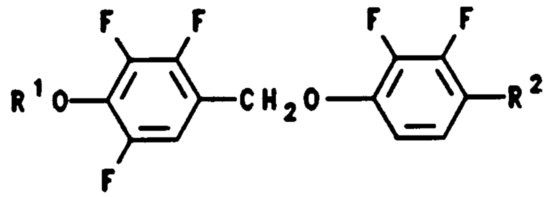

(Ie5) 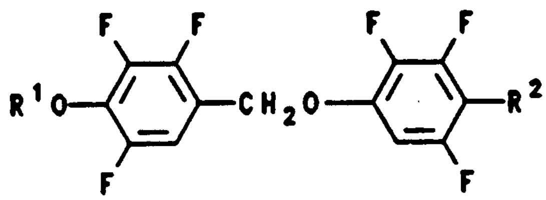

(Ie6) 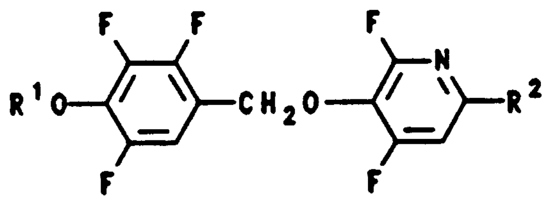

(Ie7) 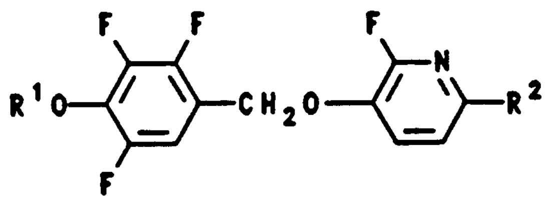

(Ie8) 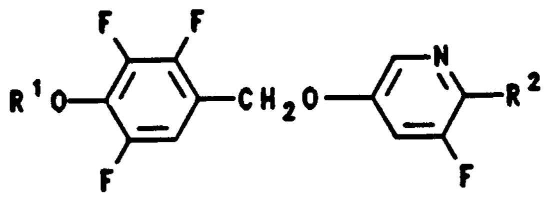

Bevorzugt werden ferner die Verbindungen der Formel

(If) 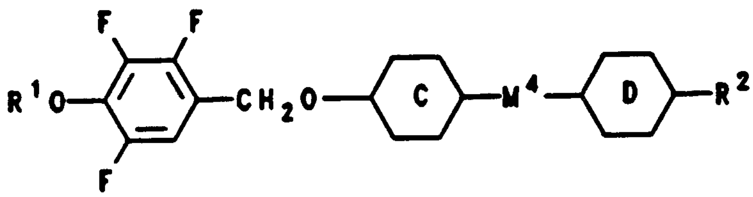

und darin besonders bevorzugt die Verbindungen

(If1) 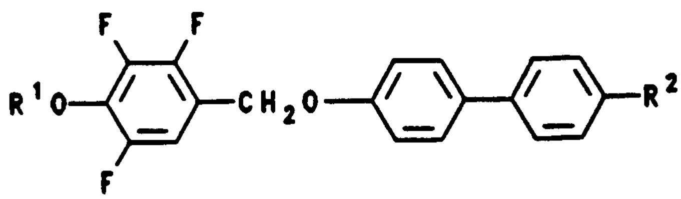

(If2) 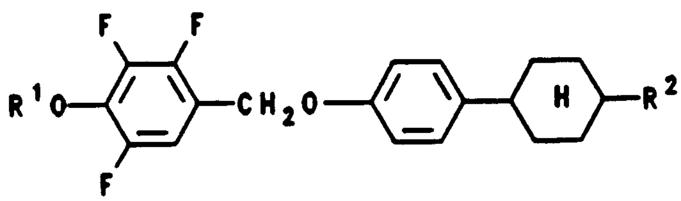

(If3) 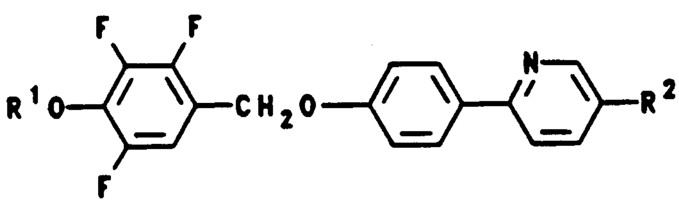

(If4) 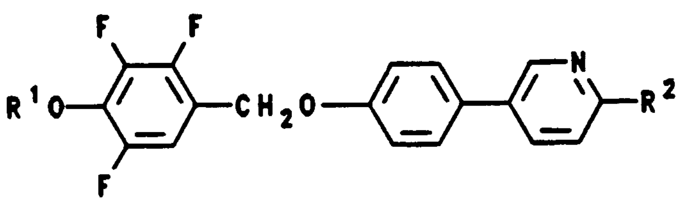

(If5)

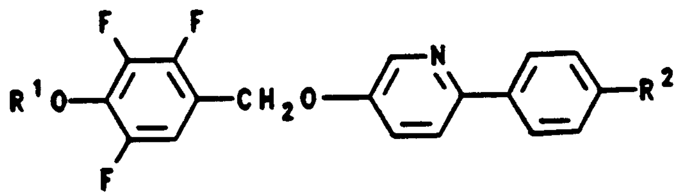

(If6)

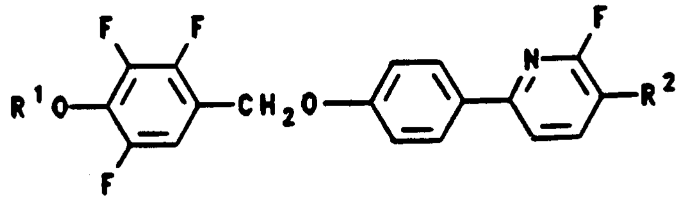

(If7)

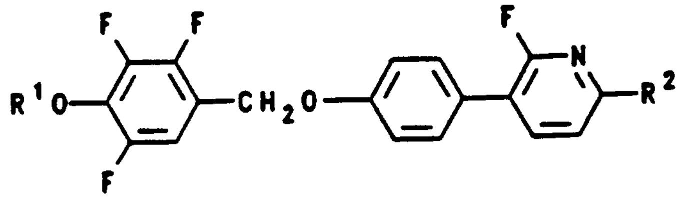

(If8)

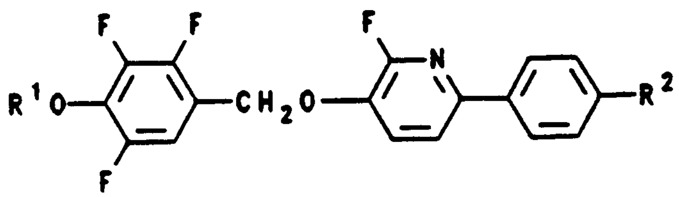

(If9)

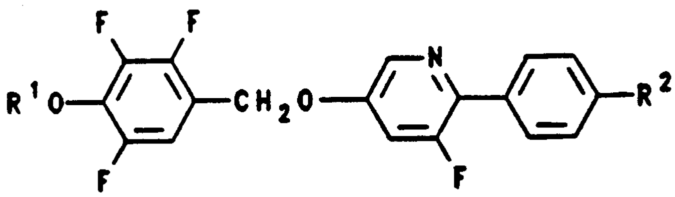

(If10)

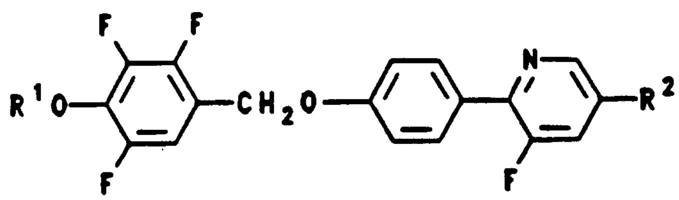

(I f 1 1) 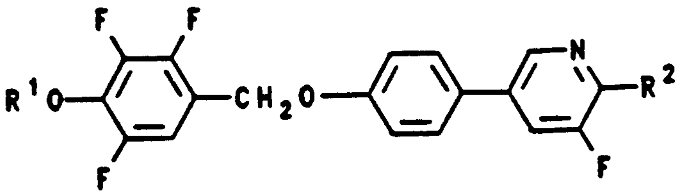

(I f 1 2) 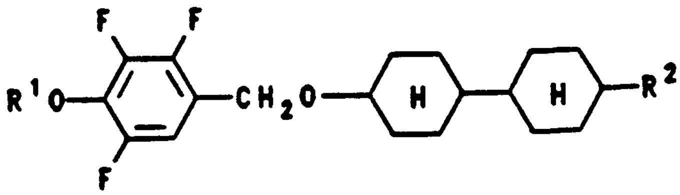

(I f 1 3) 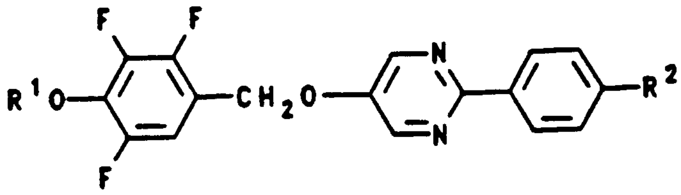

(I f 1 4) 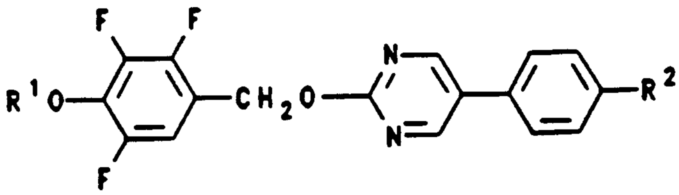

(I f 1 5) 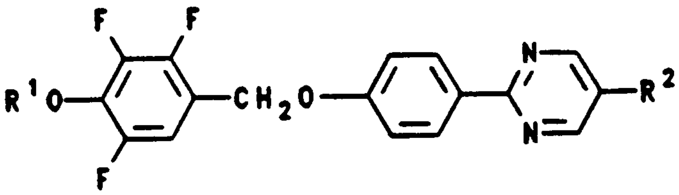

(I f 1 6) 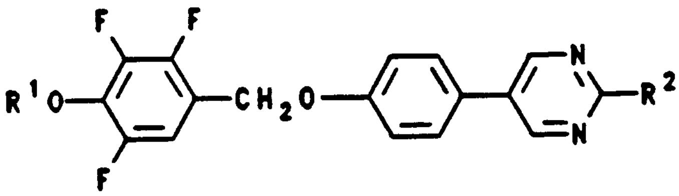

Bevorzugt werden ferner die Verbindungen der Teilstruktur

(Ig) 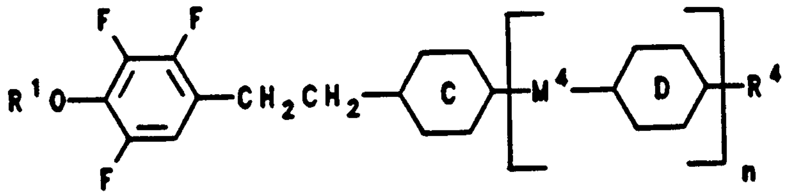

und darunter besonders bevorzugt die Verbindungen

(Ig1) 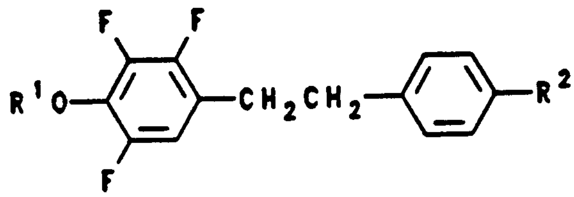

(Ig2) 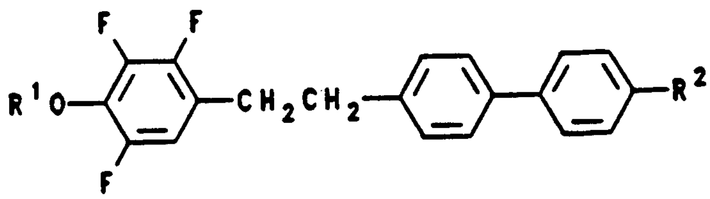

(Ig3) 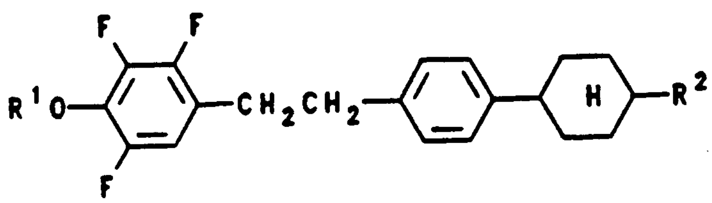

(Ig4) 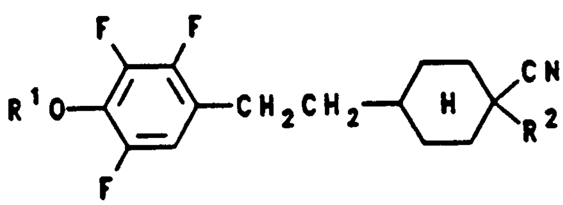

(Ig5) 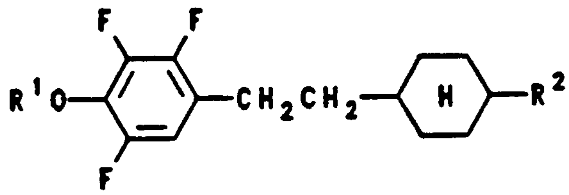

(Ig6) 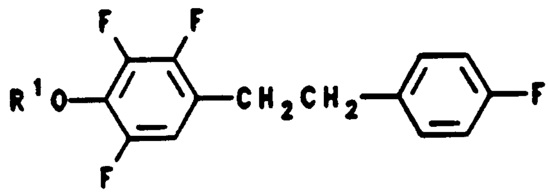

(Ig7) 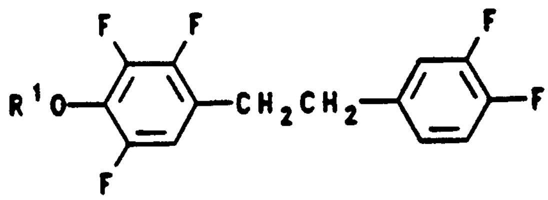

(Ig8) 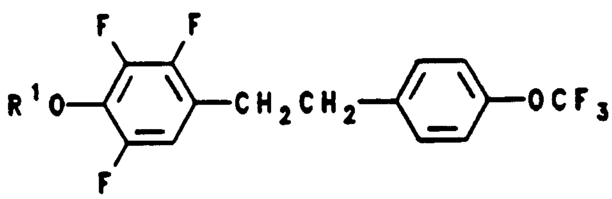

(Ig9) 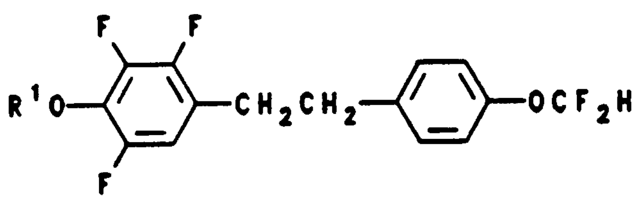

(Ig10) 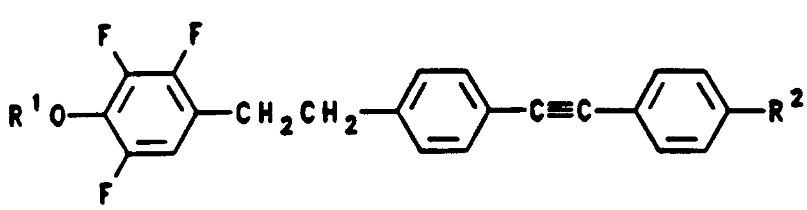

(Ig11) 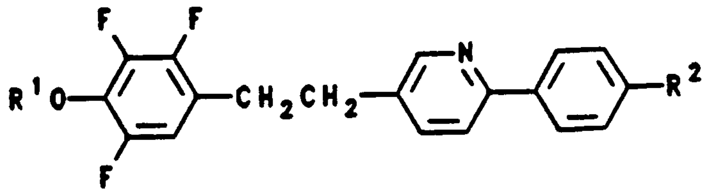

(Ig12) 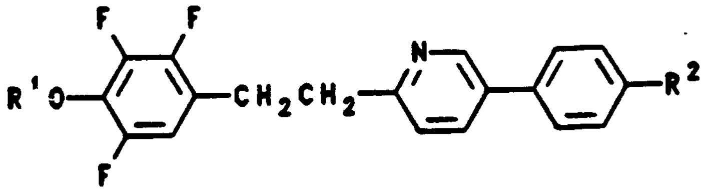

(Ig13) 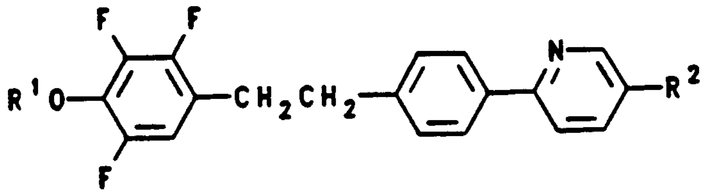

(Ig14) 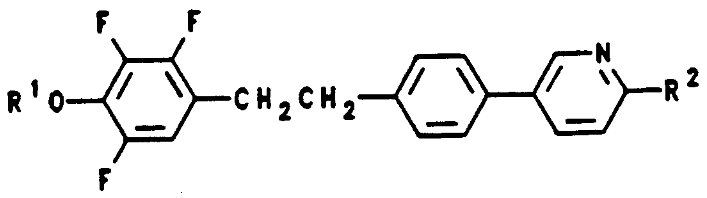

(Ig15) 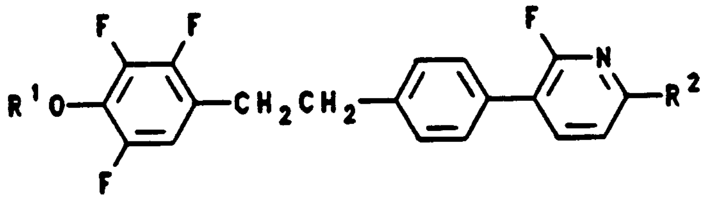

(Ig16) 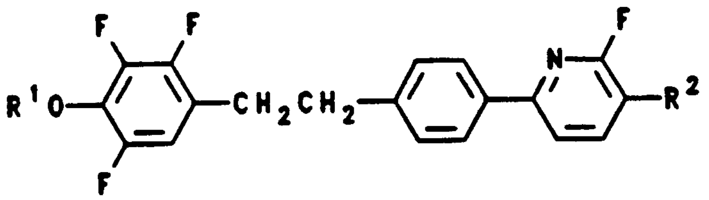

(Ig17) 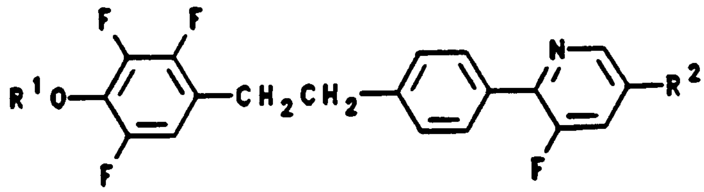

(Ig18) 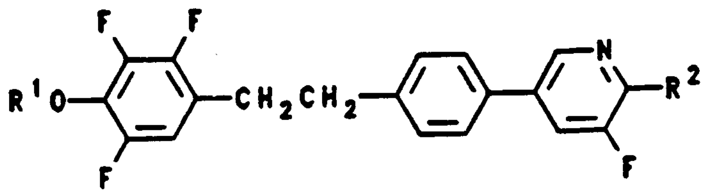

(Ig19) 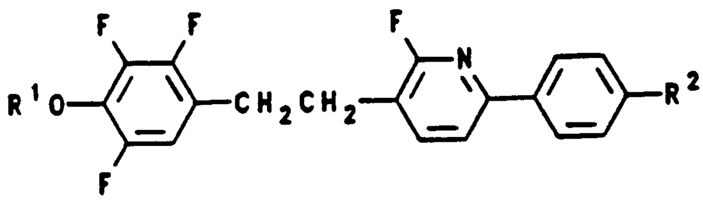

(Ig20) 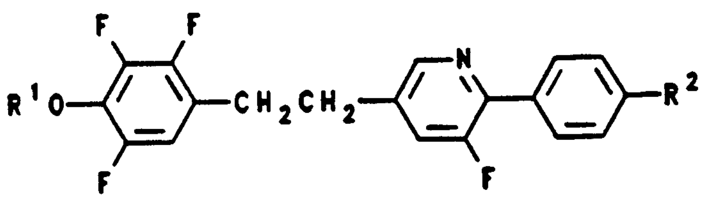

(Ig21) 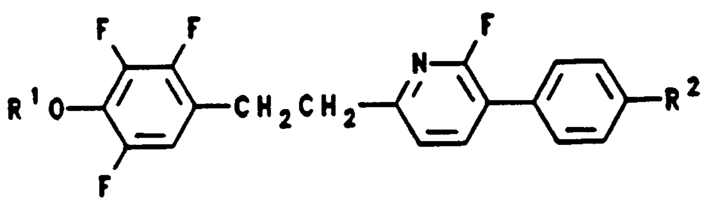

(Ig22) 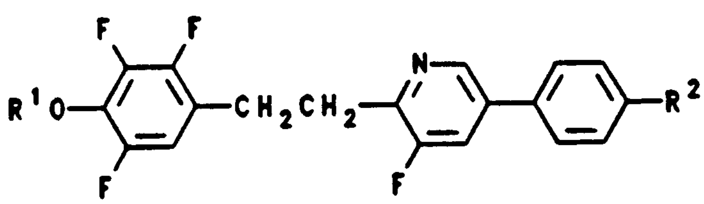

(Ig23) 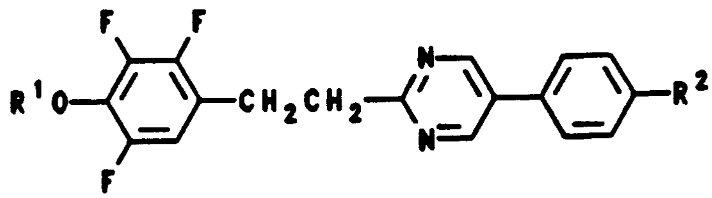

(Ig24) 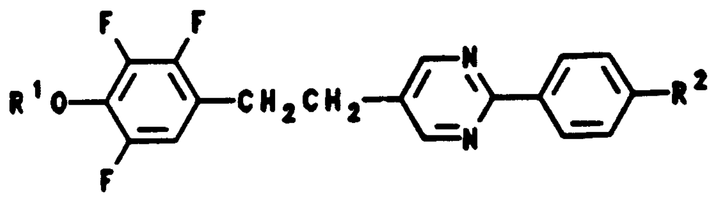

(Ig25) 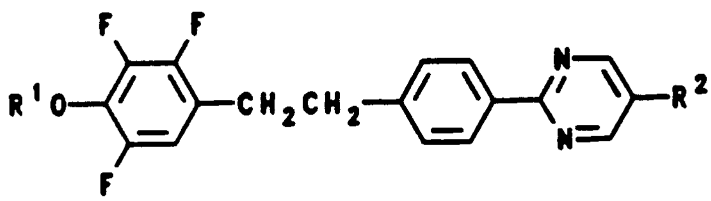

(Ig26) 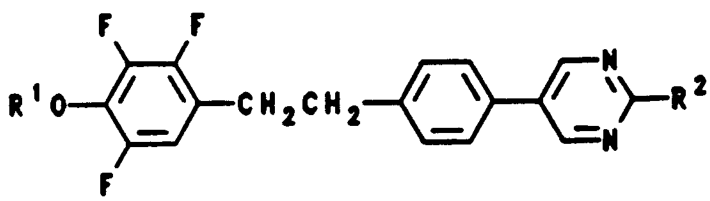

Bevorzugt werden ferner die Verbindungen der Teilstruktur

(Ih) 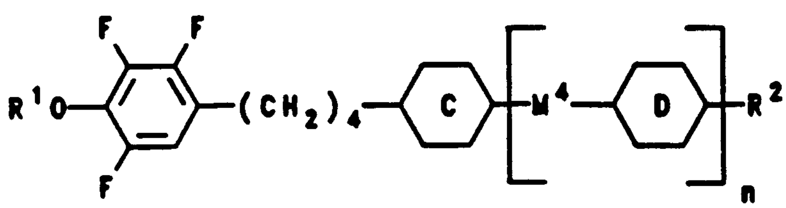

und darunter besonders bevorzugt die Verbindungen

(Ih1) 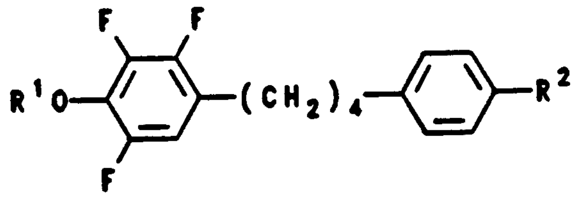

(Ih2) 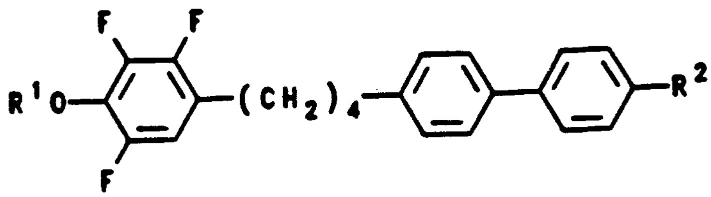

(Ih3) 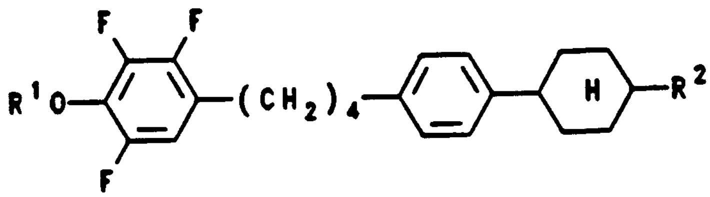

(Ih4) 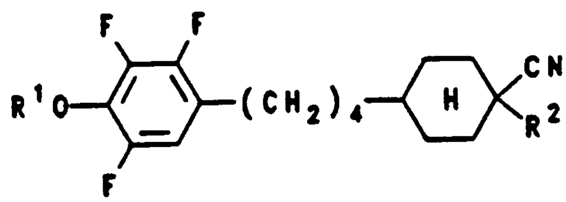

(Ih5) 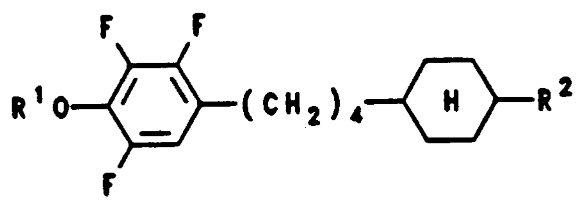

(Ih6) 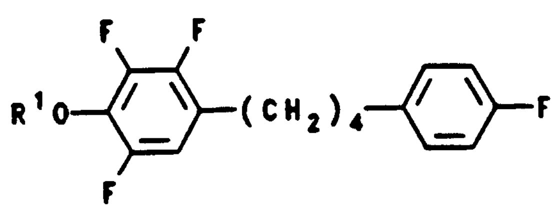

(Ih7) 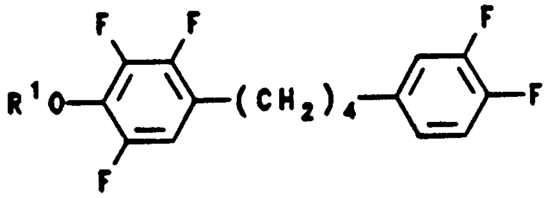

(Ih8) 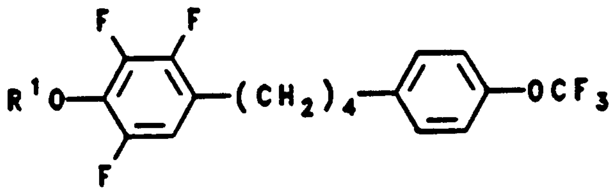

(Ih9) 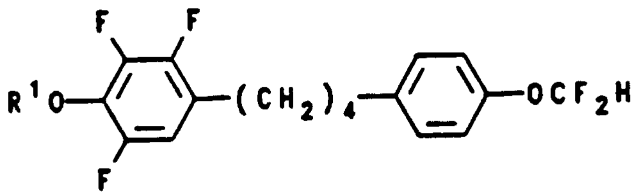

(Ih10) 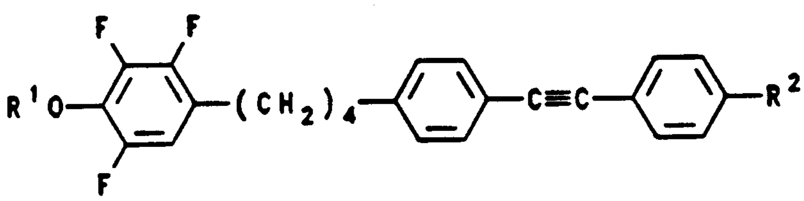

(Ih11) 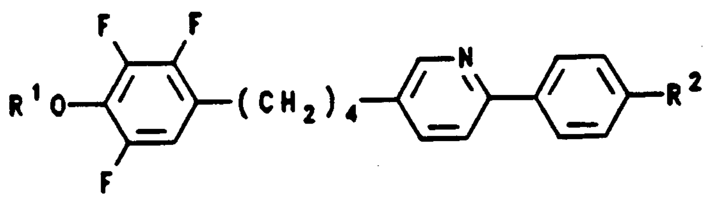

(Ih12) 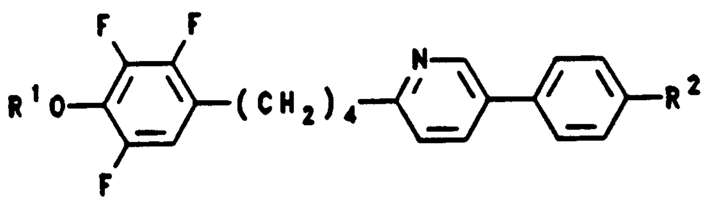

(Ih13) 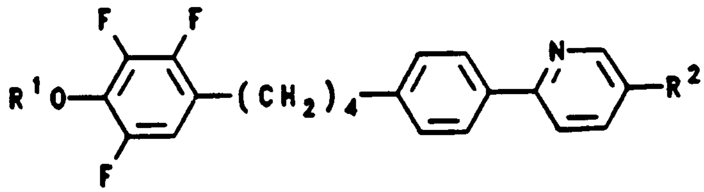

(Ih14) 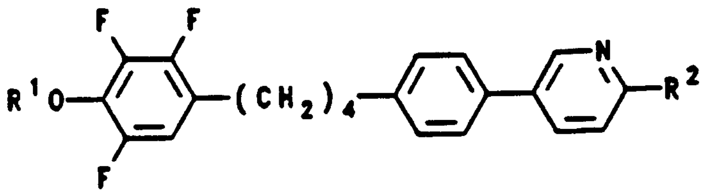

(Ih15) 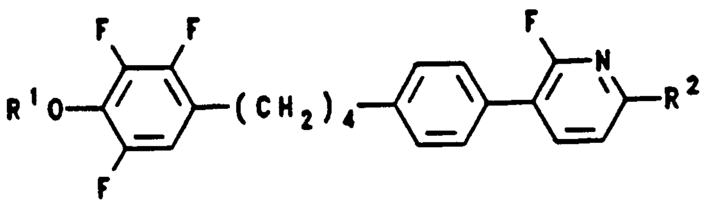

(Ih16) 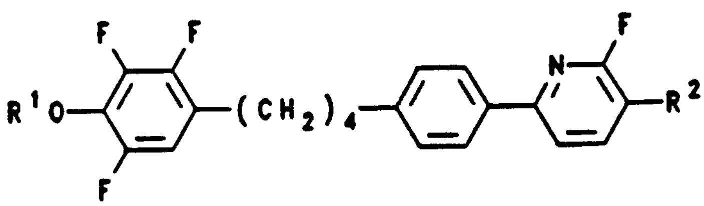

(Ih17) 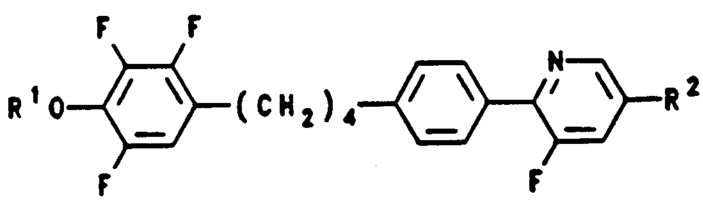

(Ih18) 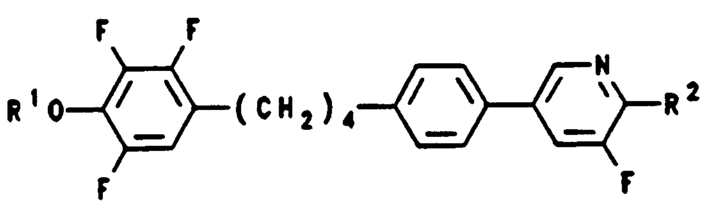

(Ih19) 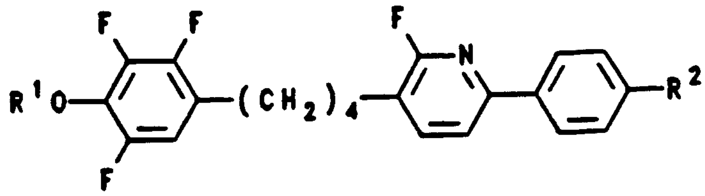

(Ih20) 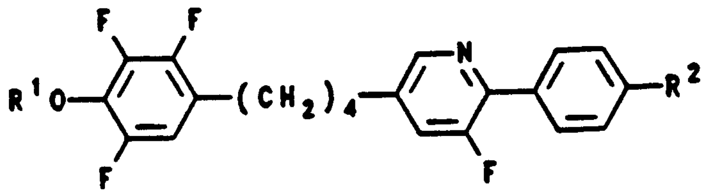

(Ih21) 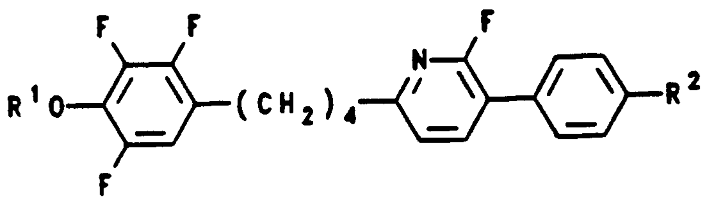

(Ih22) 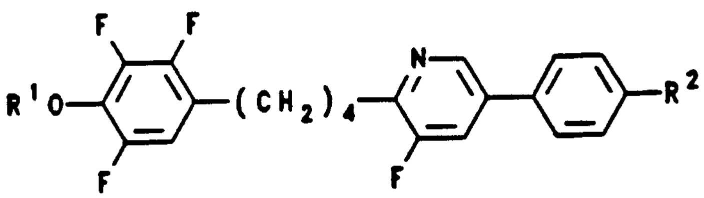

(Ih23) 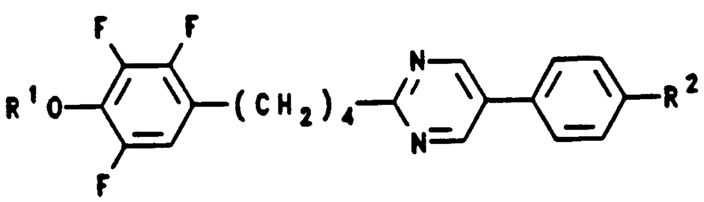

(Ih24) 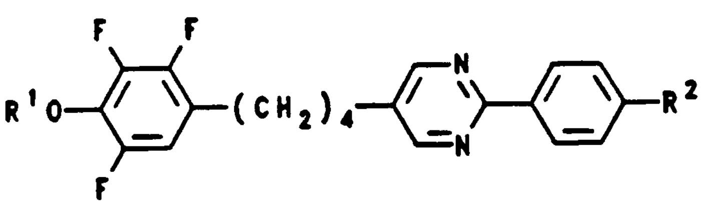

(Ih25) 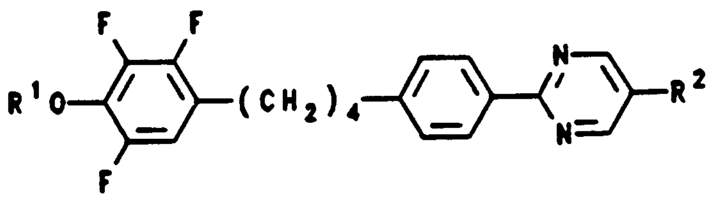

(Ih26) 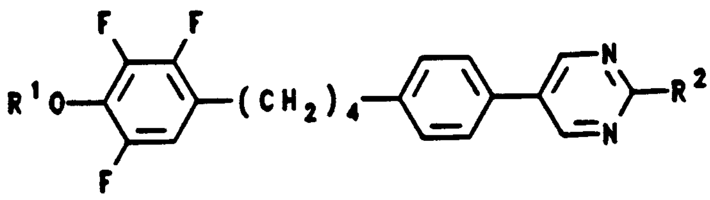

Bevorzugt werden ferner die Verbindungen der Teilstruktur

(I1) 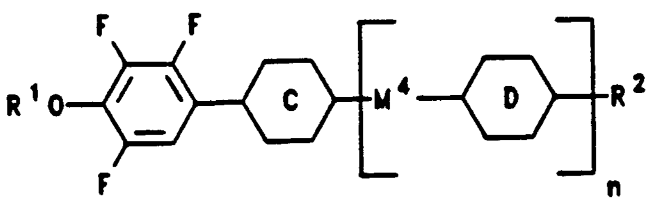

und darunter besonders bevorzugt die Verbindungen

(I11) 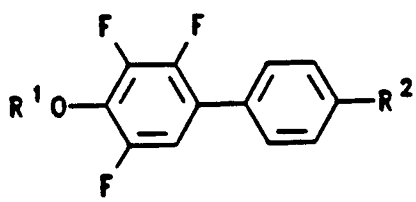

(I12) 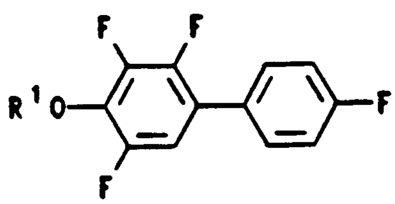

(113) 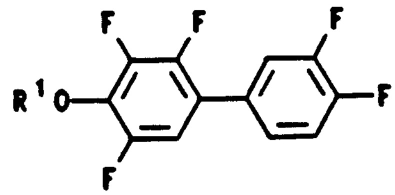

(114) 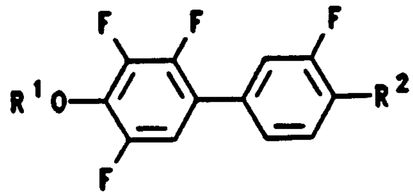

(115) 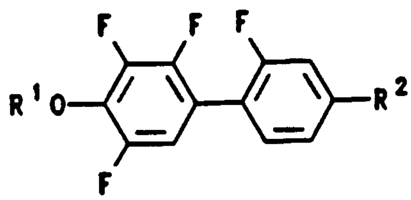

(116) 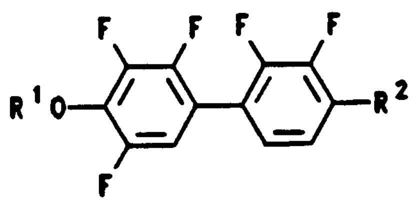

(117) 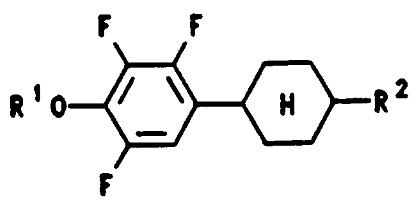

(118) 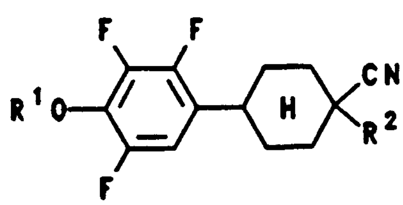

(119) 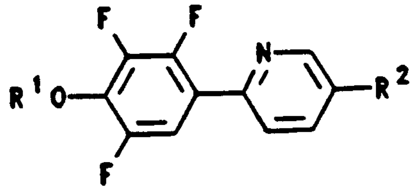

(1110) 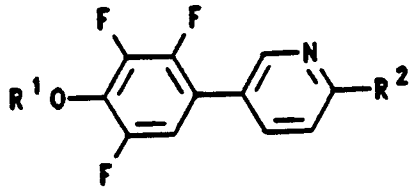

(1111) 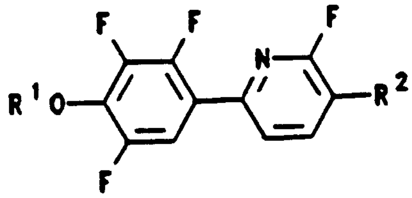

(1112) 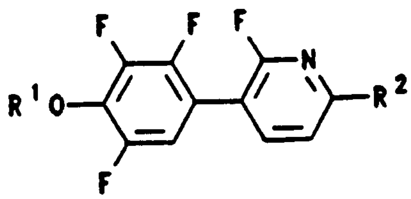

(1113) 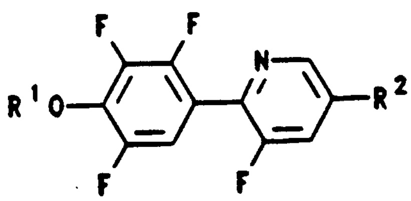

(1114) 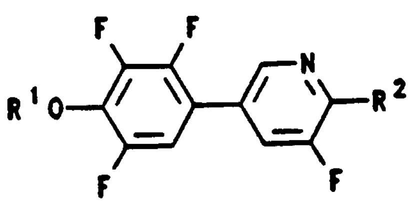

(1115) 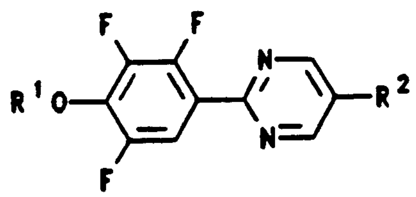

(1116) 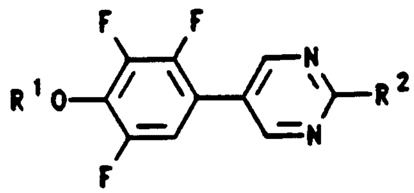

(1117) 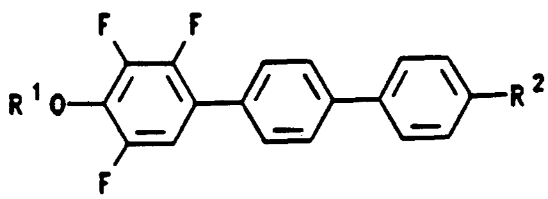

(1118) 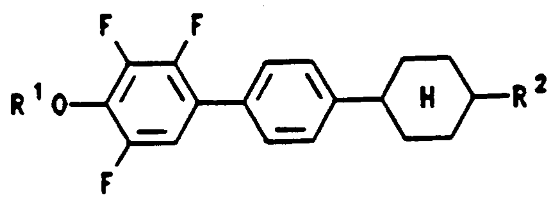

(1119) 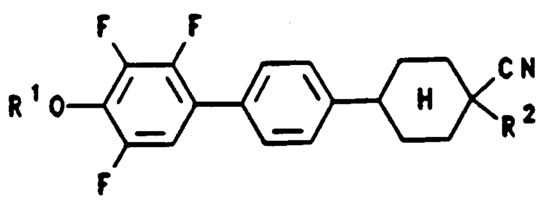

(1120) 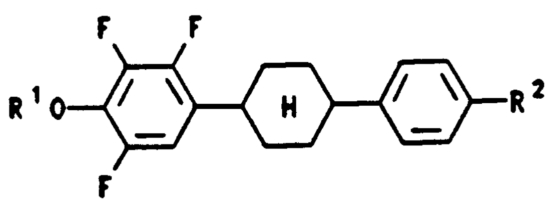

(1121) 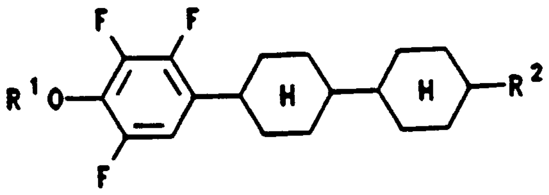

(1122) 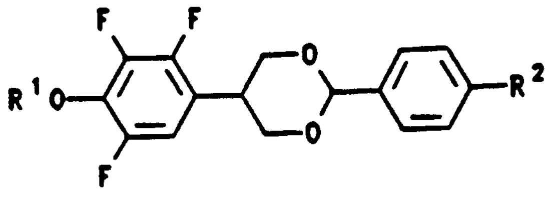

(1123) 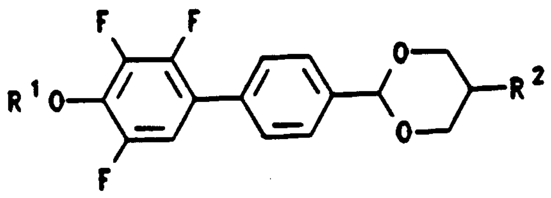

(1124) 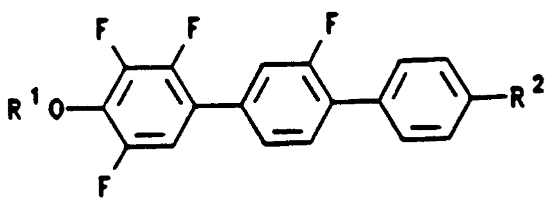

(1125) 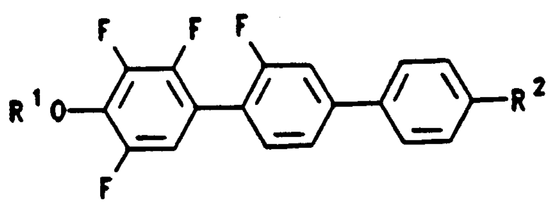

(1126) 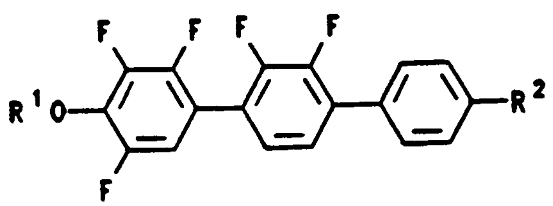

(1127)

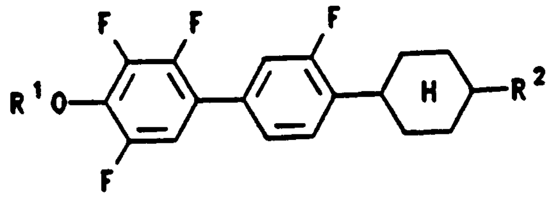

(1128)

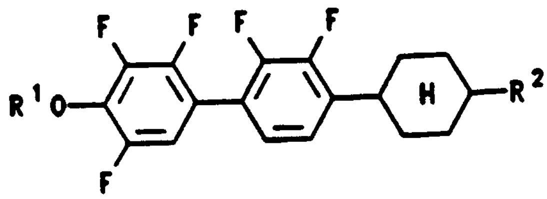

(1129)

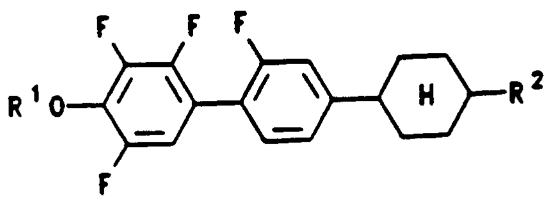

(1130)

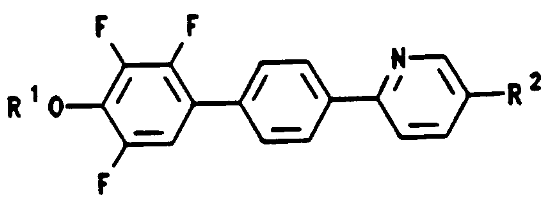

(1131)

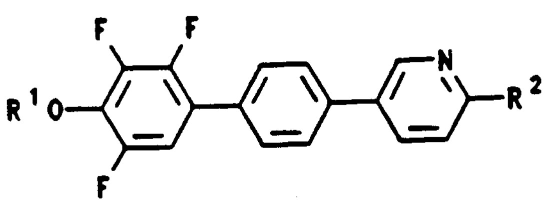

(1132)

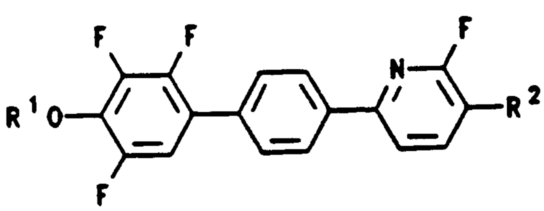

(1133) 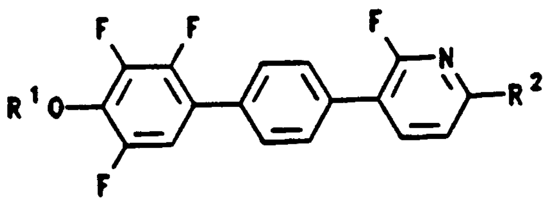

(1134) 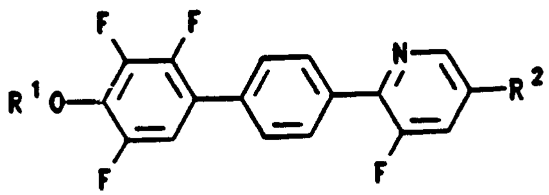

(1135) 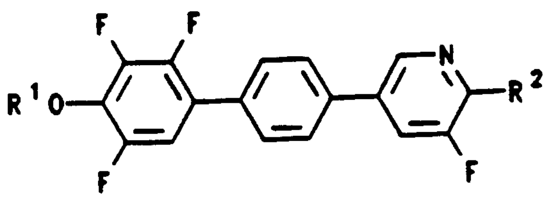

(1136) 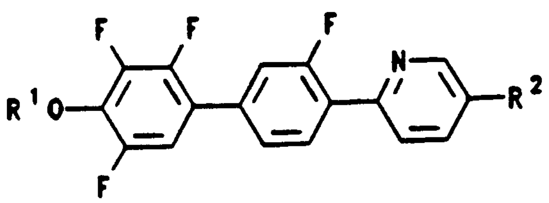

(1137) 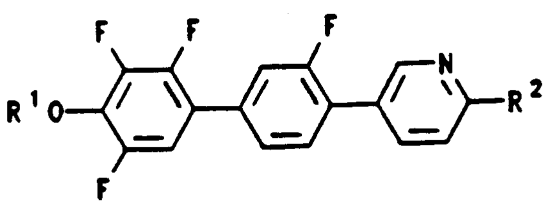

(1138) 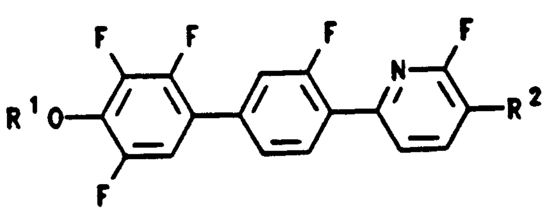

(Ii39) 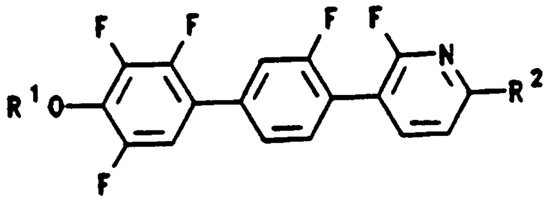

(Ii40) 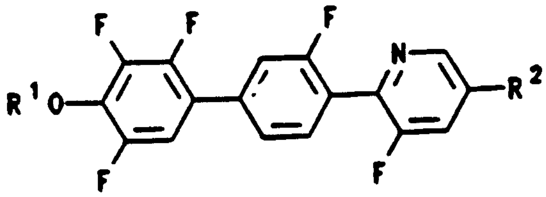

(Ii41) 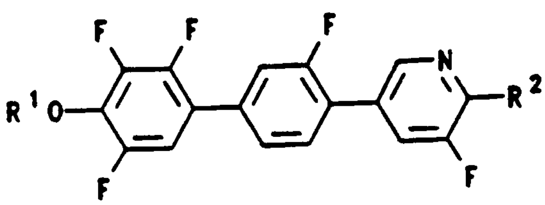

(Ii42) 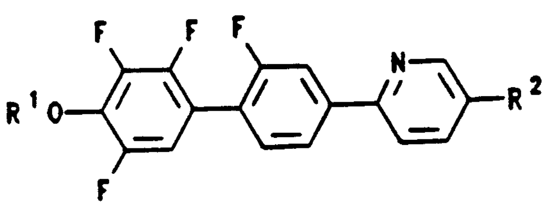

(Ii43) 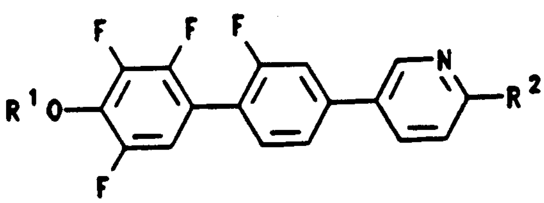

(Ii44) 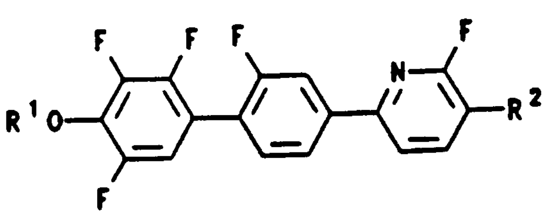

(1145) 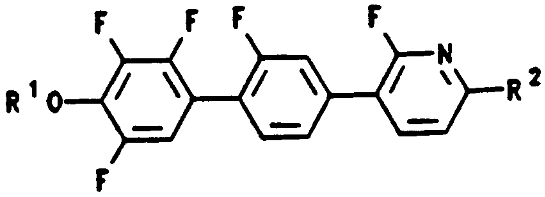

(1146) 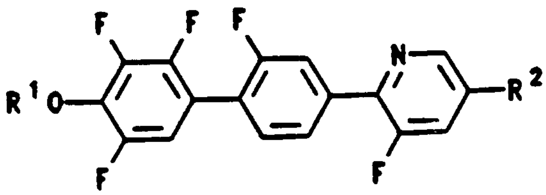

(1147) 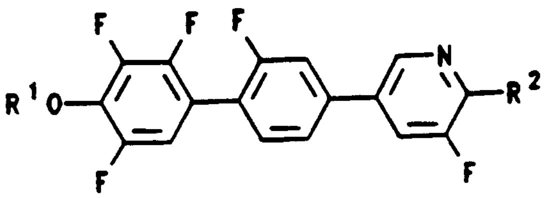

(1148) 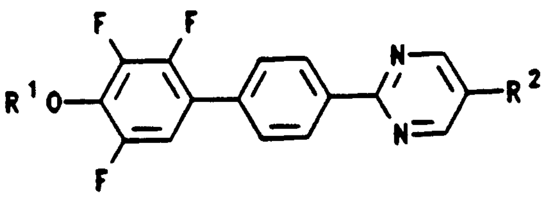

(1149) 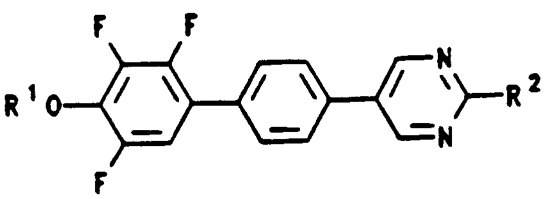

(1150) 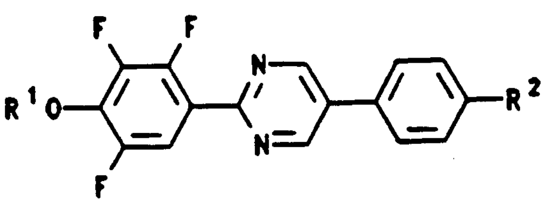

(1151) 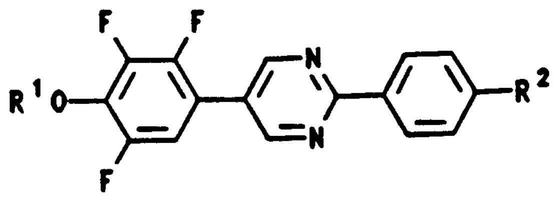

(1152) 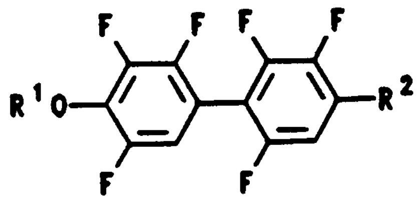

(1153) 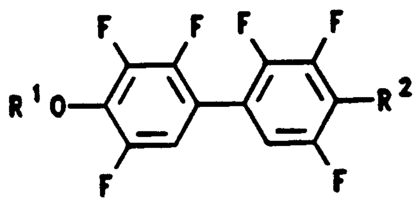

Bevorzugt werden ferner die Verbindungen der Teilstruktur

(I1) 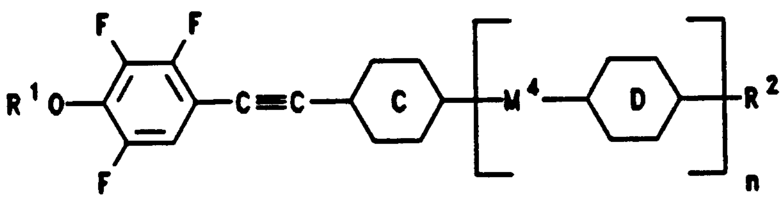

und darunter besonders bevorzugt die Verbindungen

(Il1)

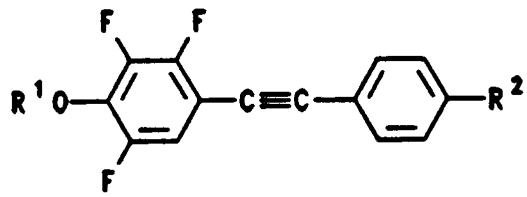

(Il2)

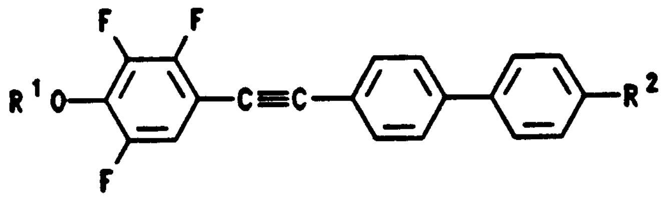

(Il3)

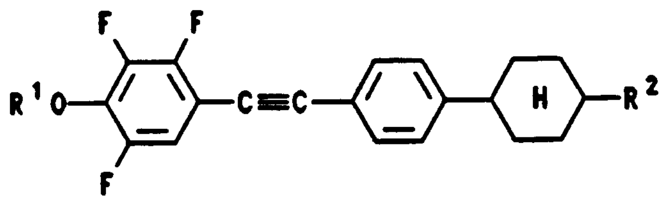

Bevorzugt werden ferner die Verbindungen der Teilstruktur

(Ik)

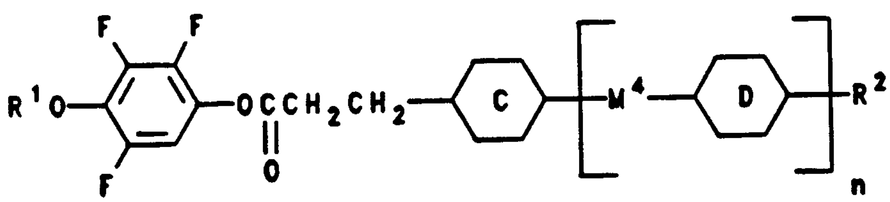

und darunter besonders bevorzugt die Verbindungen

(Ik1)

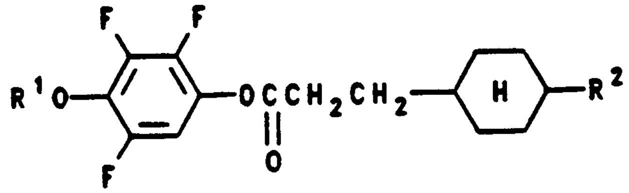

(Ik2)

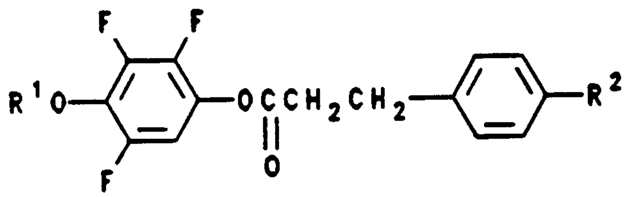

(Ik3)

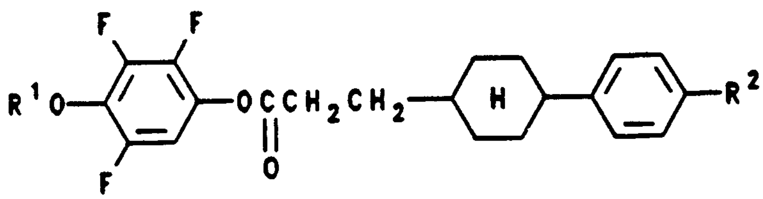

(Ik4)

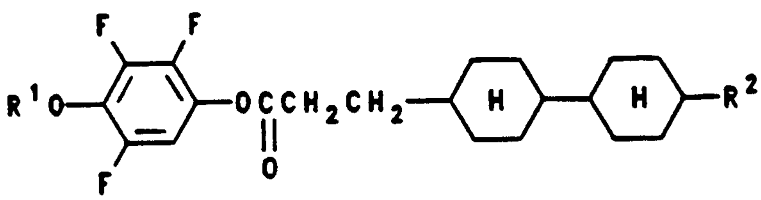

Bevorzugt werden ferner die Verbindungen der Teilstruktur

(Il)

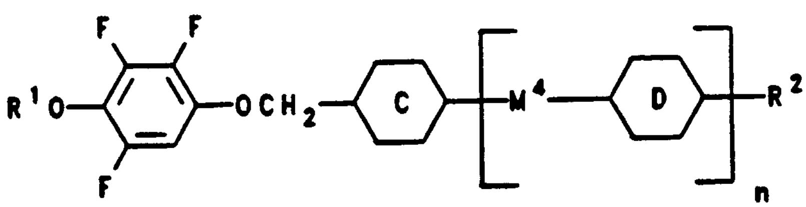

und darunter besonders bevorzugt die Verbindungen

(111) 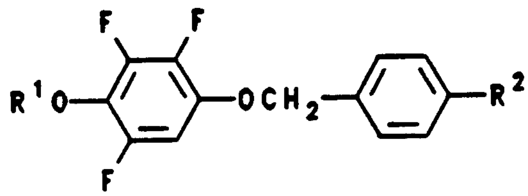

(112) 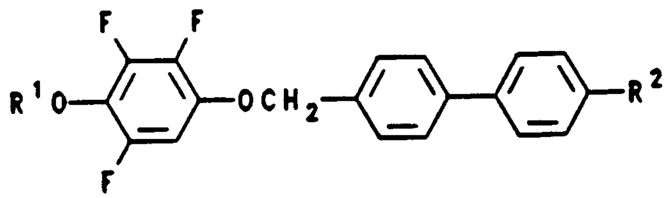

(113) 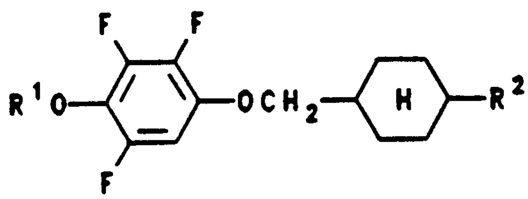

(114) 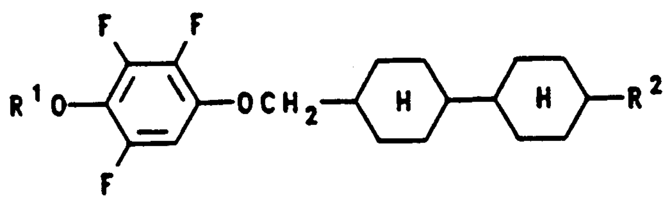

(115) 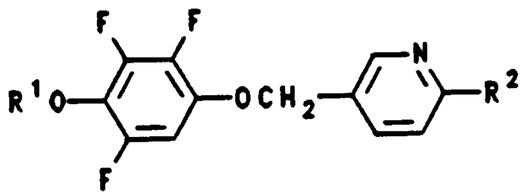

(116) 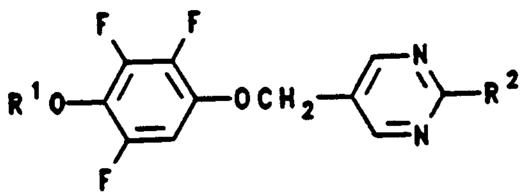

(117) 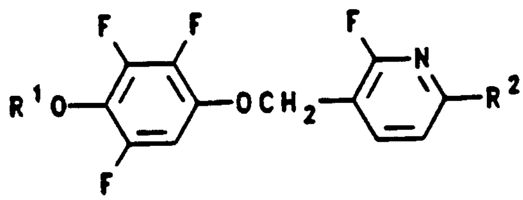

(118) 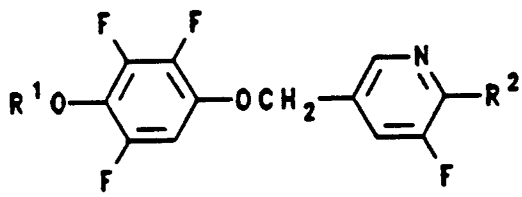

(119) 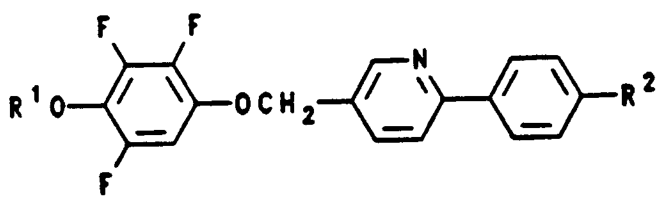

(1110) 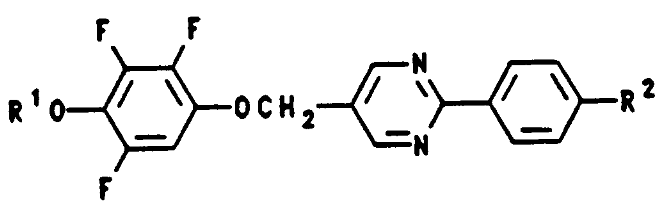

(1111) 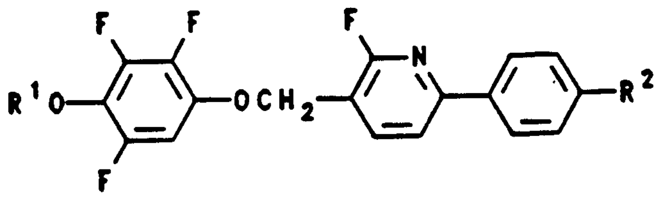

(1112) 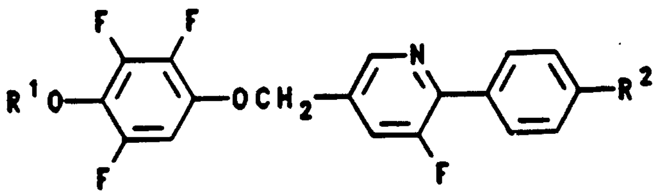

(1113) 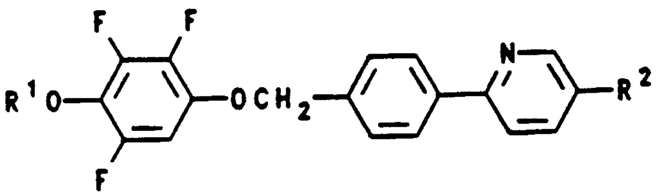

(1114) 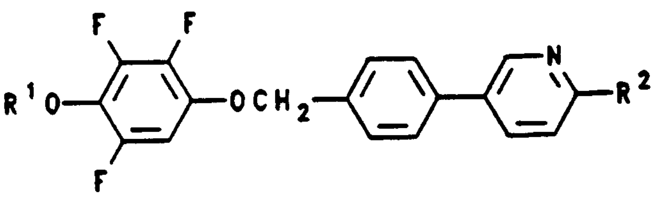

(1115) 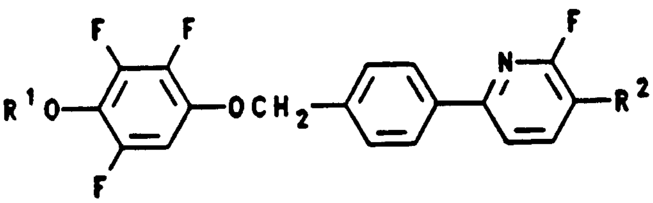

(1116) 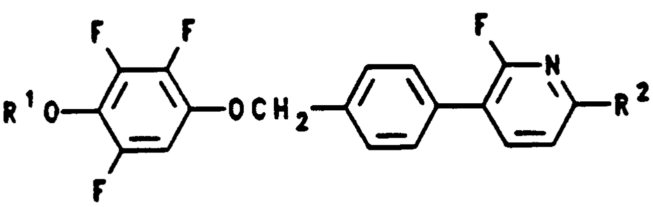

(1117) 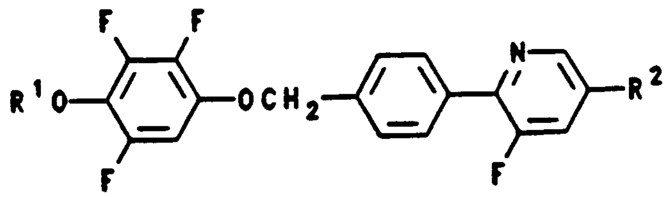

(1118) 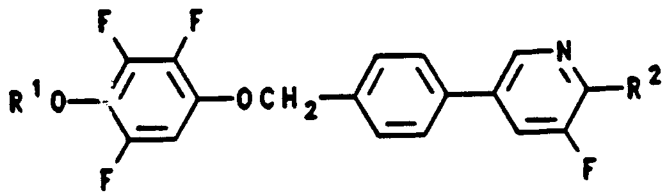

(1119) 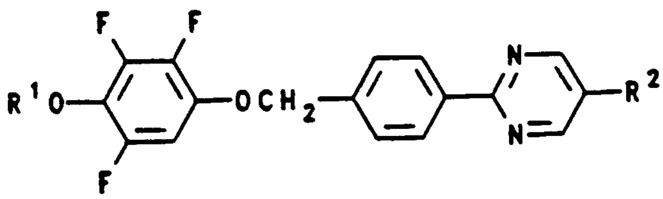

(1120) 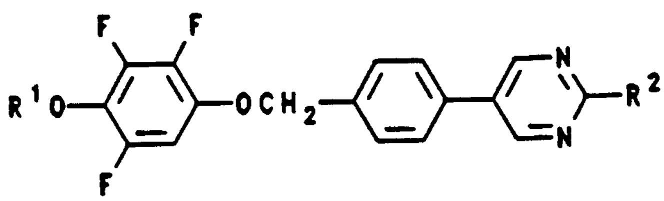

Bevorzugt werden ferner die Verbindungen der Teilstruktur

(Im) 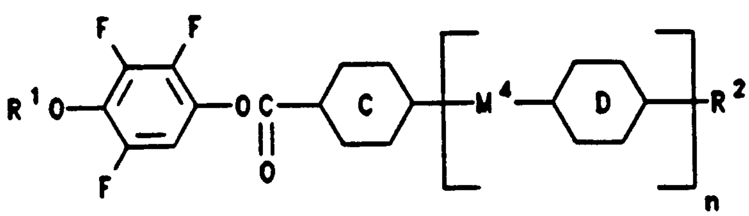

und darunter besonders bevorzugt die Verbindungen

(Im1) 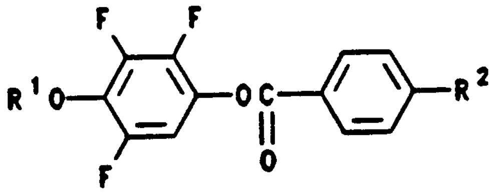

(Im2) 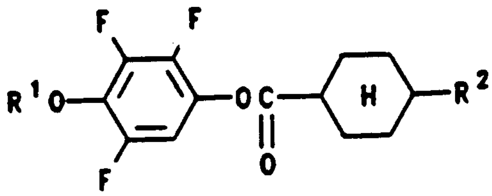

(Im3) 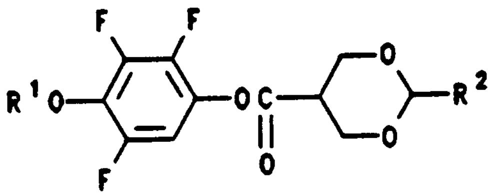

(Im4) 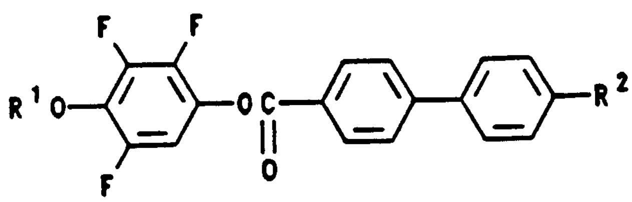

(Im5) 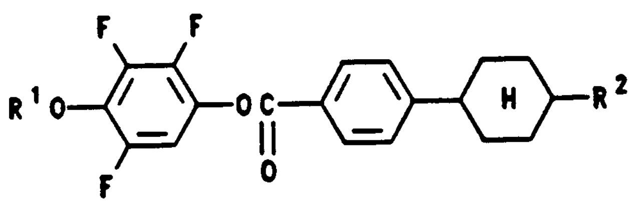

(Im6) 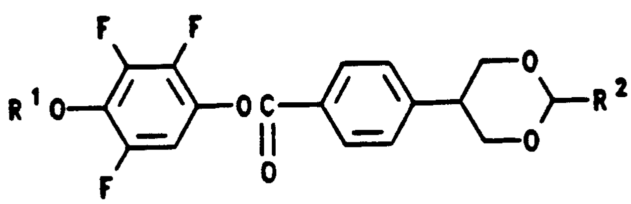

(Im7) 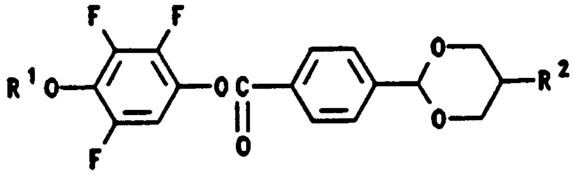

(Im8) 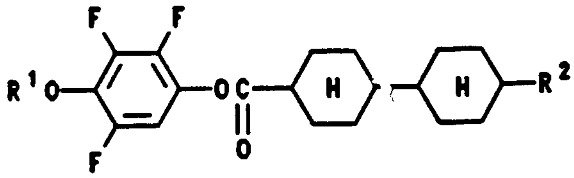

(Im9) 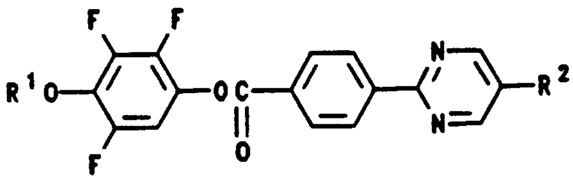

(Im10) 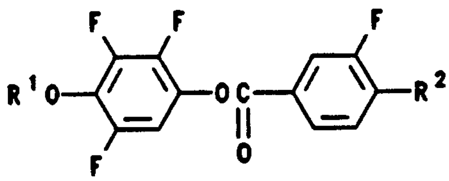

(Im11) 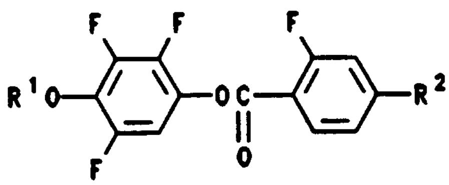

(Im12) 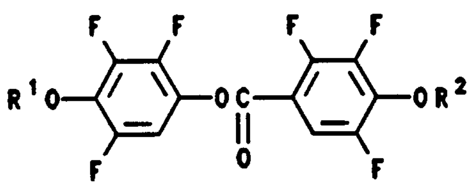

(Im13) 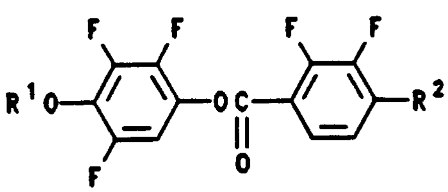

(Im14) 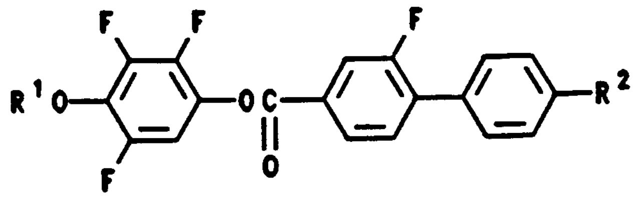

(Im15) 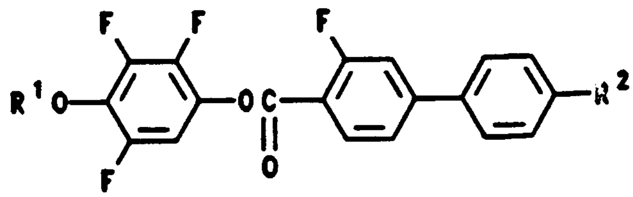

(Im16) 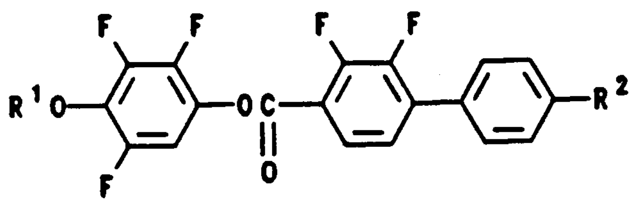

Bevorzugt werden ferner die Verbindungen der Teilstruktur

(In) 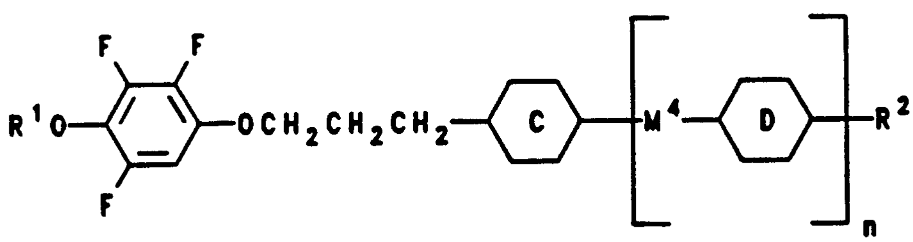

und darunter besonders bevorzugt die Verbindungen

(In1) 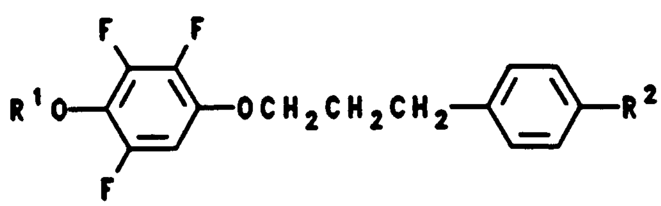

(In2) 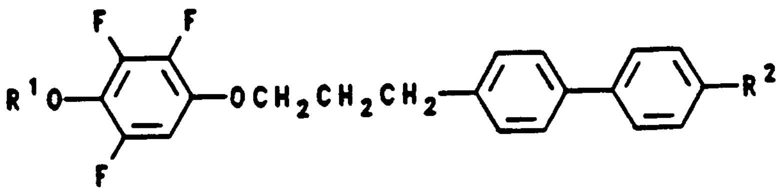

(In3) 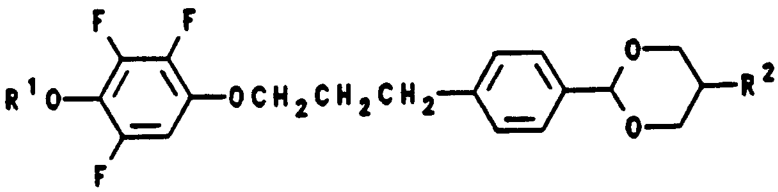

(In4) 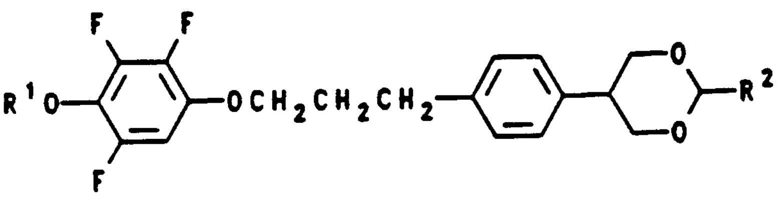

(In5) 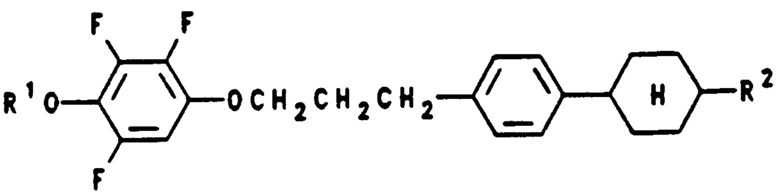

(In6) 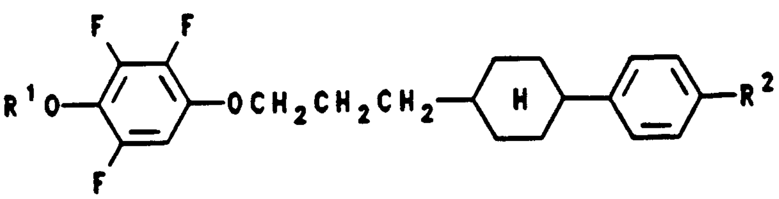

(In7) 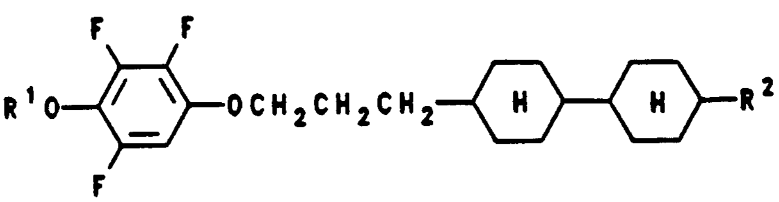

(In8) 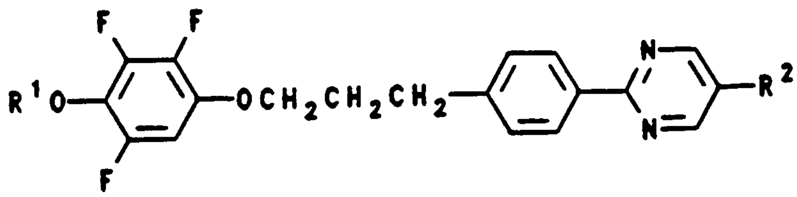

(In9) 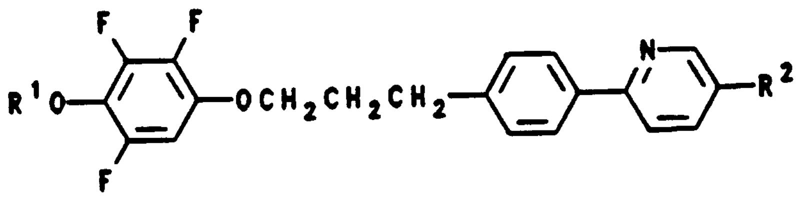

(In10) 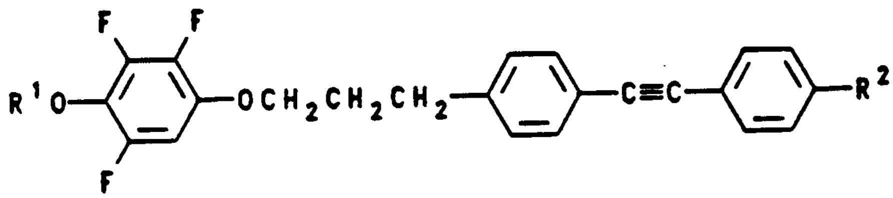

Bevorzugt werden ferner die Verbindungen der Teilstruktur

(Io) 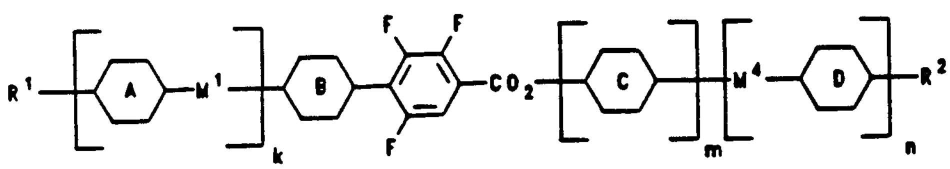

und darunter besonders bevorzugt die Verbindungen

(Io1) 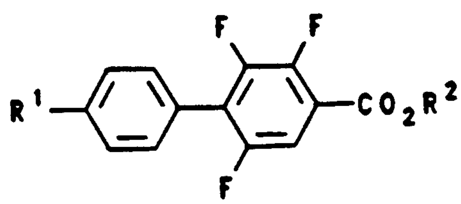

(102) 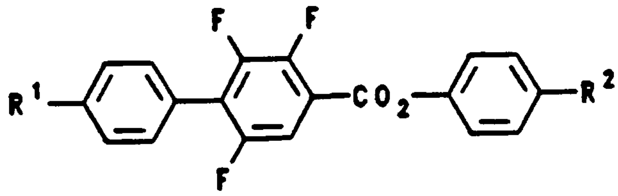

(103) 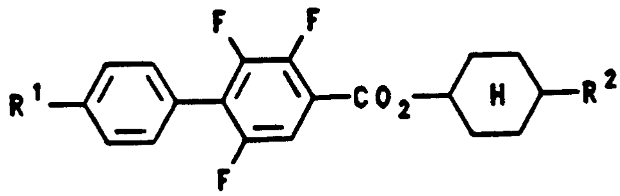

(104) 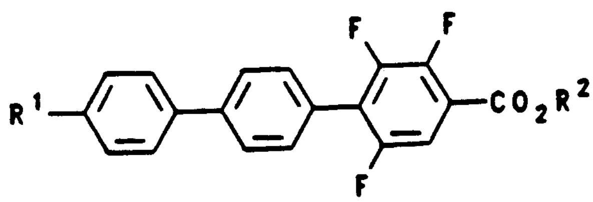

(105) 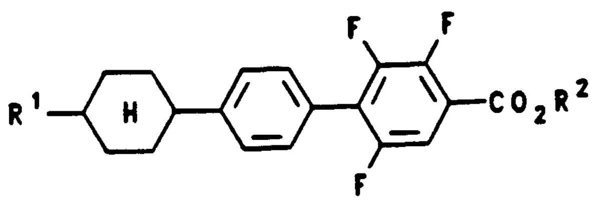

(106) 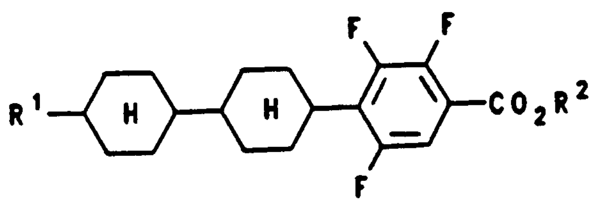

(107) 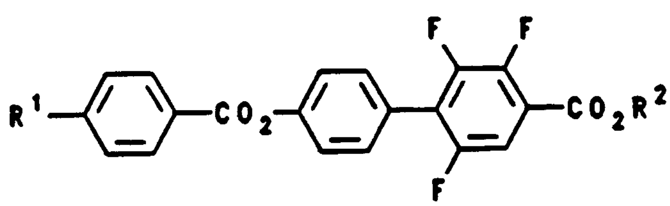

(Io8) 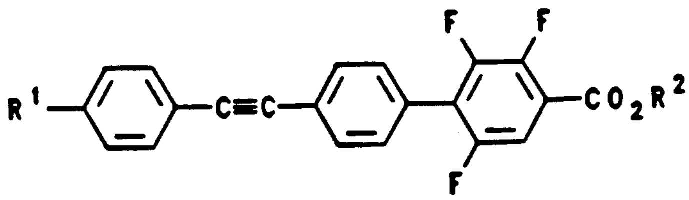

(Io9) 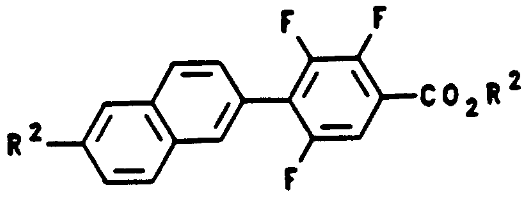

Bevorzugt werden ferner die Verbindungen der Teilstruktur (Ip)

(Ip) 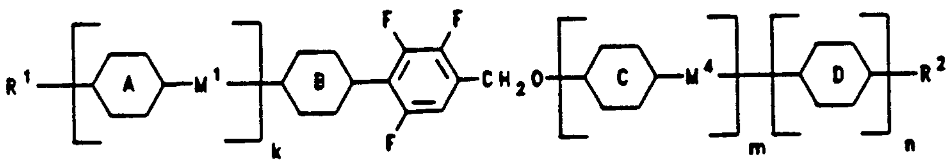

und darunter besonders bevorzugt die Verbindungen

(Ip1) 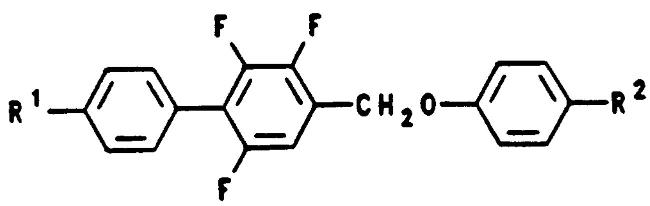

(Ip2) 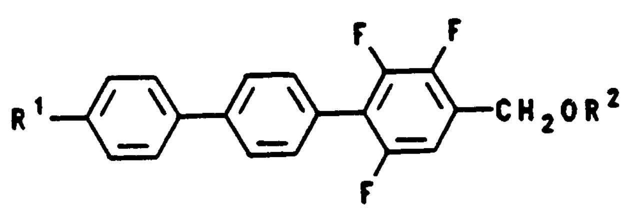

(Ip3) 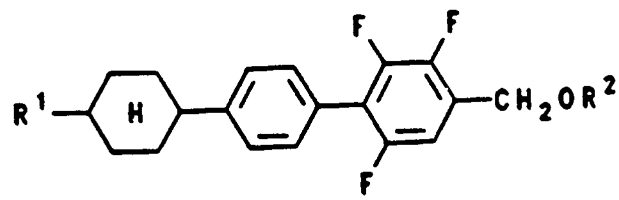

(Ip4) 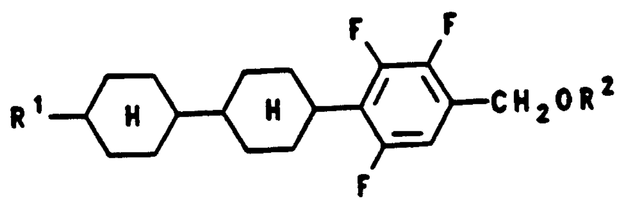

(Ip5) 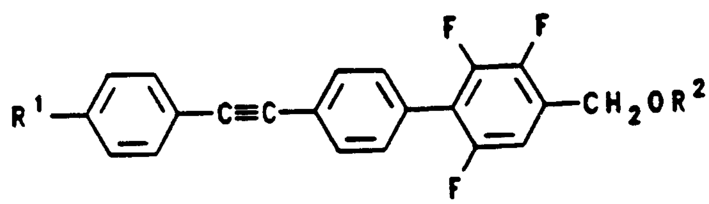

(Ip6) 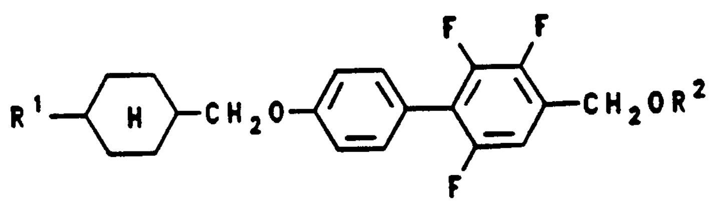

(Ip7) 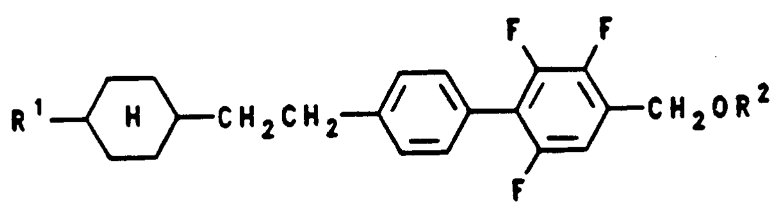

(Ip8) 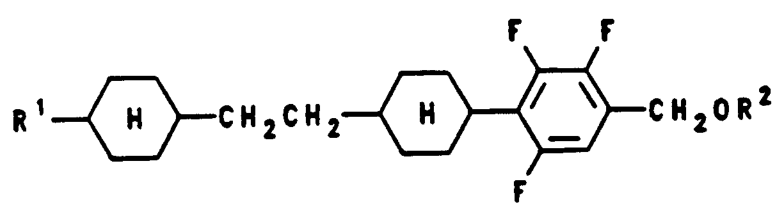

(Ip9) 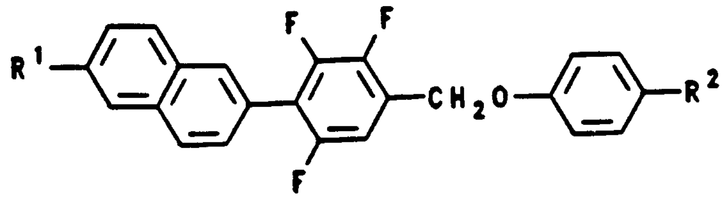

Bevorzugt werden ferner die Verbindungen der Teilstrukturen (Iq)

(Iq) 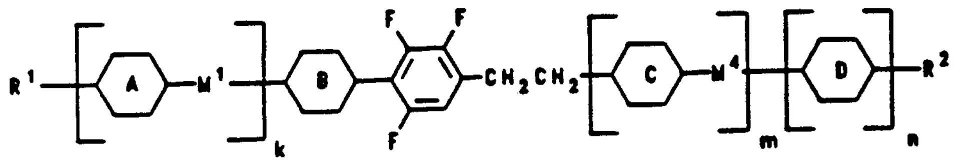

und darunter besonders bevorzugt die Verbindungen

(Iq1) 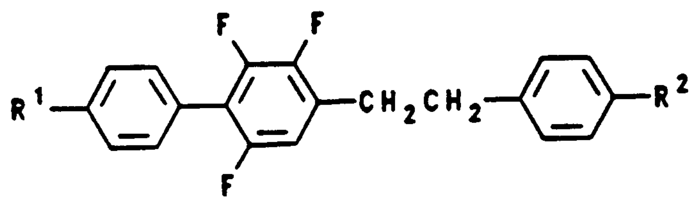

(Iq2) 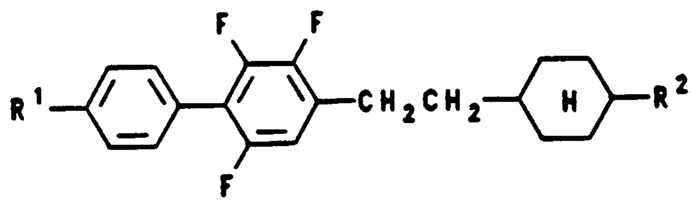

(Iq3) 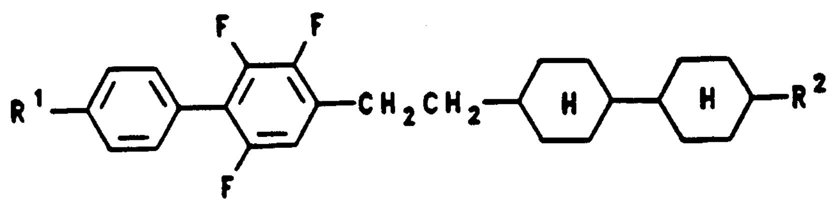

(Iq4) 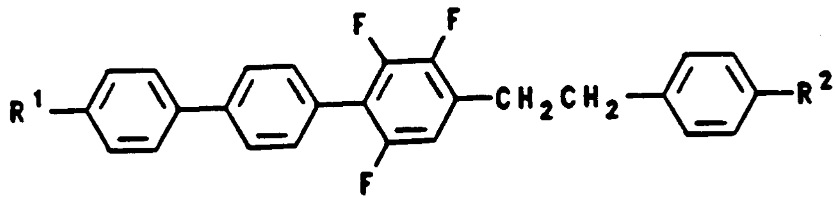

(Iq5) 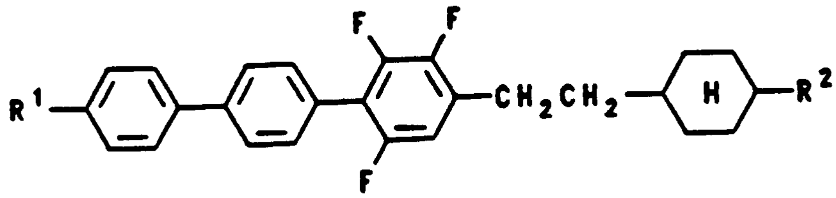

(Iq6) 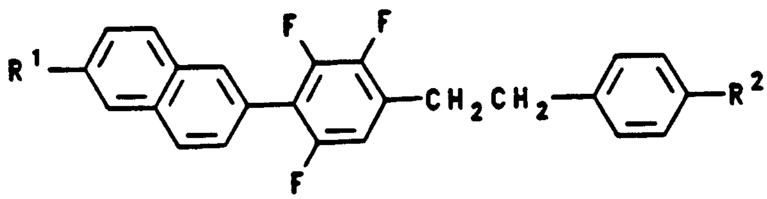

Bevorzugt werden ferner die Verbindungen der Teilstruktur

(Ir) 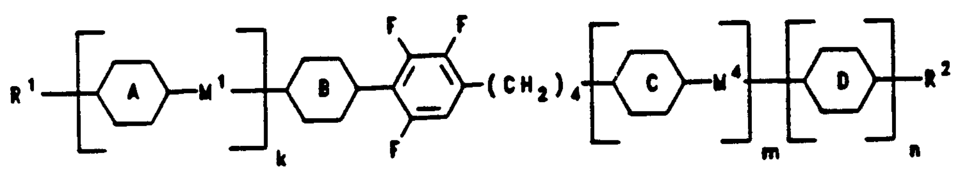

und darunter besonders bevorzugt die Verbindungen

(Ir1) 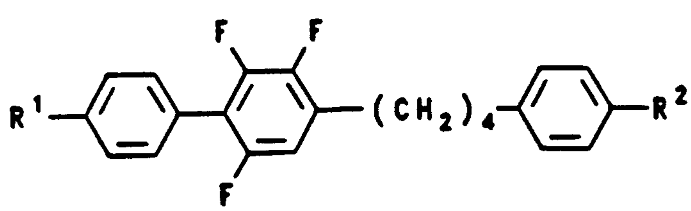

(Ir2)

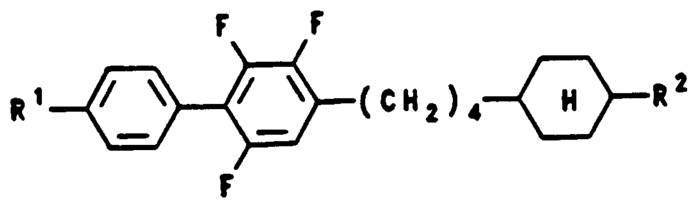

(Ir3)

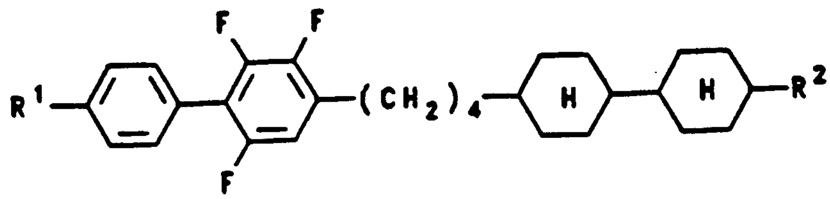

(Ir4)

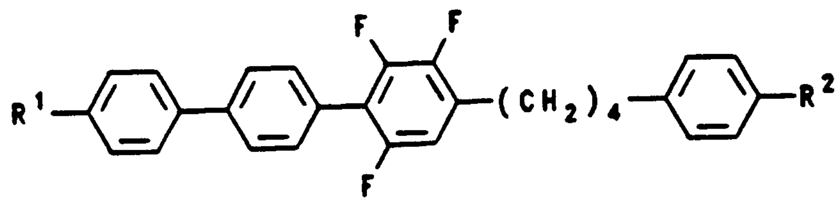

Bevorzugt werden ferner die Verbindungen der Teilstruktur

(Is)

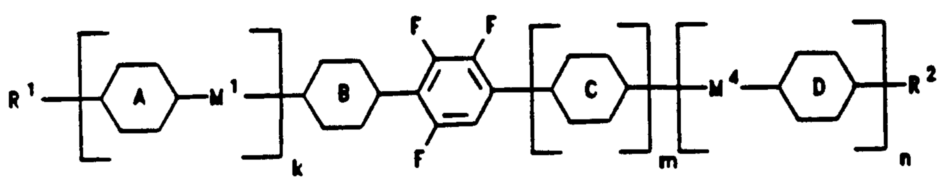

und darunter besonders bevorzugt die Verbindungen

(Is1)

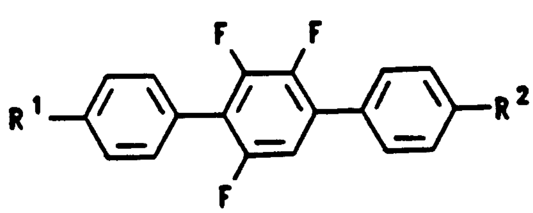

(Is2) 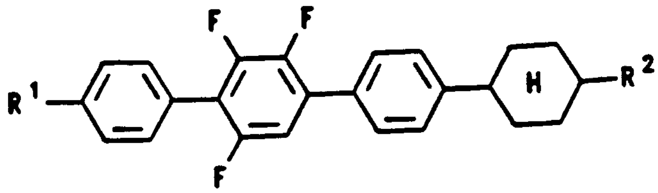

(Is3) 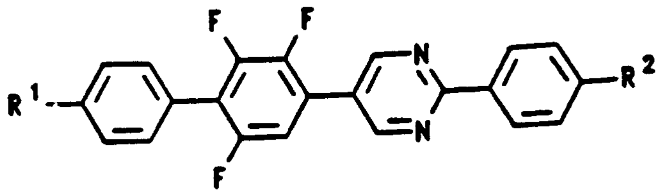

(Is4) 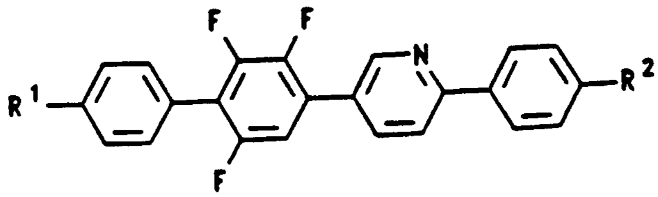

(Is5) 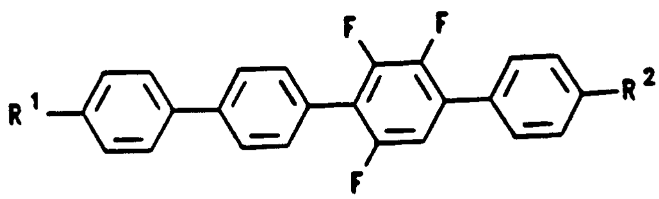

(Is6) 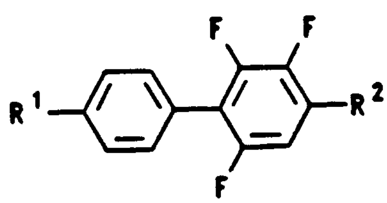

(Is7) 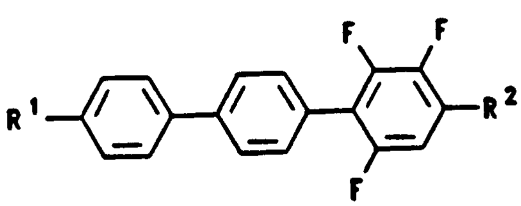

(Is8)

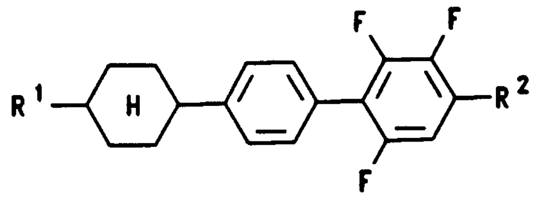

(Is9)

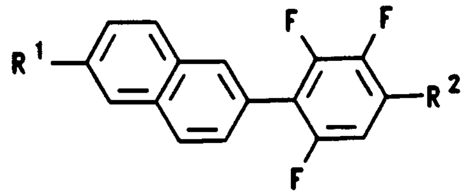

(Is10)

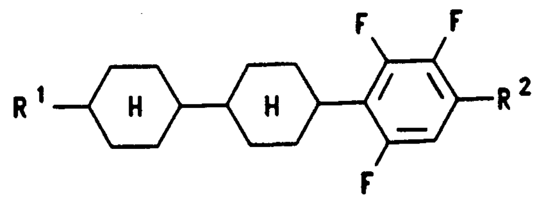

Bevorzugt werden ferner die Verbindungen der Teilstruktur

(It)

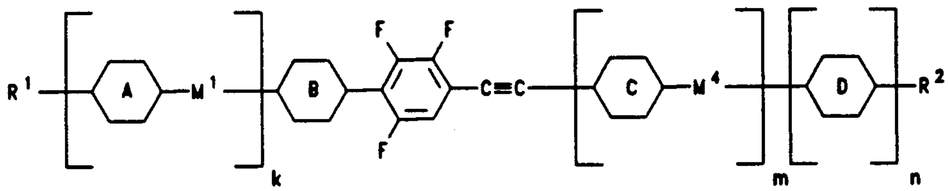

und darunter besonders bevorzugt die Verbindungen

(It1)

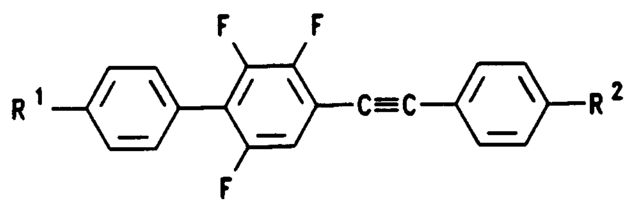

(It2)

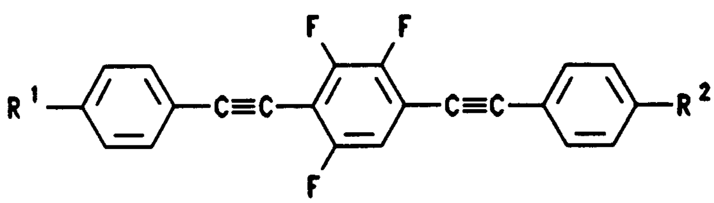

(I t 3) 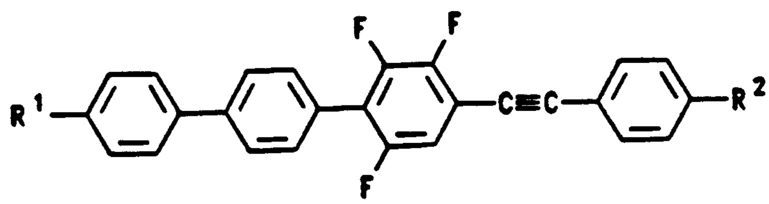

(I t 4) 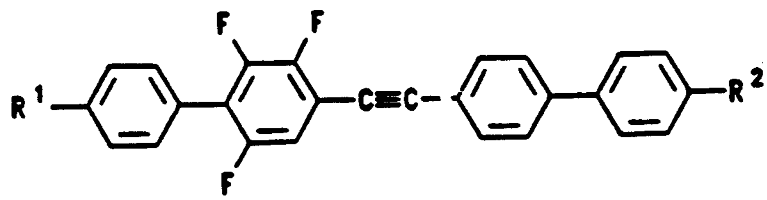

(I t 5) 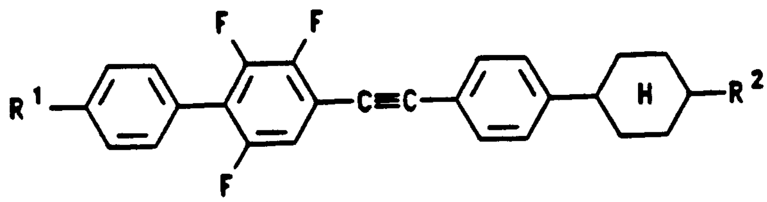

Bevorzugt werden ferner die Verbindungen der Teilstruktur

(I u) 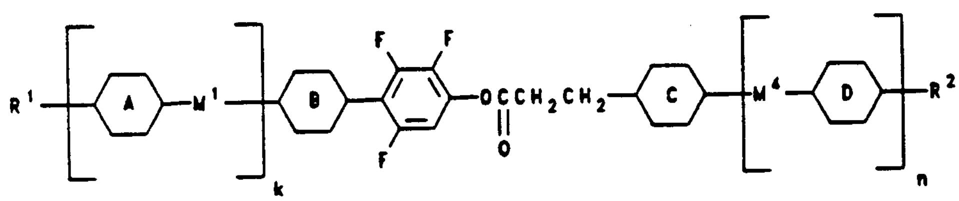

und darunter besonders bevorzugt die Verbindungen

(I u 1) 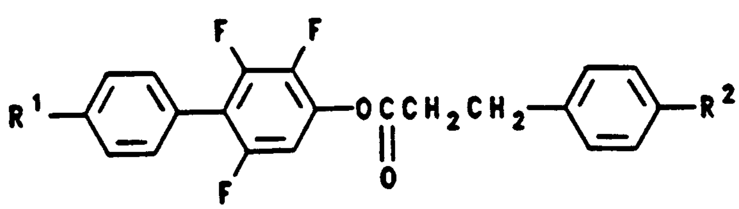

(Iu2)

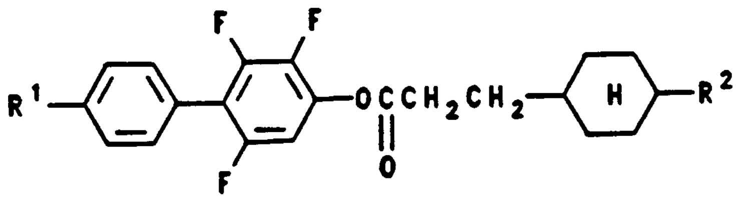

(Iu3)

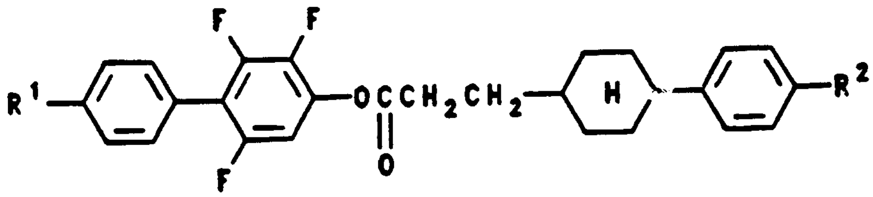

(Iu4)

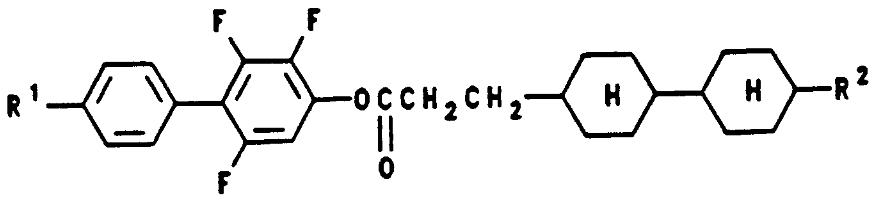

Bevorzugt werden ferner die Verbindungen der Teilstruktur

(Iv)

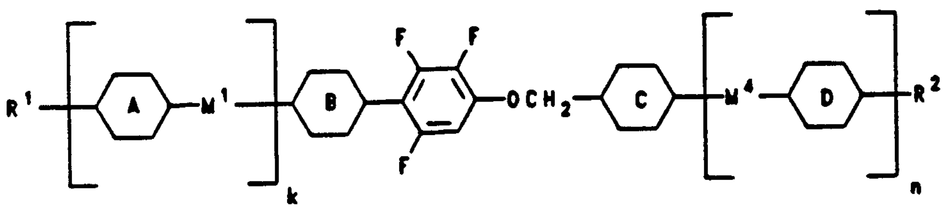

und darunter besonders bevorzugt die Verbindungen

(Iv1)

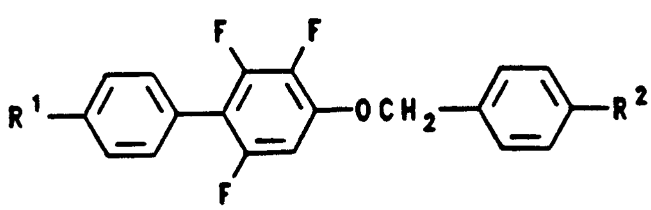

(Iv2)

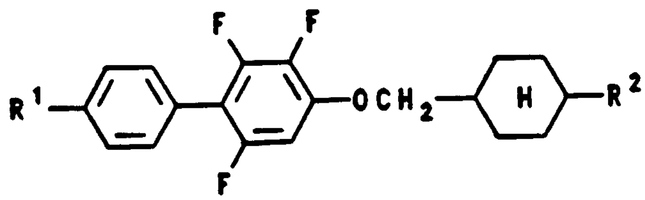

(Iv3)

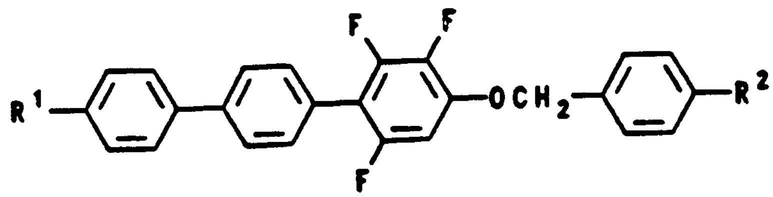

(Iv4)

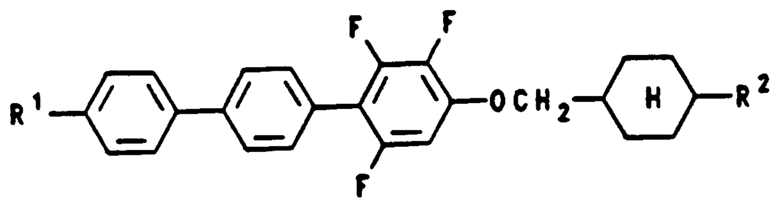

(Iv5)

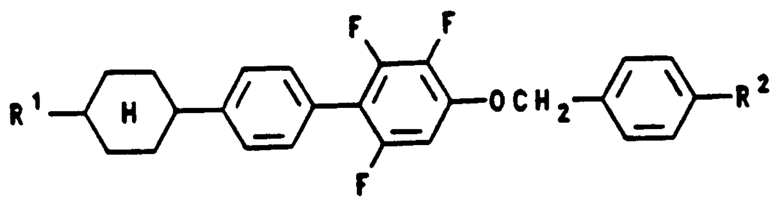

(Iv6)

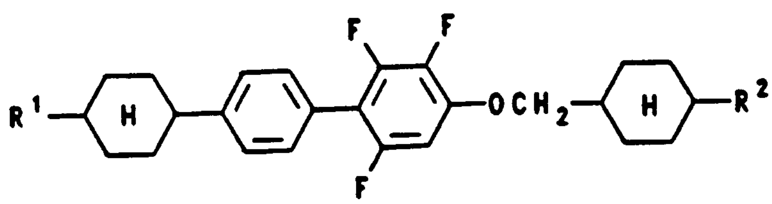

(Iv7)

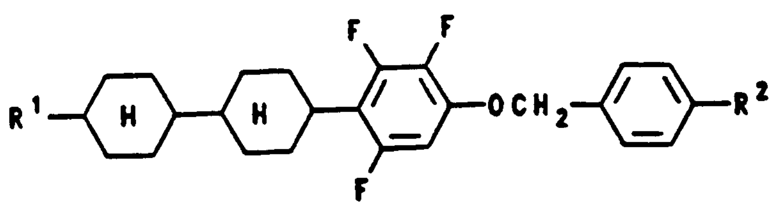

(Iv8) 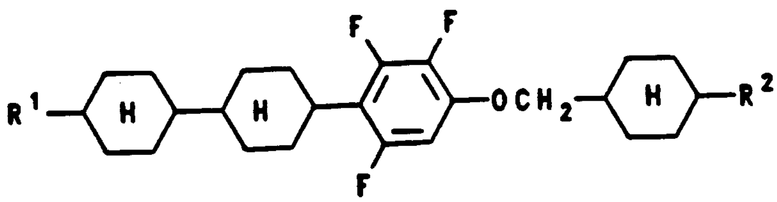

Bevorzugt werden ferner die Verbindungen der Teilstruktur

(Iw) 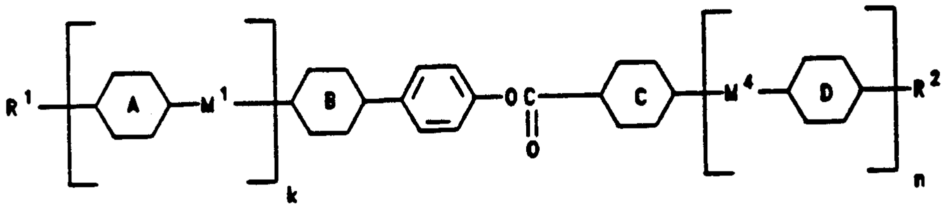

und darunter besonders bevorzugt die Verbindungen

(Iw1) 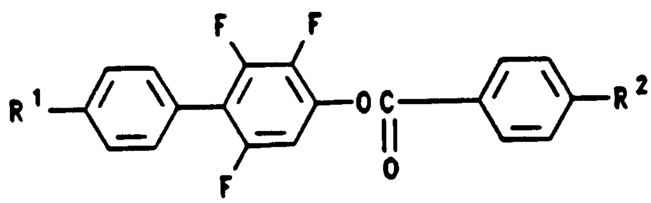

(Iw2) 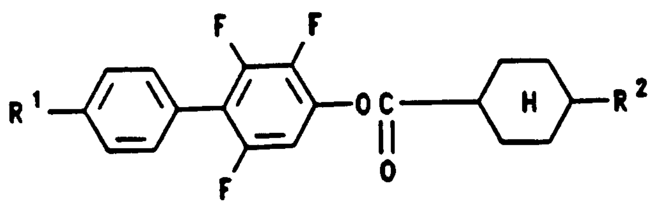

(Iw3) 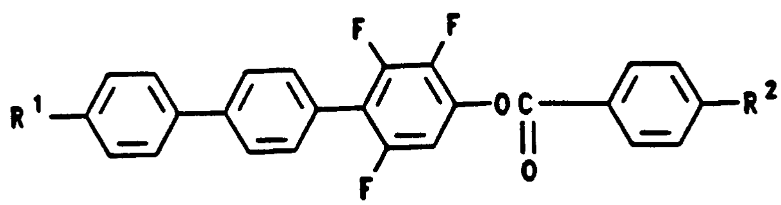

(Iw4)

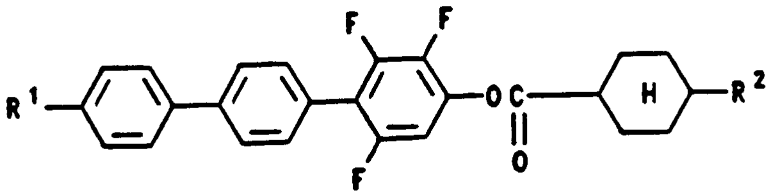

(Iw5)

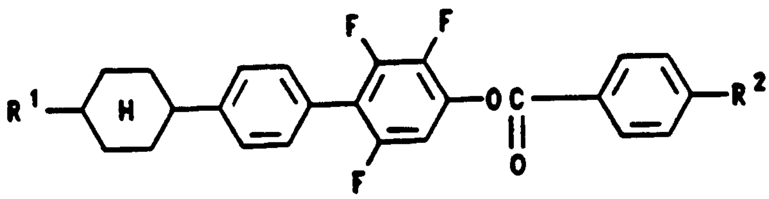

(Iw6)

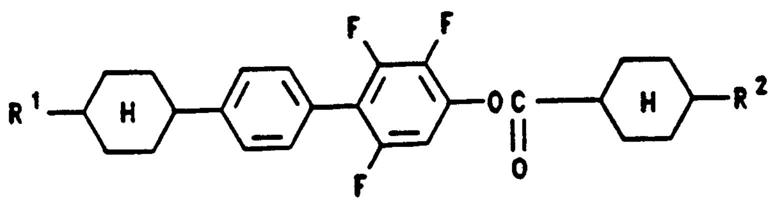

(Iw7)

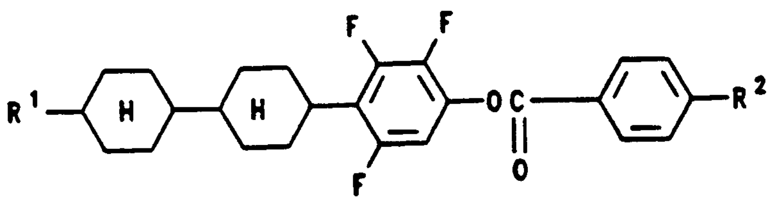

(Iw8)

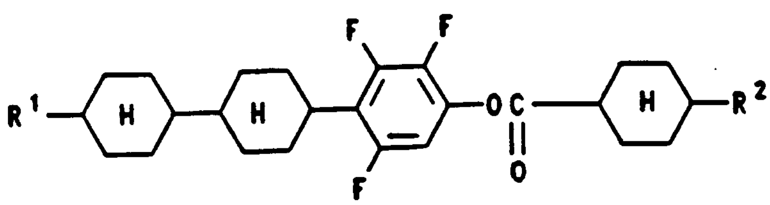

Bevorzugt werden ferner die Verbindungen der Teilstruktur

(Ix)

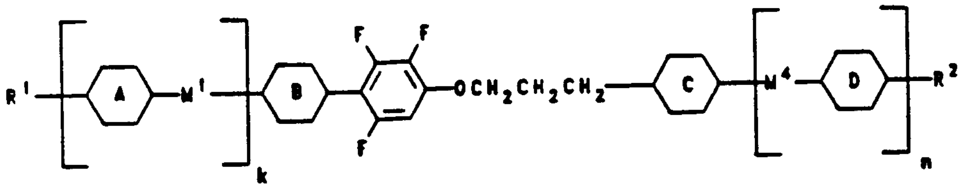

und darunter besonders bevorzugt die Verbindungen

( I x 1 ) 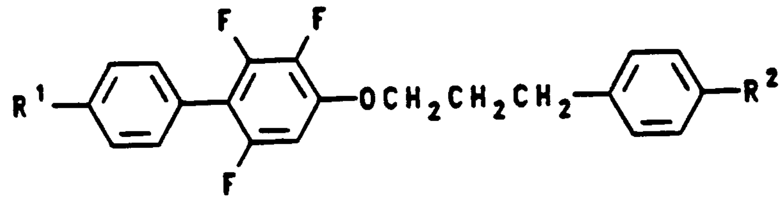

( I x 2 ) 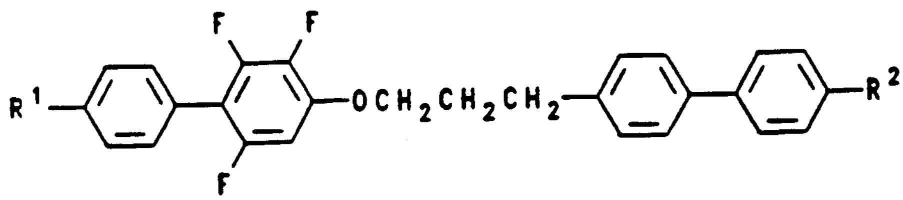

( I x 3 ) 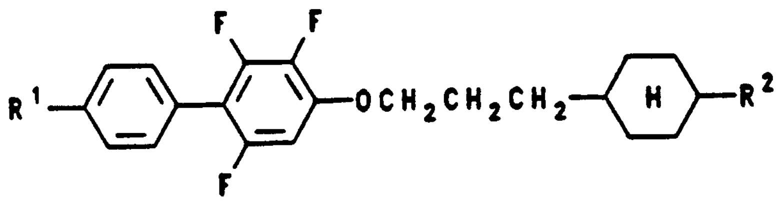

( I x 4 ) 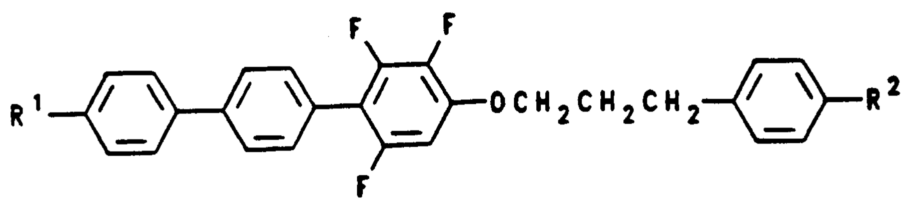

( I x 5 ) 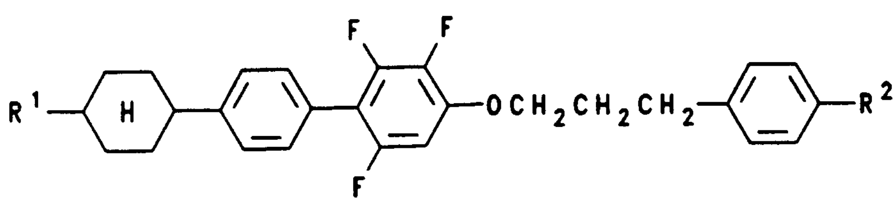

Die Herstellung der erfindungsgemäßen Verbindungen erfolgt nach an sich literaturbekannten Methoden, wie sie in Standardwerken zur Organischen Synthese, z.B. Houben-Weyl, Methoden der Organischen Chemie, Georg-Thieme-Verlag, Stuttgart, beschrieben werden.

Die Herstellung erfolgt dabei unter Reaktionsbedingungen, die für die genannten Umsetzungen bekannt und geeignet sind. Dabei kann auch von an sich bekannten, hier nicht näher erwähnten Varianten Gebrauch gemacht werden.

Beispielsweise kann, wie in DE-P 42 42 696.0 vorgeschlagen, die bifunktionelle 4-Hydroxy-2,3,5-trifluorbenzoesäure (II) vorteilhaft als Ausgangsmaterial eingesetzt werden. Dies wird in den Schemata 1 bis 7 näher beschrieben.

Schema 1 beschreibt die Synthese der Teilstrukturen Ia und Ib durch Umsetzung von (II) mit Alkylderivaten nach Standardmethoden (z.B. R.C. Larock, Comprehensive Organic Transformations, VCH Publishers, New York, p. 445) zu Alkylphenylethern und ihre anschließende Umsetzung mit Phenolen nach Standardmethoden (z.B. R.C. Larock, p.

966) zu den Estern Ia und Ib.

Nach im wesentlichen analogen Verfahren ist in Schema 2 die Synthese der Teilstrukturen Ic und Id beschrieben, wobei der erste Schritt, die Umsetzung der Carbonsäurefunktion mit Alkoholen, nach Standardmethoden erfolgen kann (z.B. R.C. Larock, p. 966).

Schema 3 beschreibt die Synthese von Benzylethern Ie bzw. If aus (II) durch Derivatisierung der Phenolfunktion, wie in Schema 1 beschrieben, anschließende Reduktion der Carboxylgruppe (z.b. nach R.C. Larock, p. 548) und Herstellung der Benzylether nach Standardmethoden (z.B. T.W. Greene, P.G.M. Wuts, Protective Groups in Organic Synthesis, J. Wiley & Sons, N.Y. 1991, pp. 156-160).

Schema 1

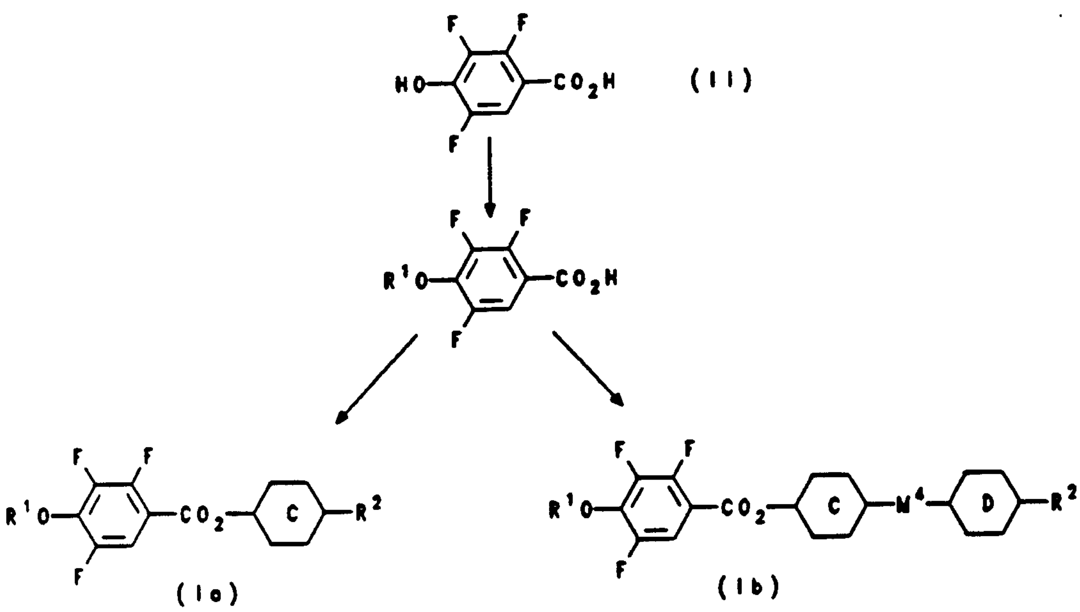

(II)

(Ia)

(Ib)

Schema 2

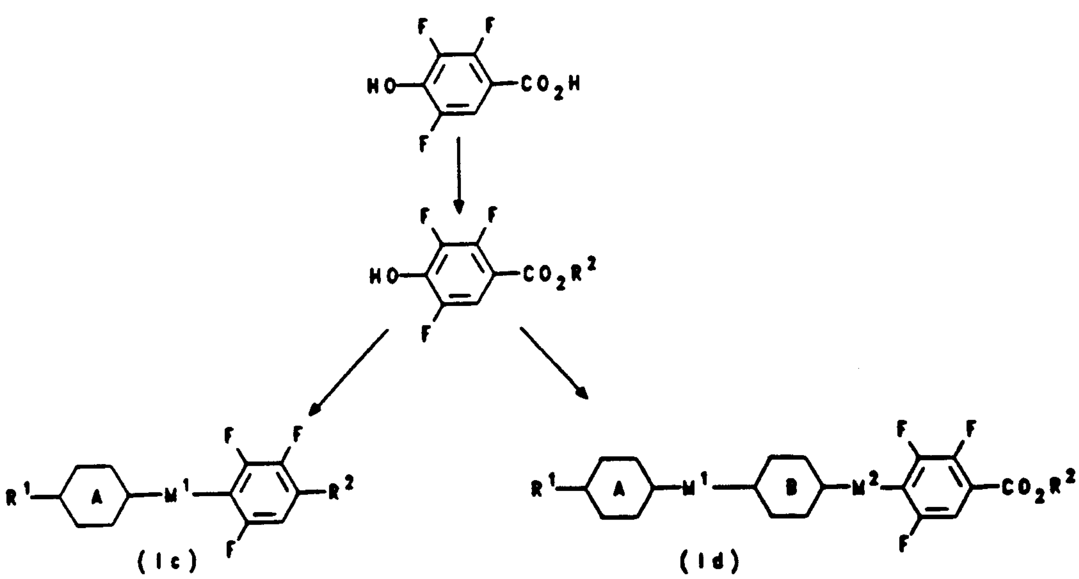

(Ic)

(Id)

Schema 3

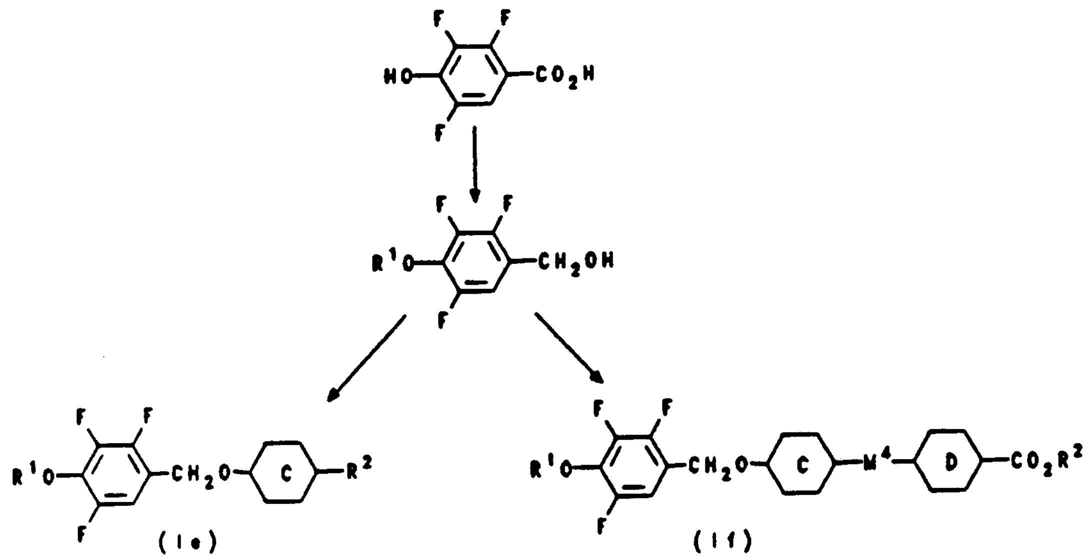

(Ie) (If)

Schema 4

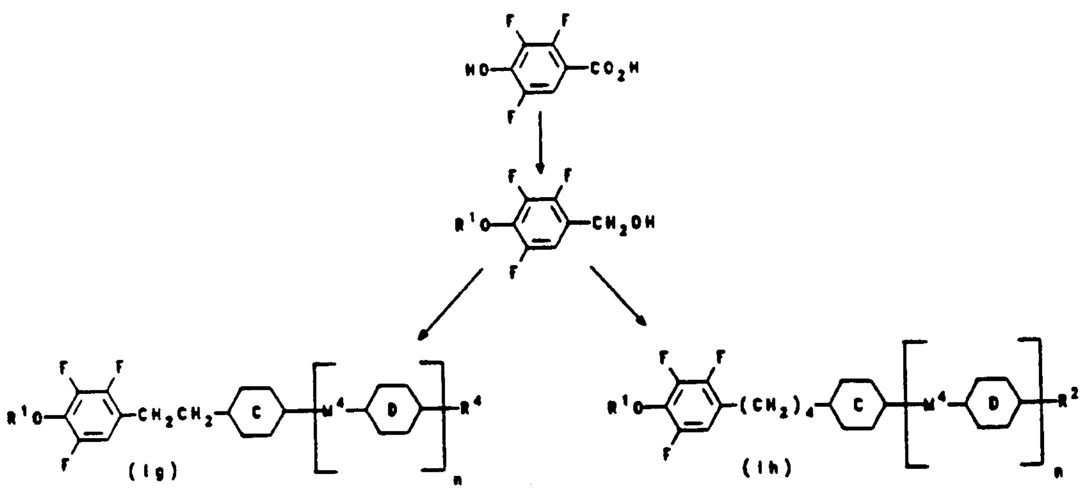

(Ig) (Ih)

Die nach Schema 3 erhaltenen Benzylalkohole können auch, wie in Schema 4 beschrieben, nach vorheriger Überführung in einen Ester der Toluolsulfonsäure mit metallorganischen Verbindungen zu den alkyl-verbrückten Teilstrukturen umgesetzt werden, wie z.B. in DE-A 32 01 721 für vergleichbare Verbindungen beschrieben.

Schema 5 beschreibt die Synthese der Teilstrukturen Ii bis In. Zentrales Vorprodukt ist die Halogenverbindung (III), die nach Standardmethoden (z.B. R.C. Larock, p. 381) aus der nach Schema 1 derivatisierten Carbonsäure erhalten werden kann. Aus (III) können nach Standardmethoden (z.B. Synthetic Comm. 11 (1981), 513; Tetrahedron Letters 28 (1987), 5093; J. Chem. Soc. Perkin Trans. II 1989, 2041; Mol. Cryst. Liq. Cryst. 172 (1989), 165; DE-C 3930663; EP-A 354434; EP-A 449015; WO 89/03821; WO 89/12039) mit metallorganischen Verbindungen oder (Aryl-)Boronsäuren die Teilstrukturen (Ii) erhalten werden. In Analogie zu Liq. Crystals 8 (1990), 861 lassen sich die Teilstrukturen (Ij) erhalten.

(III) kann auch nach Standardmethoden (z.B. R.C. Larock, p. 490) in das Phenol (IV) überführt werden, das seinerseits nach den bereits oben beschriebenen Methoden weiter zu den Teilstrukturen (Ik), (Il), (Im) und (In) umgesetzt werden kann.

## Schema 5

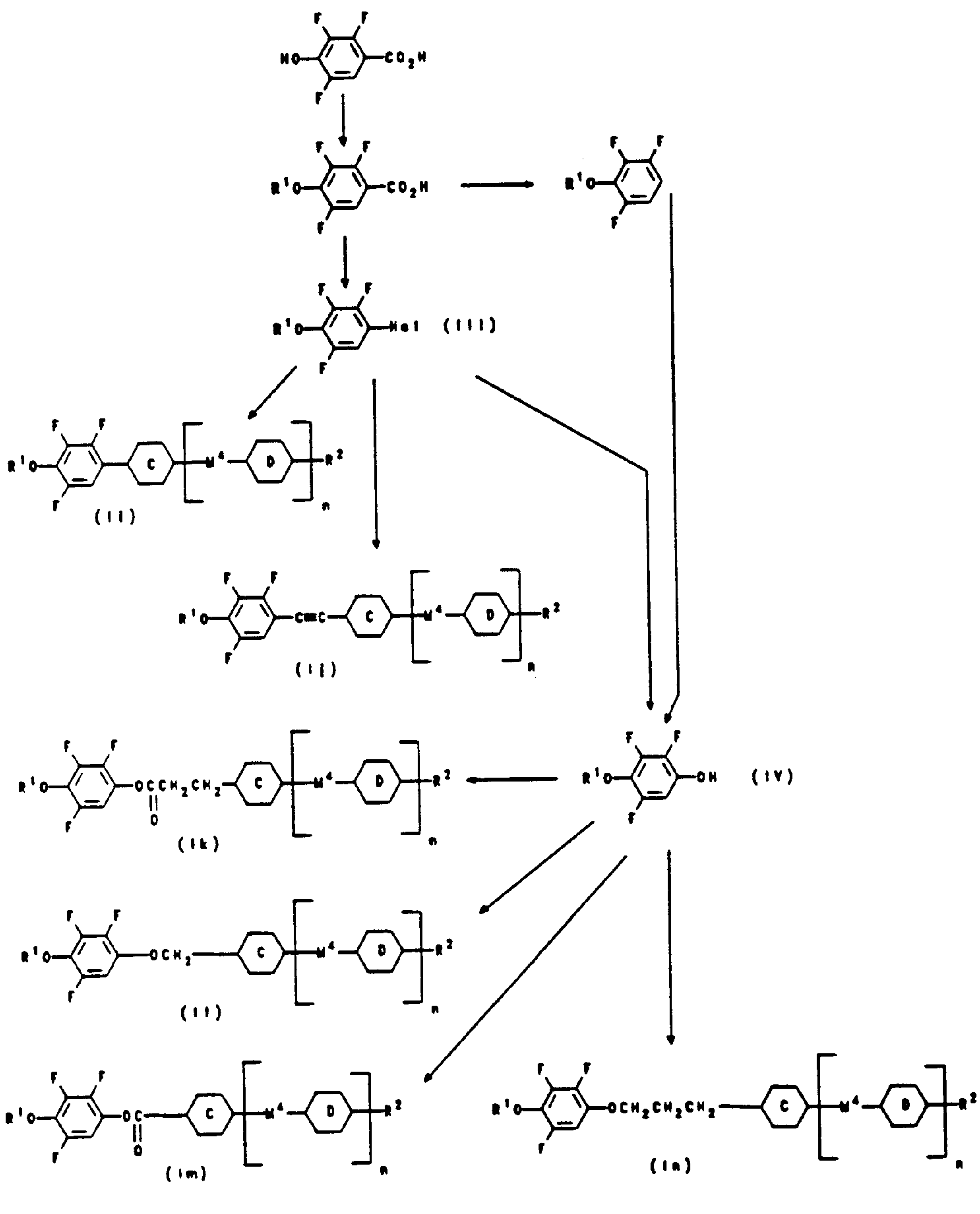

Der Teilstrukturen (Io), (Ip), (Iq) und (Ir) im Schema 6 leiten sich ebenfalls von (II) ab, das durch Reaktion mit Perfluoralkansulfonsäurederivate in den Ester (V) der Perfluoralkansulfonsäure überführt wird (nach z.B. J. Org. Chem. 30, 4322 (1965)); letzterer kann mit bereits weiter oben zitierten Verfahren in die Teilstrukturen (Io), (Ip), (Iq) und (Iv) überführt werden.

In Schema 7 ist angegeben, wie (V) nach bereits weiter oben zitierten Verfahren in die Teilstrukturen (Is) bis (Ix) überführt werden kann.

Schema 6

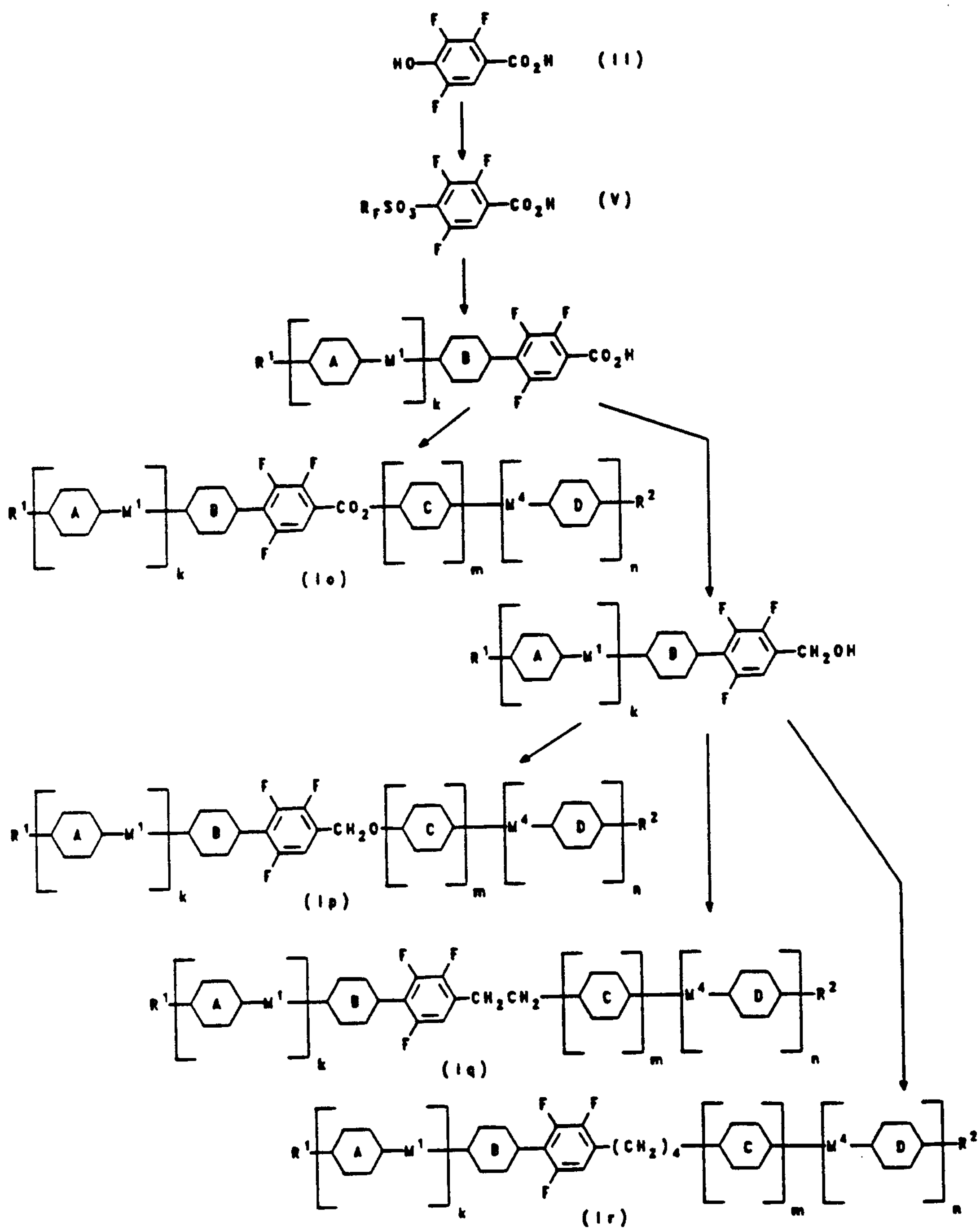

(II)

(V)

(Io)

(Ip)

(Iq)

(Ir)

Schema 7

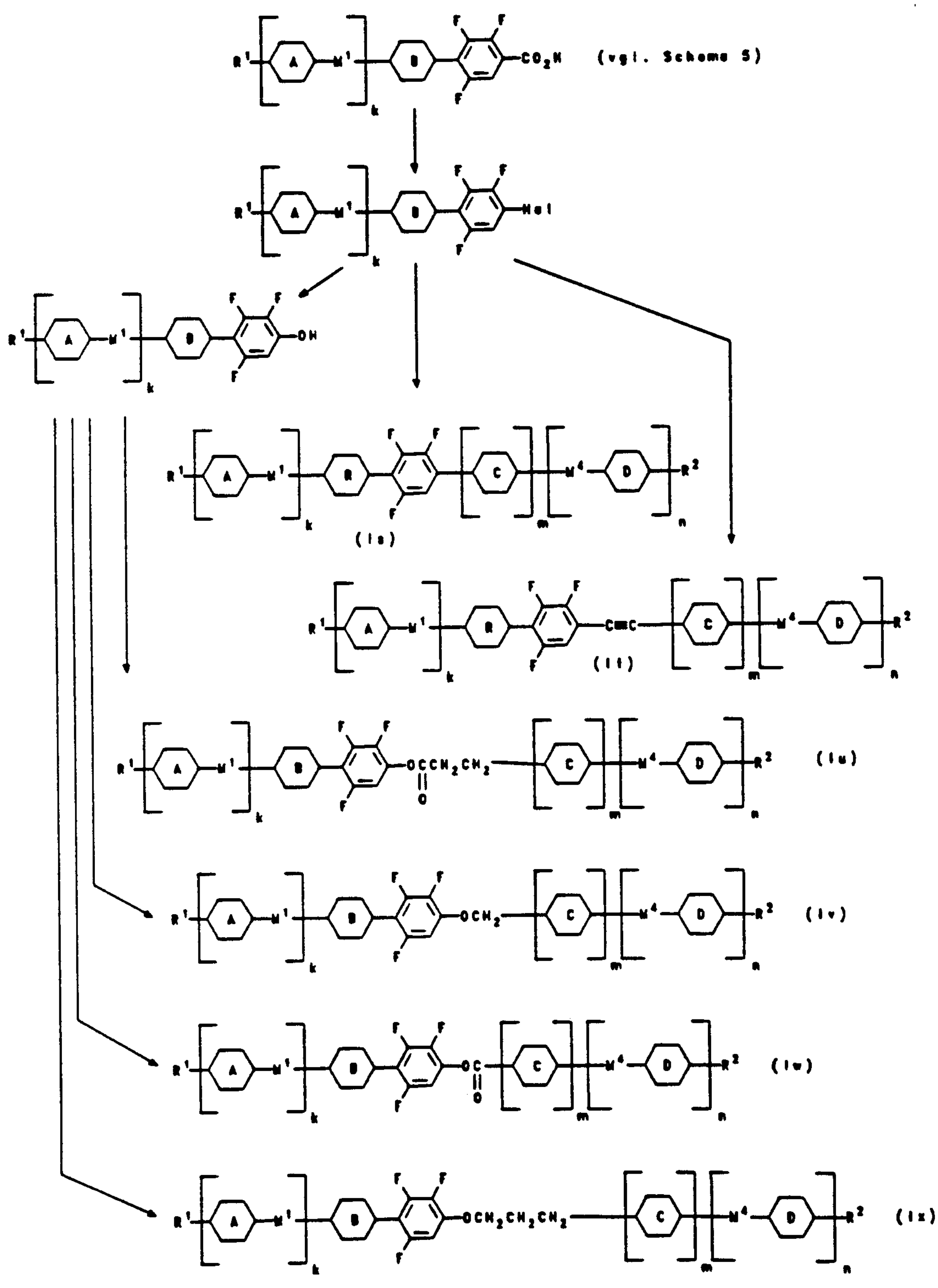


Natürlich können auch andere geeignete Ausgangsmaterialien für die Synthese der erfindungsgemäßen Verbindungen herangezogen werden.

So kann z.B. - in Analogie zu Schema 3 bzw. 7 - handelsübliches 2,3,5-Trifluorbrombenzol - verwendet werden (siehe Beispiel 18). Auch 1,2,4-Trifluorbenzol (handelsüblich ) kann - in Analogie zu z.B. Schema 5 - vorteilhaft für die Synthese der erfindungsgemäßen Verbindungen eingesetzt werden.

Die Ausgangsstoffe können gewünschtenfalls auch in situ gebildet werden, und zwar derart, daß man sie aus dem Reaktionsgemisch nicht isoliert, sondern sofort weiter zu den Verbindungen der Formel (I) umsetzt.

Die genannten Trifluorphenylen-Verbindungen sind als Komponenten für ferroelektrische Flüssigkristall-(LC)-mischungen, vorzugsweise solchen aus 2 bis 35, besonders bevorzugt 2 bis 20, Verbindungen, geeignet. Dabei können die Mischungen 0,01 bis 80 Gew.-%, bevorzugt 0,1 bis 60 Gew.-%, besonders bevorzugt 0,1 bis 30 Gew.-% an einer oder mehreren, vorzugsweise 1 bis 10, der erfindungsgemäßen Verbindungen enthalten.

Die anderen Bestandteile werden vorzugsweise ausgewählt aus den bekannten Verbindungen mit nematischen, cholesterischen und/oder smektischen Phasen; dazu gehören beispielsweise Schiffsche Basen, Biphenyle, Terphenyle, Phenylcyclohexane, Cyclohexylbiphenyle, N, S oder O-haltige Heterocyclen, z.B. Pyrimidine, Zimtsäureester, Cholesterinester oder verschieden überbrückte, terminalpolar mehrkernige Ester von p-Alkylbenzoesäuren.

Die Verbindungen der Formel I besitzen einen breiten Anwendungsbereich. In Abhängigkeit von der Auswahl der Substituenten können diese Verbindungen als Basismaterialien dienen, aus denen flüssigkristalline smektische, ferroelektrische Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie und/oder die Viskosität und/oder die spontane Polarisation und/oder die Phasenbereiche und/oder den Tiltwinkel und/oder den Pitch eines solchen Dielektrikums zu variieren.

Die erfindungsgemäßen Mischungen wiederum können beispielsweise Anwendung finden in elektrooptischen oder vollständig optischen Elementen, z.B. Anzeigelementen, Schaltelementen, Lichtmodulatoren, Elementen zur Bildbearbeitung und/oder Signalverarbeitung oder allgemein im Bereich der nichtlinearen Optik.

Elektrooptische Anzeigeelemente, deren flüssigkristalline Mischungen Verbindungen der Formel I enthalten, beinhalten darüber hinaus beispielsweise folgende Komponenten:
zwei Elektroden, zwei Trägerplatten sowie mindestens eine Orientierungsschicht. Allgemein ist der Aufbau von FLC-Displays beispielsweise in der EP-B 0 032 362 beschrieben.

Die vorliegende Erfindung wird an Hand der folgenden Beispiele näher erläutert.

Beispiel 1

4-(trans-4-Pentylcyclohexyl)benzoesäure-4-(1-methyloxycarbonyl-2,3,5-trifluor)phenyl-ester

Eine Lösung von 2,3 g 4-Hydroxy-2,3,5-trifluorbenzoesäure-methylester (hergestellt durch Umsetzung von 4-Hydroxy-2,3,5-trifluorbenzoesäure mit Methanol unter Verwendung von N,N'-Carbonyldiimidazol; Schmp. 112-114°C) in 40 ml Dichlormethan wird mit 2,3 g Dicyclohexylcarbodiimid sowie 3,1 g 4-(trans-4-Pentylcyclohexyl)benzoesäure versetzt und für 24 h bei 20°C gerührt. Nach Filtration wird über Kieselgel mit Dichlormethan/Ethylacetat 4:1 chromatographiert. Nach Umkristallisation aus Acetonitril werden 2,5 g des Produktes in Form farbloser Kristalle erhalten.

Phasenfolge: $X_1$ 60 $X_2$ 68 N 176 I

Analog werden dargestellt:
Beispiel 2

4-(2,3-Difluor-4-octyloxy)benzoesäure-4-(1-hexyloxycarbonyl-2,3,5-trifluor)phenyl-ester

Phasenfolge: X 62 I

Beispiel 3

4-(2,2,3,3-H-Perfluoroctyloxy)benzoesäure-4-(1-methyloxycarbonyl-2,3,5-trifluor)phenyl-ester

Phasenfolge: $X_1$ 71 $X_2$ 99 $S_A$ 106 I

Beispiel 4

2-(6-Decyloxy)naphthoesäure-4-(1-methyloxycarbonyl-2,3,5-trifluor)phenyl-ester

Phasenfolge: $X_1$ 86 $X_2$ 91 N 92 I

Beispiel 5

4-(4'-Octyloxy-biphenyl-1,1')carbonsäure-4-(1-methyloxycarbonyl-2,3,5-trifluor)phenyl-ester

Phasenfolge: X 107 $S_A$ 138 N 179 I

Beispiel 6

3-(trans-4-Ethyl-cyclohexyl)propionsäure-4-(1-methyloxycarbonyl-2,3,5-trifluor)phenyl-ester

Phasenfolge: X 22 I

Beispiel 7

4-(4'-Propyl-biphenyl-1,1')carbonsäure-4-(1-methyloxycarbonyl-2,3,5-trifluor)phenyl-ester

Phasenfolge: X 105 N 172 I

Beispiel 8

4-(4-Decyloxybenzoyloxy)benzoesäure-4-(1-methyloxycarbonyl-2,3,5-trifluor)phenyl-ester

Phasenfolge: X 119 (74 $S_A$ 107) N 174 I

Beispiel 9

4-(4-Octyloxybenzoyloxy)-2,3,5-trifluor-benzoesäure-octylester

Phasenfolge: X 38 I

Beispiel 10

4-[4-(1,1,2,2-H-Perfluoroctyloxy)]benzoyloxy-2,3,5-trifluor-benzoesäure-octylester

Phasenfolge: X 74 (54) $S_C$ (61) $S_A$ (62) I

Beispiel 11

4-[4-(1,1,2,2-H-Perfluoroctyloxy)]benzoyloxy-2,3,5-trifluor-benzoesäure-(1,1,2,2-H-perfluoroctyl)ester

Phasenfolge: X 95 $S_C$ 96 $S_A$ 105 I

Beispiel 12

4-(trans-4-Pentylcyclohexyl)benzoesäure-{4-[1-(1,1,2,2-H-perfluoroctyloxycarbonyl)]-2,3,5-trifluor-phenyl}ester

Phasenfolge: X 85 $S_A$ 150 I

Beispiel 13

4-(5-Octyl-pyrimidin-2-yl)phenyloxyessigsäure-[4-(1-butyloxycarbonyl)-2,3,5-trifluor]phenyl-ester

Phasenfolge: X 89 I

Beispiel 14

4-(4-Butyldimethylsilyl-butyloxy)benzoyloxy-2,3,5-trifluor-benzoesäure-butylester

Phasenfolge: X -6 I

Analog Beispiel 1, jedoch unter Verwendung von 4-Methoxy-2,3,5-trifluorbenzoesäure (hergestellt durch Umsetzung von 4-Hydroxy-2,3,5-trifluorbenzoesäure mit 2 Äquivalenten Methanol und 2 Äquivalenten "Mitsunobu-Reagenz" und anschließender Verseifung des Methylesters; Schmp. 142-145 °C werden hergestellt:

Beispiel 15

(4-Methoxy-2,3,5-trifluor)benzoesäure-[4-(5-octyloxy-pyrimidin-2-yl)phenyl]ester

Phasenfolge: X 117 ($S_A$ 98) N 149 I

Beispiel 16

(4-Methoxy-2,3,5-trifluor)benzoesäure-4-[1-methyloxycarbonyl-2,3,5-trifluor]phenyl-ester

Beispiel 17

(4-Methoxy-2,3,5-trifluor)benzoesäure-4-[1-(1,1,2,2-H-perfluordecyl)oxycarbonyl-2,3,5-trifluor]phenyl-ester

Beispiel 18

4,4''-Bis(octyloxy)-2',3',5'-trifluor-1,1':4',1''-terphenyl

Eine Lösung von 3,3 g 4-Brom-2,3,5-trifluor-4'-octyloxy-biphenyl (erhalten durch Pd-katalysierte Umsetzung des im Handel erhältlichen 2,3,5-Trifluor-brombenzols mit 4-Octyloxyphenylboronsäure zu 4-Octyloxy-2',3',5'-trifluor-biphenyl [Schmp. 31 °C], dessen Überführung in die entsprechende Boronsäure durch die Sequenz Lithiierung - Reaktion mit Trimethylborat - Hydrolyse und zuletzt Umsetzung der Boronsäure mit $Br_2$), 4,0 g 4-Octyloxyphenylboronsäure, 0,1 g Tetrakis(triphenylphosphin)-palladium(0) und 5,1 g $Na_2CO_3$ in 90 ml Toluol, 45 ml Ethanol und 30 ml $H_2O$ wird für 2 h zum Sieden erhitzt. Durch übliche Aufarbeitung, Reinigung durch Chromatographie und Umkristallisation aus Acetonitril wird das Produkt erhalten.

Phasenfolge: X 98 N 110 I

Anwendungsbeispiel 1

Einer $S_C$-Grundmischung mit einem Schmelzpunkt von +4 °C und einem Klärpunkt von 85 °C sowie der Zusammensetzung [mol-%]

| | |
|---|---|
| 5-Octyl-2-(4-hexyloxyphenyl)pyrimidin | 21,45 % |
| 5-Octyl-2-(4-decyloxyphenyl)pyrimidin | 14,30 % |
| 5-Octyl-2-(4-Octyloxyphenyl)pyrimidin | 19,25 % |
| 5-Octyloxy-2-(4-hexyloxyphenyl)pyrimidin | 13,40 % |
| 5-Octyloxy-2-(4-octyloxyphenyl)pyrimidin | 6,20 % |
| 5-Octyloxy-2-(4-butyloxyphenyl)pyrimidin | 14,15 % |
| 5-Octyloxy-2-(4-decyloxyphenyl)pyrimidin | 11,25 % |

werden 10 mol-% der Verbindung aus Beispiel 1 zugesetzt; der Schmelzpunkt wird um 5 K auf -1 °C erniedrigt, während die eher unerwünschte Absenkung des Klärpunktes mit 2 K auf 83 °C gering ausfällt. Dieses Beispiel belegt die gute Eignung der erfindungsgemäßen Verbindungen, die Eigenschaften von Flüssigkristallmischungen, in diesem Anwendungsbeispiel den Schmelzpunkt, günstig zu beeinflussen.

Anwendungsbeispiel 2

Der Grundmischung aus Anwendungsbeispiel 1 werden 10 mol-% der Verbindung aus Beispiel 15 zugesetzt; der Schmelzpunkt wird auf 1°C erniedrigt, der Klärpunkt auf 86°C erhöht.

Anwendungsbeispiel 3

Einer Grundmischung (TLC 1, Hoechst AG) mit einem X/$S_C$-Übergang von - 35°C sowie einem N/I-Übergang von 102°C werden 10 mol-% der Verbindung aus Beispiel 5 zugesetzt; der X/$S_C$-Übergang sinkt auf -40°C, der N/I-Übergang steigt auf 106°C.

Diese beiden Beispiele belegen die gute Eignung der erfindungsgemäßen Verbindungen, die Eigenschaften von Flüssigkristallmischungen, hier die Mesophasenbreite, günstig zu beeinflussen.

**Patentansprüche**

**1.** Trifluorphenylen-Verbindungen der Formel (I)

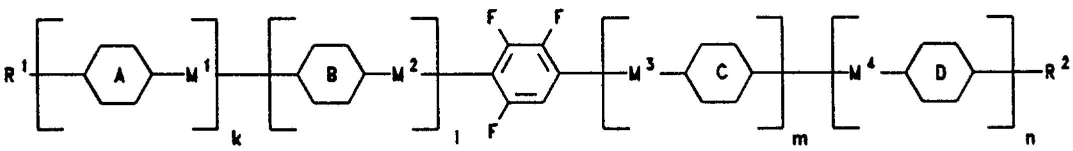

(I)

in der die Symbole und Indizes folgende Bedeutungen haben:

$R^1$, $R^2$ sind, unabhängig voneinander Wasserstoff, oder ein geradkettiger oder verzweigter Alkylrest mit 1 bis 20 C-Atomen (mit oder ohne asymmetrische C-Atome), wobei auch eine oder mehrere $CH_2$-Gruppen durch -O-, -S-,-CO-, -CH=CH-, -C≡C-, Δ,-Si$(CH_3)_2$-, 1,4-Phenylen, trans-1,4-Cyclohexylen oder trans-1,3-Cyclopentylen ersetzt sein können, mit der Maßgabe, daß Sauerstoffatome und Schwefelatome (im folgenden Chalkogene genannt) nicht unmittelbar miteinander gebunden sein dürfen, und/oder ein oder mehrere H-Atome des Alkylrestes durch -F, -Cl, - Br, -$OR^3$ substituiert sein können; oder einer der nachfolgend aufgeführten Reste

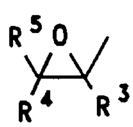
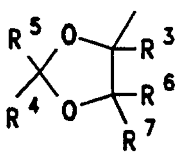
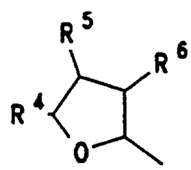

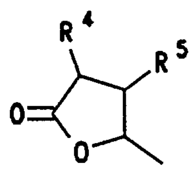
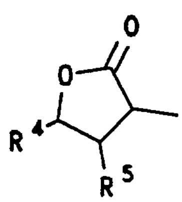
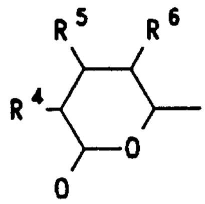

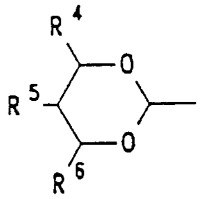
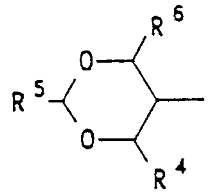
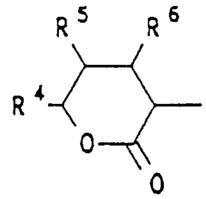

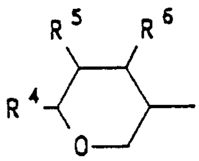
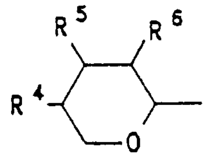
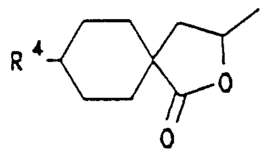

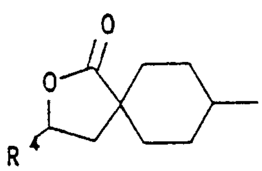
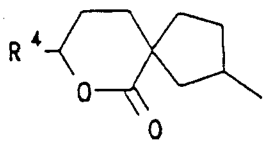
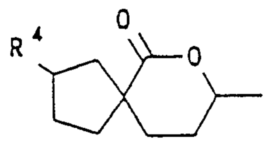

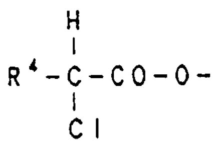
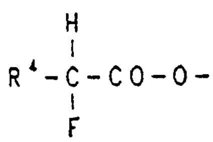
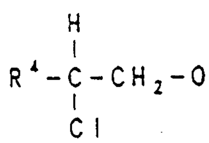
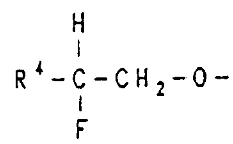

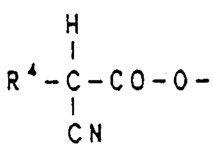
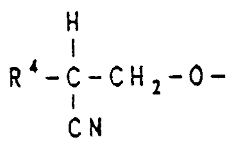
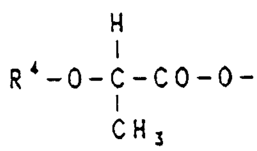
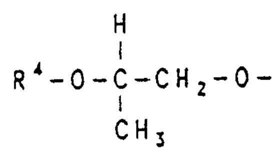

| | |
|---|---|
| $R^3$, $R^4$, $R^5$, $R^6$, $R^7$ | sind unabhängig voneinander Wasserstoff oder ein geradkettiger oder verzweigter Alkylrest mit 1-16 C-Atome (mit oder ohne asymmetrische C-Atome), wobei auch eine oder mehrere $CH_2$-Gruppen durch -O- oder -CH = CH- ersetzt sein können (mit der Maßgabe, daß Sauerstoffatome nicht unmittelbar miteinander verbunden sein dürfen) und/oder ein oder mehrere H-Atome des Alkylrestes durch -F oder -Cl substituiert sein können; $R^4$ und $R^5$ können zusammen auch - $(CH_2)_4$- oder -$(CH_2)_5$- sein, wenn sie an ein Oxiran-, Dioxolan-, Tetrahydrofuran-, Tetrahydropyran-, Butyrolacton-, oder Valerolacton-System gebunden sind; |
| $M^1$, $M^2$, $M^3$, $M^4$ | sind gleich oder verschieden -CO-O-, -O-CO-, |

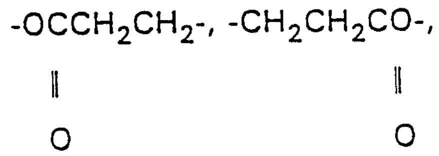

-$(CH_2)_4$-, -$O(CH_2)_3$-, -$(CH_2)_3O$-, -$CH_2$-O-, -O-$CH_2$-, -CH = CH-, -C≡C-, oder eine Einfachbindung;

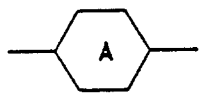 , 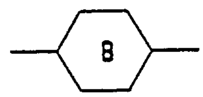 ,

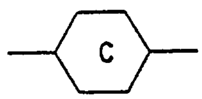 , 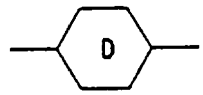 ,

sind gleich oder verschieden, 1,4-Phenylen, wobei ein, zwei, drei oder vier H-Atome durch F, Cl und/oder CN ersetzt sein können, Pyrazin-2,5-diyl, Pyridazin-3,6-diyl- Pyridin-2,5-diyl, bei dem auch 1 H-Atom durch F ersetzt sein kann, Pyrimidin-2,5-diyl, bei dem auch ein H-Atom durch F ersetzt sein kann, trans-1,4-Cyclohexylen, bei dem ein oder zwei H-Atome durch CN und/oder $CH_3$ ersetzt sein können, (1,3,4)-Thiadiazol-2,5-diyl, 1,3-Dioxan-2,5-diyl, 1,3-Thiazol-2,4-diyl, 1,3-Thiazol-2,5-diyl, Thiophen-2,4-diyl, Thiophen-2,5-diyl, Piperazin-1,4-diyl, Piperazin-2,5-diyl, Naphthalin-2,6-diyl, Bicyclo[2.2.2]octan-1,4-diyl oder 1,3-Dioxaborinan-2,5-diyl;

k, l, m, n sind null oder eins, unter der Bedingung, daß die Summe k + l + m + n 1, 2 oder 3 ist,

**2.** Verbindungen nach Anspruch 1, dadurch gekennzeichnet, daß die Symbole und Indizes folgende Bedeutung haben:

$R^1$, $R^2$ unabhängig voneinander H oder Alkyl mit 1 bis 20 C-Atomen, wobei auch eine oder mehrere $CH_2$-Gruppen durch -O-, - CH=CH-, -CO-, -C≡C- oder -$Si(CH_3)_2$-, ersetzt sein können und eine oder mehrere H-Atome durch F ersetzt sein können; oder eine der Gruppen

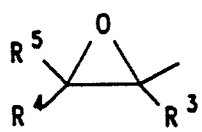 , 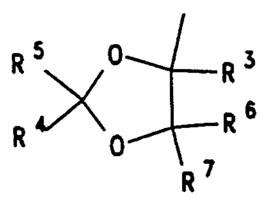

$M^1$, $M^2$, $M^3$, $M^4$ -CO-O-, -O-CO-, -$CH_2$-O-, -$OCH_2$-, -C≡C- oder eine Einfachbindung

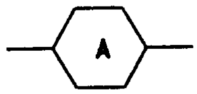 , 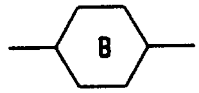 ,

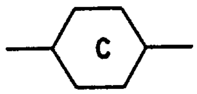 , 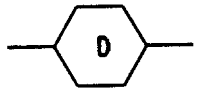 ,

1,4-Phenylen, wobei ein, zwei oder drei H-Atome durch F ersetzt sein können, Pyrimidin-2,5-diyl, Pyridin-2,5-diyl, bei denen auch 1 H-Atom durch F ersetzt sein kann, 1,4-Cyclohexylen, 1,3-Dioxan-2,5-diyl, (1,3,4)-Thiadiazol, Naphthalin-2,6-diyl,

k + l + m + n  1 oder 2.

3. Verbindungen nach Anspruch 1 oder 2, dadurch gekennzeichnet, daß die Symbole und Indizes folgende Bedeutung haben:

$R^1$, $R^2$  Alkyl mit 1 bis 16 C-Atomen, wobei auch eine oder mehrere - $CH_2$-Gruppen durch -O-, -CH=CH-, oder -$Si(CH_3)_2$- ersetzt sein können und ein oder mehrere H-Atome durch F ersetzt sein können;

$M^1$, $M^2$, $M^3$, $M^4$  -CO-O-, -O-CO-, oder eine Einfachbindung,

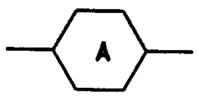 , 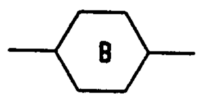 ,

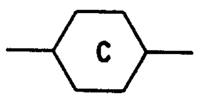 , 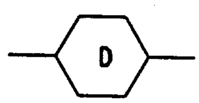 ,

1,4-Phenylen, wobei ein, zwei oder drei H-Atome durch F ersetzt sein können, Pyrimidin-2,5-diyl, Pyridin-2,5-diyl, bei denen auch 1 H-Atom durch F ersetzt sein kann, 1,4-Cyclohexylen, Naphthalin-2,6-diyl

k + l + m + n  1 oder 2.

4. Ferroelektrische flüssigkristalline Mischung, enthaltend 2 bis 35 Komponenten, von denen mindestens eine ein Trifluorophenylenderivat nach Anspruch 1, 2 oder 3 ist.

5. Flüssigkristalline Mischung nach Anspruch 4, dadurch gekennzeichnet, daß die Verbindung nach den Ansprüchen 1, 2 oder 3 zu 0,01 bis 80 Gew.-% enthalten ist.

6. Verwendung einer flüssigkristallinen Mischung nach den Ansprüchen 4 oder 5 in elektrooptischen Schalt- und/oder Anzeigeelementen.

7. Elektrooptisches Schalt- und/oder Anzeigeelement, enthaltend eine flüssigkristalline Mischung nach einem oder mehreren der Ansprüche 4 und 5.

## Claims

1. A trifluorophenylene compound of the formula (I)

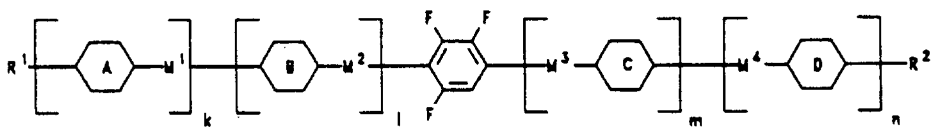 (I)

in which the symbols and indices have the following meanings:

$R^1$ and $R^2$ are, independently of one another, hydrogen, or a straight-chain or branched alkyl radical having 1 to 20 carbon atoms (with or without asymmetrical carbon atoms), in which one or more $CH_2$ groups may also be replaced by -O-, -S-, -CO-, - CH=CH-, -C≡C-, Δ, -Si(CH$_3$)$_2$-, 1,4-phenylene, trans-1,4-cyclohexylene or trans-1,3-cyclopentylene, with the proviso that oxygen atoms and sulfur atoms (referred to as chalcogens below) must not be bonded directly to one another, and/or one or more H atoms of the alkyl radical may be substituted by -F, -Cl, -Br or - OR$^3$; or are one of the radicals listed below

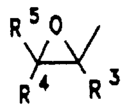
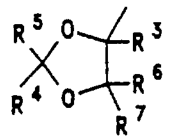
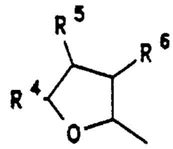

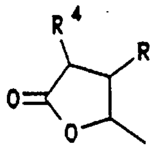
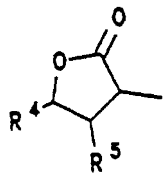
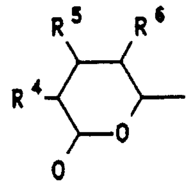

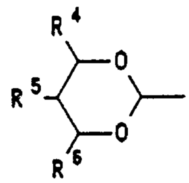
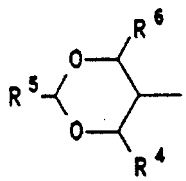
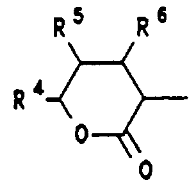

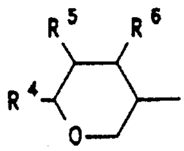
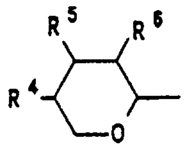
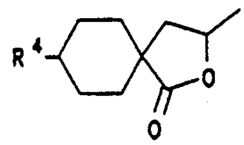

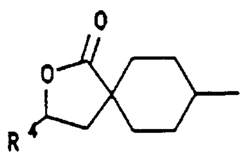
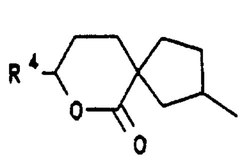
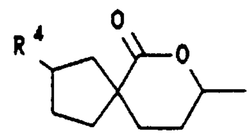

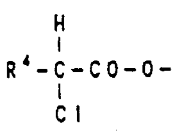
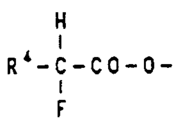
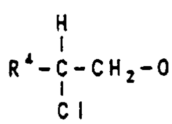
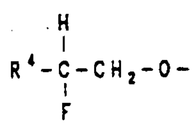

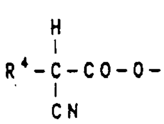
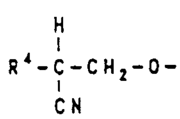
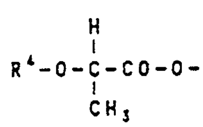
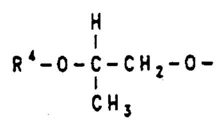

$R^3$, $R^4$, $R^5$, $R^6$ and $R^7$ are, independently of one another, hydrogen or a straight-chain or branched alkyl radical having 1-16 carbon atoms (with or without asymmetrical carbon atoms), in which one or more $CH_2$ groups may also be replaced by -O- or -CH=CH-(with the proviso that oxygen atoms must not be bonded directly to one another) and/or one or more H atoms of the

alkyl radical may be substituted by -F or -Cl; $R^4$ and $R^5$ together may also be $-(CH_2)_4-$ or $-(CH_2)_5-$ if they are bonded to an oxirane, dioxolane, tetrahydrofuran, tetrahydropyran, butyrolactone or valerolactone system;

$M^1$, $M^2$, $M^3$ and $M^4$ are identical or different and are -CO-O-, -O-CO-,

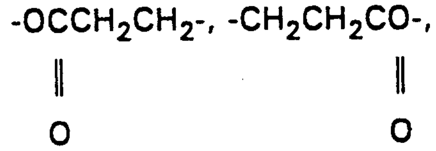

$-(CH_2)_4-$, $-O(CH_2)_3-$, $-(CH_2)_3O-$, $-CH_2-O-$, $-O-CH_2-$, -CH=CH-, -C≡C-, a single bond;

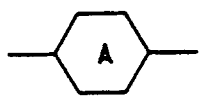 , 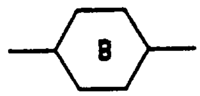 ,

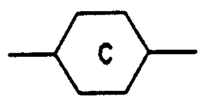 , 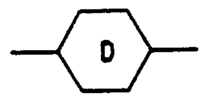 ,

are identical or different and are 1,4-phenylene, in which one, two, three or four H atoms may be replaced by F, Cl and/or CN, pyrazine-2,5-diyl, pyridazine-3,6-diyl, pyridine-2,5-diyl, in which one H atom may also be replaced by F, pyrimidine-2,5-diyl, in which 1 H atom may also be replaced by F, trans-1,4-cyclohexylene, in which one or two H atoms may be replaced by CN and/or $CH_3$, 1,3,4-thiadiazole-2,5-diyl, 1,3-dioxane-2,5-diyl, 1,3-thiazole-2,4-diyl, 1,3-thiazole-2,5-diyl, thiophene-2,4-diyl, thiophene-2,5-diyl, piperazine-1,4-diyl, piperazine-2,5-diyl, naphthalene-2,6-diyl, bicyclo[2.2.2]octane-1,4-diyl or 1,3-dioxaborinane-2,5-diyl;

k, l, m and n are zero or one, with the proviso that the sum k + l + m + n is 1, 2 or 3.

2. A compound as claimed in claim 1, wherein the symbols and indices have the following meanings:

$R^1$ and $R^2$, independently of one another, are H or alkyl having 1 to 20 carbon atoms, in which one or more $CH_2$ groups may also be replaced by -O-, -CH=CH-, -CO-, -C≡C- or $-Si(CH_3)_2-$, and one or more H atoms may be replaced by F; or are one of the groups

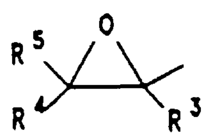 , 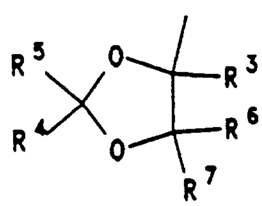

$M^1$, $M^2$, $M^3$ and $M^4$ are -CO-O-, -O-CO-, $-CH_2-O-$, $-OCH_2-$, -C≡C- or a single bond,

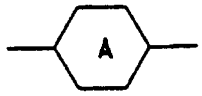 , 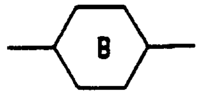 ,

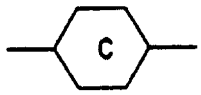 , 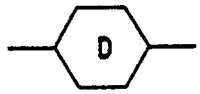 ,

are 1,4-phenylene, in which one, two or three H atoms may be replaced by F, pyrimidine-2,5-diyl, pyridine-2,5-diyl, in which 1 H atom may also be replaced by F, 1,4-cyclohexylene, 1,3-dioxane-2,5-diyl, 1,3,4-thiadiazole or naphthalene-2,6-diyl, and

k + l + m + n is 1 or 2.

**3.** A compound as claimed in claim 1 or 2, wherein the symbols and indices have the following meanings:

$R^1$ and $R^2$ are alkyl having 1 to 16 carbon atoms, in which one or more $-CH_2-$ groups may also be replaced by -O-, -CH=CH- or $-Si(CH_3)_2-$, and one or more H atoms may be replaced by F;

$M^1$, $M^2$, $M^3$ and $M^4$ are -CO-O-, -O-CO- or a single bond,

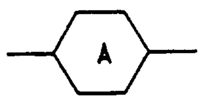 , 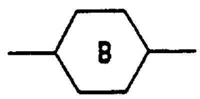 ,

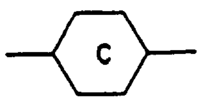 , 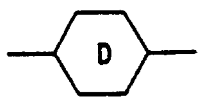 ,

are 1,4-phenylene, where one, two or three H atoms may be replaced by F, pyrimidine-2,5-diyl, pyridine-2,5-diyl, in which 1 H atom may also be replaced by F, 1,4-cyclohexylene or naphthalene-2,6-diyl, and

k + l + m + n is 1 or 2.

**4.** A ferroelectric liquid-crystalline mixture comprising from 2 to 35 components, of which at least one is a trifluorophenylene derivative as claimed in claim 1, 2 or 3.

**5.** A liquid-crystalline mixture as claimed in claim 4, wherein from 0.01 to 80% by weight of a compound as claimed in claim 1, 2 or 3 is present.

**6.** The use of a liquid-crystalline mixture as claimed in claim 4 or 5 in electro-optical switching and/or display elements.

**7.** An electro-optical switching and/or display element containing a liquid-crystalline mixture as claimed in one or more of claims 4 and 5.

**Revendications**

**1.** Composés de trifluorophénylène de formule (I)

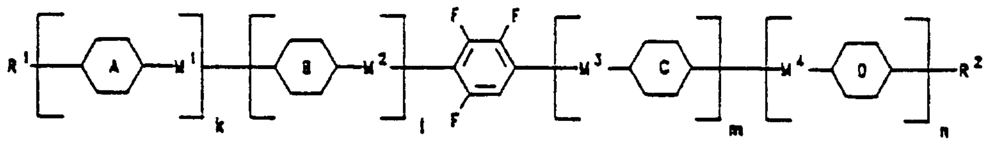 ( I )

dans laquelle les symboles et les indices ont la signification suivante:

$R^1$, $R^2$ sont, indépendamment l'un de l'autre, un atome d'hydrogène ou un reste alkyle linéaire ou ramifié de 1 à 20 atomes de carbone (avec ou sans atomes de carbone asymétriques) dont un ou plusieurs groupes - $CH_2$- peuvent être remplacés par -O-, -S-, -CO-, -CH=CH-, -C≡C-, Δ, - $Si(CH_3)_2$-, 1,4-phénylène, trans-1,4-cyclohexylène ou trans-1,3-cyclopentylène, à condition que des atomes d'oxygène et des atomes de soufre (appelés chalcogènes dans ce qui suit) ne soient pas liés directement entre eux, et/ou un ou plusieurs atomes d'hydrogène du reste alkyle peuvent être remplacés par - F, -Cl, -Br, $-OR_3$; ou l'un des restes décrits ci-dessous:

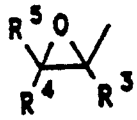

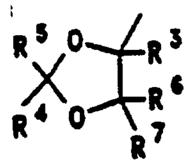

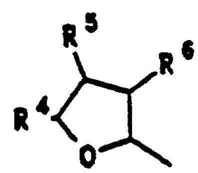

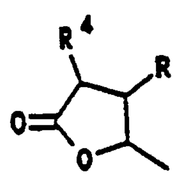

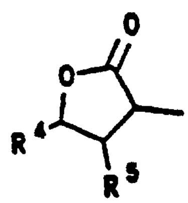

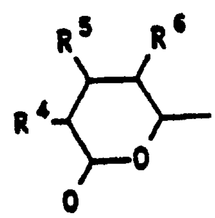

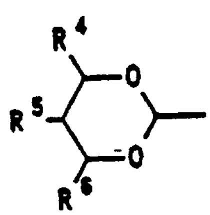

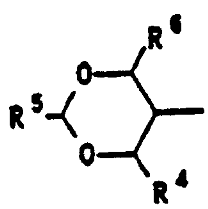

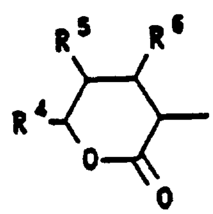

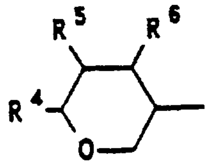 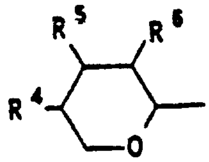 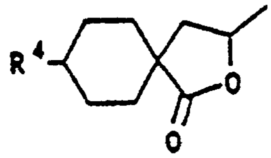

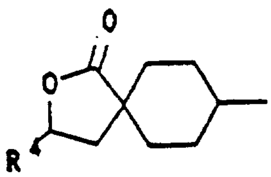 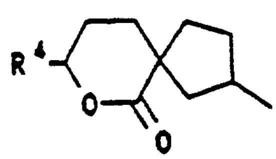 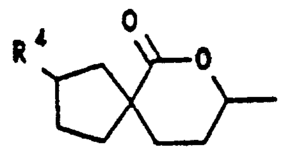

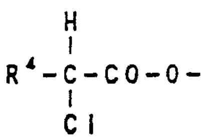 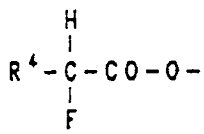 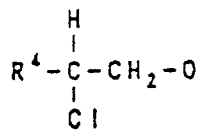 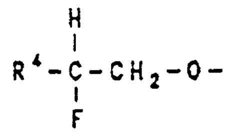

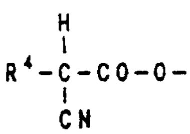 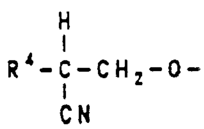 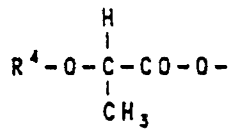 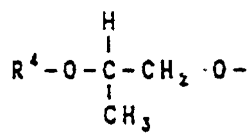

$R^3$, $R^4$, $R^5$, $R^6$, $R^7$ sont indépendamment les uns des autres un atome d'hydrogène ou un reste alkyle linéaire ou ramifié de 1 à 16 atomes de carbone (avec ou sans atomes de carbone asymétriques), un ou plusieurs groupes $CH_2$ pouvant être remplacés par -O- ou -CH=CH- (à condition que des atomes d'oxygène ne soient pas liés directement entre eux) et/ou un ou plusieurs atomes d'hydrogène du reste alkyle pouvant être remplacés par -F ou -Cl; $R^4$ et $R^5$ peuvent aussi former ensemble un reste $-(CH_2)_4-$ ou $-(CH_2)_5-$ lorsqu'ils sont liés à un système oxirane, dioxolane, tétrahydrofurane, tétrahydropyrane, butyrolactone ou valérolactone;

$M^1$, $M^2$, $M^3$, $M^4$ sont identiques ou différents et représentent des restes -CO-O-, -O-CO-,

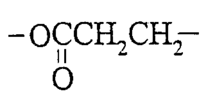 , 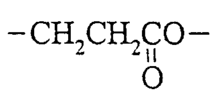 ,

$-(CH_2)_4-$, $-O(CH_2)_3-$, $-(CH_2)_3O-$, $-CH_2-O-$, $-O-CH_2-$, -CH=CH-, C≡C- ou une liaison simple;

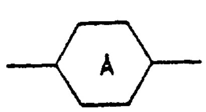 , 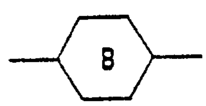 ,

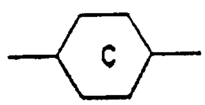 , 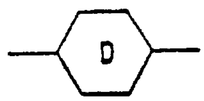 ,

sont identiques ou différents et représentent des restes 1,4-phénylène dont un, deux, trois ou quatre atomes d'hydrogène peuvent être remplacés par F, Cl et/ou CN, pyrazine-2,5-diyle, pyridazine-2,5-diyle, pyridine-2,5-diyle dont un atome d'hydrogène peut aussi être remplacé par F, pyrimidine-2,5-diyle dont un atome d'hydrogène peut aussi être remplacé par F, trans-1,4-cyclohexylène dont un ou deux atomes d'hydrogène peuvent être remplacés par CN et/ou $CH_3$, (1,3,4)-thiadiazole-2,5-diyle, 1,3-dioxane-2,5-diyle, 1,3-thiazole-2,4-diyle, 1,3-thiazole-2,5-diyle, thiophène-2,4-diyle, thiophène-2,5-diyle, pipérazine-1,4-diyle, pipérazine-2,5-diyle, naphtalène-2,6-diyle, bicyclo[2.2.2]octane-1,4-diyle ou 1,3-dioxaborinane-2,5-diyle;

k, l, m, n sont égaux à 0 ou 1, à condition que la somme k + l + m + n soit égale à 1, 2 ou 3.

**2.** Composés selon la revendication 1, caractérisés en ce que les symboles et les indices ont la signification suivante:

$R^1$, $R^2$ sont, indépendamment l'un de l'autre, un atome d'hydrogène ou un reste alkyle de 1 à 20 atomes de carbone dont un ou plusieurs groupes $CH_2$ peuvent être remplacés par -O-, -CH=CH-, -CO-, -C≡C- ou $-Si(CH_3)_2-$, et un ou plusieurs atomes d'hydrogène peuvent être remplacés par -F; ou l'un des groupes

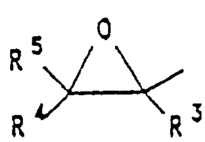 , 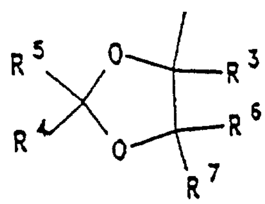

$M^1$, $M^2$, $M^3$, $M^4$ représentent des restes -CO-O-, -O-CO-, $-CH_2O-$, $-OCH_2-$, -C≡C- ou une liaison simple;

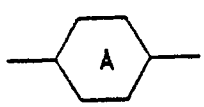 , 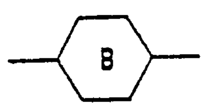 ,

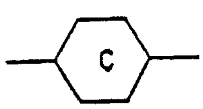 , 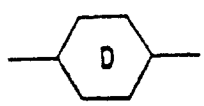 ,

représentent des restes 1,4-phénylène dont un, deux ou trois atomes d'hydrogène peuvent être remplacés par F, pyrimidine-2,5-diyle ou pyridine-2,5-diyle dans lesquels un atome d'hydrogène peut aussi être remplacé par F, 1,4-cyclohexylène, 1,3-dioxane-2,5-diyle, (1,3,4)-thiadiazole ou naphtalène-2,6-diyle;

la somme k + l + m + n est égale à 1 ou 2.

**3.** Composés selon la revendication 1 ou 2, caractérisés en ce que les symboles et les indices ont la signification suivante:

$R^1$, $R^2$ sont des restes alkyle de 1 à 16 atomes de carbone dont un ou plusieurs groupes $CH_2$ peuvent être remplacés par -O-, - CH=CH- ou $-Si(CH_3)_2-$, et un ou plusieurs atomes d'hydrogène peuvent être remplacés par - F;

$M^1$, $M^2$, $M^3$, $M^4$ représentent des restes -CO-O-, -O-CO- ou une liaison simple;

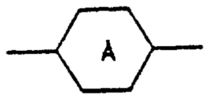 , 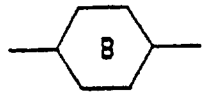 ,

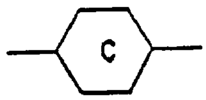 , 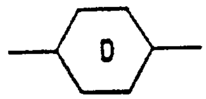 ,

représentent des restes 1,4-phénylène dont un, deux ou trois atomes d'hydrogène peuvent être remplacés par F, pyrimidine-2,5-diyle ou pyridine-2,5-diyle dans lesquels un atome d'hydrogène peut aussi être remplacé par F, 1,4-cyclohexylène ou naphtalène-2,6-diyle;

la somme k + l + m + n est égale à 1 ou 2.

4. Mélange cristallin liquide ferroélectrique contenant 2 à 35 constituants, dont l'un au moins est un dérivé de trifluorophénylène selon la revendication 1, 2 ou 3.

5. Mélange cristallin liquide selon la revendication 4, caractérisé en ce que le composé selon les revendications 1, 2 ou 3 est contenu en une quantité de 0,01 à 80 % en masse.

6. Utilisation d'un mélange cristallin liquide selon les revendications 4 ou 5 dans un élément de commutation et/ou d'affichage électro-optique.

7. Elément de commutation et/ou d'affichage électro-optique, contenant un mélange cristallin liquide selon l'une ou plusieurs des revendications 4 et 5.